# EUROPEAN PATENT SPECIFICATION

(11) **EP 4 448 534 B1**
(45) Date of publication and mention of the grant of the patent: **26.08.2026**
(21) Application number: 22836097.0
(22) Date of filing: 13.12.2022
(51) Int. Cl.: C07D 495/04, C07D 513/04, A01N 43/56

(54) **MICROBIOCIDAL BICYCLE HETEROCYCLIC DERIVATIVES**
MIKROBIZIDE BICYCLISCHE HETEROCYCLISCHE DERIVATE
DÉRIVÉS HÉTÉROCYCLIQUES POUR BICYCLETTE MICROBIOCIDE

(30) Priority: 15.12.2021 IN 202111058396; 18.03.2022 EP 22163082
(43) Date of publication of application: 23.10.2024
(73) Proprietor: Syngenta Crop Protection AG, 4058 Basel (CH)
(72) Inventor: EDMUNDS, Andrew, 4332 Stein (CH); SCARBOROUGH, Christopher Charles, 4332 Stein (CH); MAHAJAN, Atul, Corlim Ilhas, Goa 403 110 (IN); LAMBERTH, Clemens, 4332 Stein (CH); STIERLI, Daniel, 4332 Stein (CH); GROSHEVA, Daria, 4332 Stein (CH); PINSON, Benjamin, 4332 Stein (CH)
(74) Representative: SYNGENTA IP
(86) International application number: PCT/EP2022/085617
(87) International publication number: WO 2023/110869

(56) References cited:
- WO-A1-2019/141957

## Description

The present invention relates to microbiocidal bicycle heterocyclic derivatives, e.g. as active ingredients, which have microbiocidal activity, in particular fungicidal activity. The invention also relates to preparation of these bicycle heterocyclic derivatives, to intermediates useful in the preparation of these bicycle heterocyclic derivatives, to the preparation of these intermediates, to agrochemical compositions which comprise at least one of the bicycle heterocyclic derivatives, to preparation of these compositions and to the use of the bicycle heterocyclic derivatives or compositions in agriculture or horticulture for controlling or preventing infestation of plants, harvested food crops, seeds or non-living materials by phytopathogenic microorganisms, in particular fungi. WO2019/141957 discloses N-pyrimidinyl hydroxy pyrazole compounds, which have been found to be potent inhibitors of fungal growth.

According to a first aspect of the present invention, there is provided a compound of formula (I): 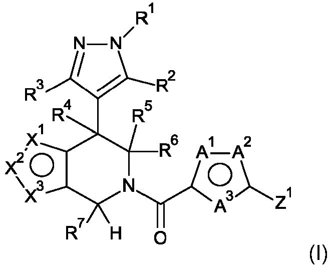 wherein
R¹ is selected from the group consisting of hydrogen, C₁-C₄ alkyl, C₂-C₄ alkenyl, C₂-C₄ alkynyl and C₃-C₆ cycloalkyl;
R² is selected from the group consisting of hydrogen, halogen, C₁-C₄ alkyl, C₂-C₄ alkenyl, C₂-C₄ alkynyl, C₁-C₄ haloalkyl, C₃-C₆ cycloalkyl, C₁-C₄ alkylcarbonyl, N-C₁-C₄ alkoxy-C-C₁-C₄ alkyl-carbonimidoyl, N-hydroxy-C-C₁-C₄ alkyl-carbonimidoyl and C₁-C₄ alkoxycarbonyl;
R³ is selected from the group consisting of hydrogen, halogen and C₁-C₄ alkyl;
R⁴ is selected from the group consisting of hydrogen, halogen and C₁-C₄ alkyl;
R⁵ and R⁶ are independently selected from the group consisting of hydrogen and C₁-C₄ alkyl;
R⁷ is selected from the group consisting of hydrogen, C₁-C₄ alkyl, C₁-C₄ alkylcarbonyl, N-C₁-C₄ alkoxy-C-C₁-C₄ alkyl-carbonimidoyl, N-hydroxy-C-C₁-C₄ alkyl-carbonimidoyl, C₁-C₄ alkoxycarbonyl, N-methoxy-N-methyl-carbonyl, C₁-C₄ alkylaminocarbonyl, di(C₁-C₄ alkylamino)carbonyl, phenyl, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆-cycloalkyl are optionally substituted by 1, 2 or 3 substituents independently selected from halogen, cyano, C₁-C₄ alkyl, C₁-C₄ haloalkyl or C₁-C₄ alkoxy;
Z¹ is selected from the group consisting of C₁-C₄ alkyl, phenyl, 5- or 6-membered heteroaryl and C₃-C₆-cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆-cycloalkyl are optionally substituted by 1, 2 or 3 substituents independently selected from halogen, cyano, C₁-C₄ alkyl, C₁-C₄ haloalkyl, C₁-C₄ alkoxy, C₁-C₄ haloalkoxy, C₁-C₄ alkylsulfanyl, C₁-C₄ alkylsulfinyl, C₁-C₄ alkylsulfonyl or C₂-C₄ alkynyl;
X¹, X² and X³ are independently selected from the group consisting of CR⁸, N and S, with the proviso that one of X¹, X² and X³ is S;
R⁸ is selected from the group consisting of hydrogen, halogen, and C₁-C₄ alkyl;
A¹, A² and A³ are independently selected from the group consisting of CR⁹, N, NR¹⁰, O and S, with the proviso that at least one of A¹, A² and A³ is selected from N, O and S, and that no more than one of A¹, A² and A³ is O or S;
R⁹ is selected from the group consisting of hydrogen, C₁-C₄ alkyl, C₂-C₄ alkenyl and C₂-C₄ alkynyl;
R¹⁰ is selected from the group consisting of hydrogen, C₁-C₄ alkyl, C₂-C₄ alkenyl and C₂-C₄ alkynyl; or an agrochemically acceptable salt, stereoisomer, enantiomer, tautomer or N-oxide thereof.

Surprisingly, it has been found that the compounds of formula (I) have, for practical purposes, a very advantageous level of biological activity for protecting plants against diseases that are caused by fungi.

According to a second aspect of the invention, there is provided an agrochemical composition comprising a fungicidally effective amount of a compound of formula (I) according to the invention. Such an agricultural composition may further comprise at least one additional active ingredient and/or an agrochemically-acceptable diluent or carrier.

According to a third aspect of the invention, there is provided a method of controlling or preventing infestation of useful plants by phytopathogenic microorganisms, wherein a fungicidally effective amount of a compound of formula (I) according to the invention, or a composition comprising the compound of formula (I), is applied to the plants, to parts thereof or the locus thereof.

According to a fourth aspect of the invention, there is provided the use of a compound of formula (I) according to the invention as a fungicide. According to this particular aspect of the invention, the use may exclude methods for the treatment of the human or animal body by surgery or therapy.

As used herein, the term "halogen" or "halo" refers to fluorine (fluoro), chlorine (chloro), bromine (bromo) or iodine (iodo), preferably fluorine, chlorine or bromine.

As used herein, cyano means a -CN group.

As used herein, the term "hydroxyl" or "hydroxy" means an -OH group.

As used herein, oxo means an =O group, e.g., sulfinyl (-S(O)-) or sulfonyl (-S(O)₂-) oxygen.

As used herein, the term "C₁-C₄ alkyl" refers to a straight or branched hydrocarbon chain radical consisting solely of carbon and hydrogen atoms, containing no unsaturation, having from one to four carbon atoms, and which is attached to the rest of the molecule by a single bond. The terms "C₁-C₃ alkyl" and "C₁-C₂ alkyl" are to be construed accordingly. Examples of C₁-C₄alkyl include, but are not limited to, methyl, ethyl, *n-*propyl, 1-methylethyl (isopropyl), n-butyl, and 1,1-dimethylethyl (t-butyl). A "C₁-C₄ alkylene" group refers to the corresponding definition of C₁-C₄alkyl, except that such radical is attached to the rest of the molecule by two single bonds. Examples of C₁-C₄ alkylene, are -CH₂- and -CH₂CH₂-.

As used herein, the term "C₂-C₄ alkenyl" refers to a straight or branched hydrocarbon chain radical group consisting solely of carbon and hydrogen atoms, containing at least one double bond that can be of either the (E) or (Z) configuration, having from two to four carbon atoms, which is attached to the rest of the molecule by a single bond. The term "C₃-C₄ alkenyl" is to be construed accordingly. Examples of C₂-C₄ alkenyl include, but are not limited to, ethenyl and prop-1-enyl.

As used herein, the term "C₂-C₄ alkynyl" refers to a straight or branched hydrocarbon chain radical group consisting solely of carbon and hydrogen atoms, containing at least one triple bond, having from two to four carbon atoms, and which is attached to the rest of the molecule by a single bond. The term "C₃-C₄ alkynyl" is to be construed accordingly. Examples of C₃-C₄ alkynyl include, but are not limited to, ethynyl, prop-1-ynyl, propargyl (prop-2-ynyl), but-1-ynyl and 3-methyl-but-1-ynyl.

As used herein, the term "C₁-Cₙ-haloalkyl" refers to a straight-chain or branched saturated alkyl radical attached via any of the carbon atoms having 1 to n carbon atoms (as mentioned above), where some or all of the hydrogen atoms in these radicals may be replaced by fluorine, chlorine, bromine and/or iodine, i.e., for example, any one of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3- pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. According a term "C₁-C₂fluoroalkyl" would refer to a C₁-C₂alkyl radical which carries 1, 2, 3, 4, or 5 fluorine atoms, for example, any one of difluoromethyl, trifluoromethyl, 1-fluoroethyl, 2-fluoroethyl, 2,2-difluoroethyl, 2,2,2-trifluoroethyl, 1,1,2,2-tetrafluoroethyl or pentafluoroethyl. Similarly "C₁-Cₙ-haloalkoxy" as used herein refers to a C₁-Cₙ-alkoxyl radical respectively substituted with one or more halo atoms which may be the same or different.

As used herein, the term "C₁-C₄ alkoxy" refers to a radical of the formula RₐO- where Rₐ is a C₁-C₄ alkyl radical as generally defined above. The terms "C₁-C₃ alkoxy" and "C₁-C₂ alkoxy" are to be construed accordingly. Examples of C₁-C₄ alkoxy include, but are not limited to, methoxy, ethoxy, propoxy, iso-propoxy, and t-butoxy.

As used herein, the term "C₁-C₄ alkoxy-C₁-C₄ alkyl" refers to radical of the formula R_{b}-O-Rₐ- where R_{b} is a C₁-C₄ alkyl radical as generally defined above, and Rₐ is a C₁-C₄ alkylene radical as generally defined above.

As used herein, the term "C₁-C₄ alkylcarbonyl" refers to a radical of the formula -C(O)Rₐ where Rₐ is a C₁-C₄ alkyl radical as generally defined above.

As used herein, the term "C₁-C₄ alkoxycarbonyl" refers to a radical of the formula -C(O)ORₐ where Rₐ is a C₁-C₄ alkyl radical as generally defined above.

As used herein, the term "C₁-C₄ alkylaminocarbonyl" refers to a radical of the formula -C(O)NHRₐ where Rₐ is a C₁-C₄alkyl radical as generally defined above.

As used herein, the term "di(C₁-C₄ alkylamino)carbonyl" refers to a radical of the formula -C(O)NRₐ(Rₐ) where each Rₐ is a C₁-C₄alkyl radical, which may be the same or different, as generally defined above.

As used herein, the term "C₂-C₄ alkenyloxy" refers to a radical of the formula -ORₐ where Rₐ is a C₂-C₄ alkenyl radical as generally defined above.

As used herein, the term "C₂-C₄ alkynyloxy" refers to a radical of the formula -ORₐ where Rₐ is a C₂-C₄ alkynyl radical as generally defined above.

As used herein, the term "C₁-Cₙ-alkylthio" or "C₁-Cₙ-alkylsulfanyl"refers to a C₁-Cₙ-alkyl group linked through a sulfur atom.

As used herein, the term "C₁-Cₙ-alkylsulfinyl"refers to a C₁-Cₙalkyl group linked through the sulfur atom of a sulfinyl (or S(=O)-) group.

As used herein, the term "C₁-Cₙ-alkylsulfonyl"refers to a C₁-Cₙalkyl group linked through the sulfur atom of a sulfonyl (or S(=O)₂-) group.

As used herein, the term "N-C₁-C₄ alkoxy-C-C₁-C₄ alkyl-carbonimidoyl" refers to a radical of the formula - C(Rₐ)=NO(R_{b}) where Rₐ is a C₁-C₄ alkyl radical as generally defined above, and R_{b} is a C₁-C₄ alkyl radical as generally defined above.

As used herein the term "N-hydroxy-C-C₁-C₄ alkyl-carbonimidoyl" refers to a radical of the formula - C(Rₐ)=NOH where Rₐ is a C₁-C₄ alkyl radical as generally defined above.

As used herein, the term "C₃-C₆ cycloalkyl" refers to a stable, monocyclic ring radical which is saturated or partially unsaturated and contains 3 to 6 carbon atoms. The terms "C₃-C₄ cycloalkyl" and "C₃-C₅cycloalkyl" are to be construed accordingly. Examples of C₃-C₆ cycloalkyl include, but are not limited to, cyclopropyl, cyclobutyl, cyclopentyl, cyclohexyl, cyclopenten-1-yl, cyclopenten-3-yl, and cyclohexen-3-yl.

As used herein, the term "C₃-C₆ cycloalkylC₁-C₄alkyl" refers to a C₃-C₆ cycloalkyl ring as defined above attached to the rest of the molecule by a C₁-C₄alkylene radical as defined above. Examples of C₃-C₆cycloalkylC₁-C₄alkyl include, but are not limited to, cyclopropyl-methyl, cyclobutyl-ethyl, and cyclopentylmethyl.

Examples of a 5- or 6-membered heteroaryl ring, which comprise 1 to 4 heteroatoms independently selected from nitrogen, oxygen and sulfur, include, but are not limited to, pyridyl, pyrimidyl, pyrrolyl, pyrazolyl, furyl, thienyl, imidazolyl, isoxazolyl, oxazolyl, thiazolyl, isothiazolyl, triazolyl, oxadiazolyl, thiadiazolyl, tetrazolyl, pyrazinyl, pyridazinyl and triazinyl.

The compounds of formula (I) or the intermediate compounds of formula (III) and (IV) according to the invention, which have at least one basic centre, can form, for example, acid addition salts, for example with strong inorganic acids such as mineral acids, for example perchloric acid, sulfuric acid, nitric acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄-alkane- or arylsulfonic acids which are unsubstituted or substituted, for example by halogen, for example methane- or p-toluenesulfonic acid.

The compounds of formula (I) or the intermediate compounds of formula (III) and (IV) according to the invention, which have at least one acidic group, can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

The presence of one or more possible asymmetric carbon atoms in a compound of formula (I) according to the invention means that the compounds may occur in chiral isomeric forms, i.e., enantiomeric or diastereomeric forms. Also, atropisomers may occur as a result of restricted rotation about a single bond. Formula (I) is intended to include all those possible isomeric forms and mixtures thereof. The present invention includes all those possible isomeric forms and mixtures thereof for a compound of formula (I) according to the invention. Likewise, a compound of formula (I) is intended to include all possible tautomers (including lactam-lactim tautomerism and keto-enol tautomerism) where present. The present invention includes all possible tautomeric forms for a compound of formula (I) according to the invention.

In each case, the compounds of formula (I) according to the invention are in free form, in oxidized form as an N-oxide, in covalently hydrated form, or in salt form, e.g., an agronomically usable or agrochemically acceptable salt form. N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991. The compounds of formula (I) according to the invention also include hydrates, which may be formed during salt formation.

The following list provides definitions, including preferred definitions, for substituents R¹, R², R³, R⁴, R⁵, R⁶, R⁷, R⁸, R⁹, R¹⁰, Z¹, A¹, A², A³, X¹, X² and X³ with reference to the compounds of formula (I) of the present invention. For any one of these substituents, any of the definitions given below may be combined with any definition of any other substituent given below or elsewhere in this document.

In an embodiment of the invention, R¹ is hydrogen, C₁-C₄ alkyl or C₂-C₄ alkynyl. Preferably, R¹ is hydrogen, methyl, ethyl or isopropyl. More preferably, R¹ is methyl.

In an embodiment of the invention, R² is selected from the group consisting of hydrogen, halogen, C₁-C₄ alkyl, C₃-C₆cycloalkyl, C₁-C₄ alkylcarbonyl, N-C₁-C₄ alkoxy-C-C₁-C₄ alkyl-carbonimidoyl and N-hydroxy-C-C₁-C₄ alkyl-carbonimidoyl. Preferably, R² is selected from the group consisting of hydrogen, halogen, methyl, ethyl, cyclopropyl, C₁-C₂ alkylcarbonyl, N-C₁-C₂ alkoxy-C-C₁-C₂ alkyl-carbonimidoyl and N-hydroxy-C-C₁-C₂ alkyl-carbonimidoyl. More preferably, R² is selected from the group consisting of hydrogen, fluorine, chlorine, bromine, methyl, ethyl, cyclopropyl, acetyl, -C(CH₃)=NOCH₃, -C(CH₃)=NOCH₂CH₃ and - C(CH₃)=NOH. Even more preferably, R² is selected from the group consisting of hydrogen, fluorine, chlorine and methyl.

In an embodiment of the invention, R³ is selected from the group consisting of hydrogen, halogen and C₁-C₄ alkyl. Preferably, R³ is selected from the group consisting of hydrogen, fluorine, chlorine, methyl and ethyl. More preferably, R³ is selected from the group consisting of hydrogen and methyl.

In an embodiment of the invention, R⁴ is selected from the group consisting of hydrogen, halogen and C₁-C₄ alkyl. Preferably, R⁴ is selected from the group consisting of hydrogen, fluorine, chlorine, methyl and ethyl. More preferably, R⁴ is selected from the group consisting of hydrogen and methyl.

In an embodiment of the invention, each of R⁵ and R⁶ is independently selected from the group consisting of hydrogen, methyl and ethyl.

In another embodiment of the invention, R⁷ is selected from the group consisting of hydrogen, C₁-C₄alkyl, C₁-C₄ alkylcarbonyl, N-C₁-C₄ alkoxy-C-C₁-C₄ alkyl-carbonimidoyl, N-hydroxy-C-C₁-C₄ alkyl-carbonimidoyl, C₁-C₄ alkoxycarbonyl, N-methoxy-N-methyl-carbonyl, phenyl, 4-cyanophenyl, cyclopropyl and 1-cyanocyclopropyl. Preferably, R⁷ is selected from the group consisting of hydrogen, methyl, acetyl, - C(CH₃)=NOCH₃, -C(CH₃)=NOCH₂CH₃, -C(CH₃)=NOH, methoxycarbonyl, ethoxycarbonyl, N-methoxy-N-methyl-carbonyl, phenyl and cyclopropyl. More preferably, R⁷ is selected from the group consisting of hydrogen and methyl.

In an embodiment of the invention, Z¹ is selected from the group consisting of C₁-C₄ alkyl, phenyl, 5- or 6-membered heteroaryl and C₃-C₆-cycloalkyl, wherein the 5- or 6-membered heteroaryl comprises 1, 2, 3 or 4 heteroatoms individually selected from N, O and S, and wherein said phenyl and 5- or 6-membered heteroaryl are optionally substituted by 1, 2 or 3 substituents independently selected from halogen, cyano, C₁-C₄ alkyl, C₁-C₄ haloalkyl, C₁-C₄ alkoxy, C₁-C₄ haloalkoxy, C₁-C₄ alkylsulfanyl, C₁-C₄ alkylsulfinyl or C₁-C₄ alkylsulfonyl, and wherein C₃-C₆-cycloalkyl are optionally substituted by 1, 2 or 3 substituents independently selected from halogen, cyano, C₁-C₄ alkyl, C₁-C₄ haloalkyl or C₁-C₄ alkoxy. Preferably, Z¹ is selected from the group consisting of methyl, n-propyl, cyclopropyl, 1-methylcyclopropyl, cyclobutyl, cyclopentyl, cyclohexyl, phenyl, 2-methylphenyl, 3-methylphenyl, 4-methylphenyl, 2-fluorophenyl, 3-fluorophenyl, 4-fluorophenyl, 2-chlorophenyl, 3-chlorophenyl, 4-chlorophenyl, 3-methoxyphenyl, 4-ethynyl-2-fluoro-phenyl, 4-fluoro-2-methoxy-phenyl, 2,3-difluorophenyl, 2,4-difluorophenyl, 2,5-difluorophenyl, 3,4-difluorophenyl, 2,3,4-trifluorophenyl, 2,4,6-trifluorophenyl, 2-fluoro-4-methoxy-phenyl, 2-fluoro-4-methylsulfonyl-phenyl, 2-furyl, 2-thienyl, 3-thienyl and 1-methylpyrazol-4-yl. More preferably, Z¹ is selected from the group consisting of methyl, cyclobutyl, cyclopentyl, cyclohexyl, 2-methylphenyl, 2-fluorophenyl, 4-fluorophenyl, 3-chlorophenyl, 4-fluoro-2-methoxy-phenyl, 2,4-difluorophenyl, 3,4-difluorophenyl, 2,4,6-trifluorophenyl, 2-furyl, 2-thienyl, 3-thienyl and 1-methylpyrazol-4-yl. Still more preferably, Z¹ is selected from the group consisting of methyl, cyclobutyl, cyclopentyl, cyclohexyl, 2-methylphenyl, 2-fluorophenyl, 4-fluorophenyl, 3-chlorophenyl, 4-fluoro-2-methoxy-phenyl, 2,4-difluorophenyl, 3,4-difluorophenyl, 2,4,6-trifluorophenyl, 3,5-difluoro-2-pyridyl, 2-furyl, 2-thienyl, 3-thienyl and 1-methylpyrazol-4-yl. Even more preferably, Z¹ is selected from the group consisting of phenyl, 2-fluorophenyl, 4-fluorophenyl and 2,4-difluorophenyl. Still even more preferably Z¹ is selected from 2,4-difluorophenyl, 3,5-difluoro-2-pyridyl, 2-fluorophenyl, 4-fluorophenyl or phenyl.

In another embodimnent Z¹ is selected from 1-methylpyrazol-4-yl, 2,3,4-trifluorophenyl, 2,3-difluorophenyl, 3,4-difluorophenyl, 2,4,6-trifluorophenyl, 2,4-difluorophenyl, 2,5-difluorophenyl, 3,5-difluoro-2-pyridyl, 5-fluoro-2-pyridyl, 3-fluoro-2-pyridyl, 2-fluoro-4-methoxy-phenyl, 2-fluoro-4-methylsulfonyl-phenyl, 2-fluorophenyl, 3-fluorophenyl, 4-fluorophenyl, 3,5-difluoro-2-furyl, 3-fluoro-2-furyl, 5-fluoro-2-furyl, 3,5-difluoro-2-thienyl, 3-fluoro-2-thienyl, 5-fluoro-2-thienyl, 2-methylphenyl, 3-methylphenyl, 4-methylphenyl, 2-chlorophenyl, 3-chlorophenyl, 4-chlorophenyl, 3-methoxyphenyl, 4-ethynyl-2-fluoro-phenyl, 4-fluoro-2-methoxy-phenyl, cyclopropyl, 1-methylcyclopropyl, cyclobutyl, cyclohexyl, cyclopentyl, methyl, n-propyl or phenyl. More preferably Z¹ is selected from 1-methylpyrazol-4-yl, 2,4,6-trifluorophenyl, 2,4-difluorophenyl, 2,5-difluorophenyl, 2-chlorophenyl, 2-fluorophenyl, 3,5-difluoro-2-pyridyl, 2-furyl, 2-methylphenyl, 2-thienyl, 3,4-difluorophenyl, 3-chlorophenyl, 3-fluorophenyl, 3-methylphenyl, 3-thienyl, 4-fluoro-2-methoxy-phenyl, 4-fluorophenyl, 4-methylphenyl, cyclobutyl, cyclohexyl, cyclopentyl, methyl or phenyl. Even more preferably Z¹ is selected from 1-methylpyrazol-4-yl, 2,4,6-trifluorophenyl, 3,5-difluoro-2-pyridyl, 2,4-difluorophenyl, 2-fluorophenyl, 2-furyl, 2-methylphenyl, 2-thienyl, 3,4-difluorophenyl, 3-chlorophenyl, 3-thienyl, 4-fluoro-2-methoxy-phenyl, 4-fluorophenyl, cyclobutyl, cyclohexyl, cyclopentyl or phenyl. Still more preferably, Z¹ is selected from the group consisting of methyl, cyclobutyl, cyclopentyl, cyclohexyl, 2-methylphenyl, 2-fluorophenyl, 4-fluorophenyl, 3-chlorophenyl, 4-fluoro-2-methoxy-phenyl, 2,4-difluorophenyl, 3,4-difluorophenyl, 2,4,6-trifluorophenyl, 3,5-difluoro-2-pyridyl, 2-furyl, 2-thienyl, 3-thienyl and 1-methylpyrazol-4-yl. Still even more preferably Z¹ is selected from 2,4-difluorophenyl, 3,5-difluoro-2-pyridyl, 2-fluorophenyl, 4-fluorophenyl or phenyl.

In an embodiment of the invention, X¹ is S, and X² and X³ are independently selected from the group consisting of CR⁸ and N. In another embodiment of the invention, X² is S, and X¹ and X³ are independently selected from the group consisting of CR⁸ and N. In another embodiment of the invention, X³ is S, and X¹ and X² are independently selected from the group consisting of CR⁸ and N.

In an embodiment of the invention, R⁸ is selected from the group consisting of hydrogen, chlorine, bromine, methyl and ethyl. Preferably, R⁸ is selected from the group consisting of hydrogen, chlorine and methyl. More preferably, R⁸ is hydrogen.

In an embodiment of the invention, A¹, A² and A³ are independently selected from the group consisting of CR⁹, N, O and S, with the proviso that at least one of A¹, A² and A³ is selected from N, O and S, and that no more than one of A¹, A² and A³ is O or S.

In an embodiment of the invention, R⁹ is hydrogen or C₁-C₄ alkyl, preferably hydrogen or methyl.

In an embodiment of the invention, R¹⁰ is hydrogen or C₁-C₄ alkyl, preferably hydrogen or methyl.

In an embodiment of the invention, the compound of formula (I) may be a compound of formula (I-A): 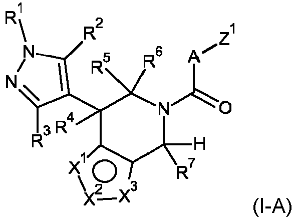 wherein
R¹, R², R³, R⁴, R⁵, R⁶, R⁷, X¹, X², X³ and Z¹ are as defined for the compounds of formula (I) according to the present invention, and

A is selected from the group consisting of A1 to A36: 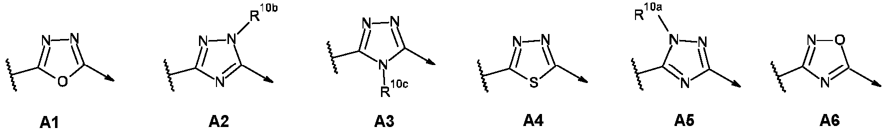 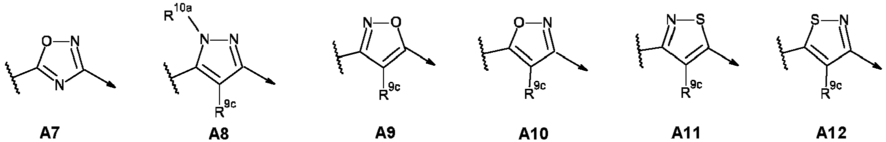 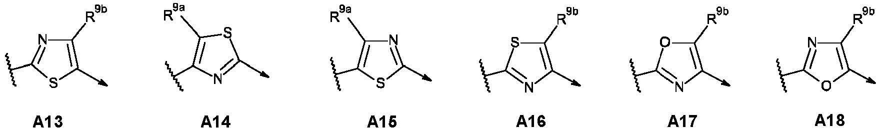 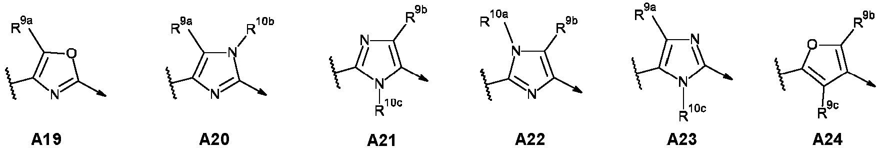 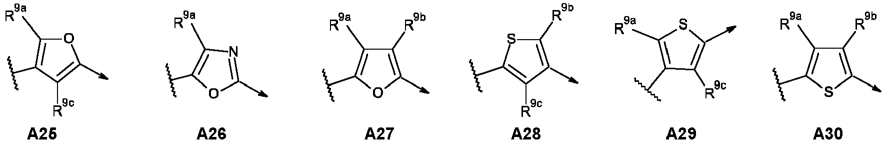 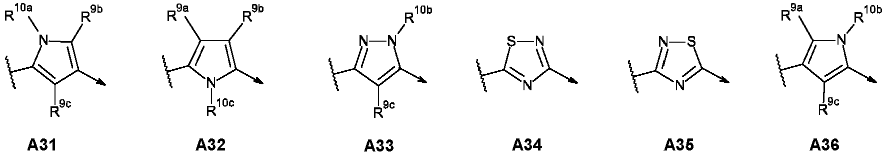 wherein 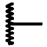 indicates the bond to the C(=O) group and the arrow the bond to the Z¹ group, and R^{9a}, R^{9b}, R^{9c}, R^{10a}, R^{10b} and R^{10c} are independently selected from the group consisting of hydrogen, C₁-C₄ alkyl, C₂-C₄ alkenyl and C₂-C₄ alkynyl.

In an embodiment of the invention, in the compound of formula (I-A) A is selected from the group consisting of A1, A4, A6, A7, A9, A10, A13 and A15: 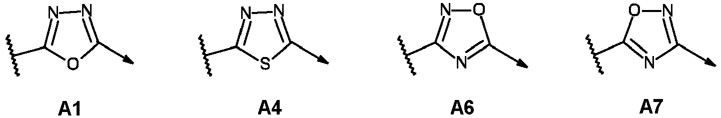 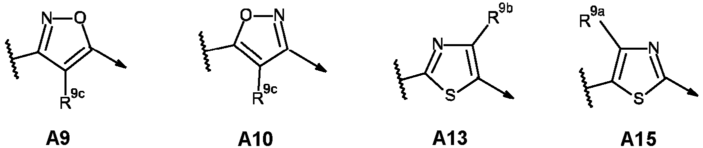 wherein 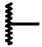 indicates the bond to the C(=O) group and the arrow the bond to the Z¹ group, and R^{9a}, R^{9b} and R^{9c} are independently selected from the group consisting of hydrogen, C₁-C₄ alkyl, C₂-C₄ alkenyl and C₂-C₄ alkynyl.

In another embodiment of the invention, in the compound of formula (I-A) A is selected from the group consisting of A1, A4, A9, A10, A13 and A15: 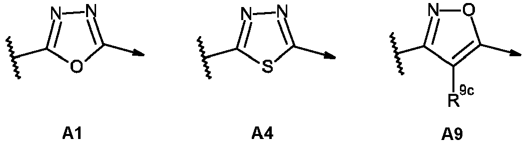 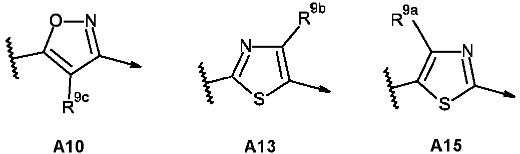 wherein 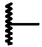 indicates the bond to the C(=O) group and the arrow the bond to the Z¹ group, wherein R^{9a}, R^{9b} and R^{9c} are hydrogen.

In another embodiment of the invention, in the compound of formula (I-A) A is selected from the group consisting of A1, A4, A9 and A10: 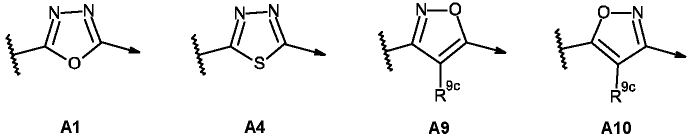 wherein 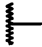 indicates the bond to the C(=O) group and the arrow the bond to the Z¹ group, wherein R^{9c} is hydrogen.

In another embodiment of the invention, in the compound of formula (I-A) A is selected from the group consisting of A4, A6, A9 and A10: 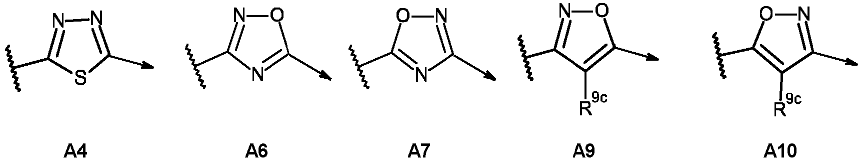 wherein 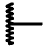 indicates the bond to the C(=O) group and the arrow the bond to the Z¹ group.

Preferably in one embodiment of the invention, in the compound of formula (I-A) A is selected from the group consisting of A4, A6, A9 and A10, and R^{9c} is hydrogen.

In another embodiment of the invention, in the compound of formula (I-A) A is selected from the group consisting of A4, A9 and A10: 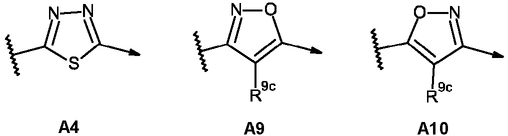 wherein 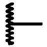 indicates the bond to the C(=O) group and the arrow the bond to the Z¹ group.

Preferably in one embodiment of the invention, in the compound of formula (I-A) A is selected from the group consisting of A4, A9 and A10, and R^{9c} is hydrogen.

In another embodiment of the invention, in the compound of formula (II) A is selected from the group consisting of A4, A7 and A9, 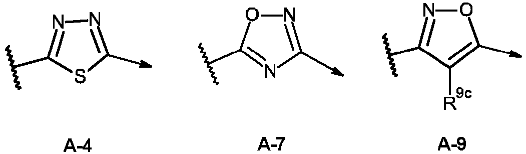 wherein 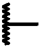 indicates the bond to the C(=O) group and the arrow the bond to the Z¹ group, and R^{7c} is hydrogen.

More preferably in the compound of formula (II) A is selected from the group consisting of A4, A7 and A9 and R^{7e} is hydrogen.

More preferably in the compound of formula (II) A is A9 and R^{7c} is hydrogen.

In an embodiment of the invention, R^{9a}, R^{9b}, R^{9c}, R^{10a}, R^{10b} and R^{10c} are independently hydrogen or methyl.

In another embodiment of the invention, R^{9a}, R^{9b}, R^{9c}, R^{10a}, R^{10b} and R^{10c} are hydrogen.

In another embodiment of the invention, R^{9a}, R^{9b}, R^{9c}, R^{10a}, R^{10b} and R^{10c} are methyl.

In an embodiment of the invention, the compound of formula (I) may be a compound of formula (II-A) wherein X¹ is S: 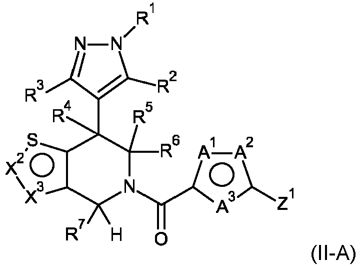 wherein X² and X³ are independently selected from the group consisting of CR⁸ and N, and R', R², R³, R⁴, R⁵, R⁶, R⁷, R⁸, A¹, A², A³ and Z¹ are as defined for the compounds of formula (I) according to the present invention.

Preferably, in the compound of formula (II-A) of the invention,
X² is CR⁸ and X³ is CR⁸, or
X² is N and X³ is CR⁸, or
X² is CR⁸ and X³ is N, or
X² is N and X³ is N, and
R¹, R², R³, R⁴, R⁵, R⁶, R⁷, R⁸, A¹, A², A³ and Z¹ are as defined for the compounds of formula (I) according to the present invention.

In another embodiment of the invention, the compound of formula (I) may be a compound of formula (II-B) wherein X² is S: 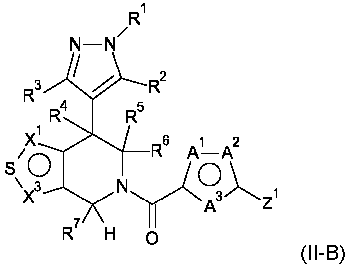 wherein X¹ and X³ are independently selected from the group consisting of CR⁸ and N, and R¹, R², R³, R⁴, R⁵, R⁶, R⁷, R⁸, A¹, A², A³ and Z¹ are as defined for the compounds of formula (I) according to the present invention.

Preferably, in the compound of formula (II-B) of the invention,
X¹ is CR⁸ and X³ is CR⁸, or
X¹ is N and X³ is CR⁸, or
X¹ is CR⁸ and X³ is N, or
X¹ is N and X³ is N, and
R¹, R², R³, R⁴, R⁵, R⁶, R⁷, R⁸, A¹, A², A³ and Z¹ are as defined for the compounds of formula (I) according to the present invention.

In another embodiment of the invention, the compound of formula (I) may be a compound of formula (II-C) wherein X³ is S: 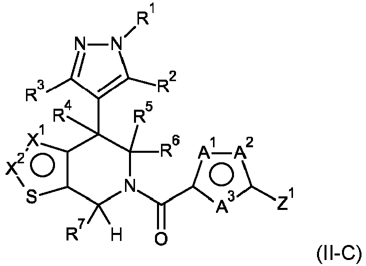 wherein X¹ and X² are independently selected from the group consisting of CR⁸ and N, and R¹, R², R³, R⁴, R⁵, R⁶, R⁷, R⁸, A¹, A², A³ and Z¹ are as defined for the compounds of formula (I) according to the present invention.

Preferably, in the compound of formula (II-C) of the invention,
X¹ is CR⁸ and X² is CR⁸, or
X¹ is N and X² is CR⁸, or
X¹ is CR⁸ and X² is N, or
X¹ is N and X² is N, and
R¹, R², R³, R⁴, R⁵, R⁶, R⁷, R⁸, A¹, A², A³ and Z¹ are as defined for the compounds of formula (I) according to the present invention.

More preferably, the compound of formula (I) is selected from compounds A.01, A.02, A.03, A.04, A.05, A.06, A.07, A.08, A.09, A.10, A.11 and A.12 as defined in Table A below, wherein R¹, R², R³, R⁴, R⁵, R⁶, R⁷, R⁸, A¹, A², A³ and Z¹ are as defined for the compounds of formula (I) according to the present invention.

**Table A**

| **Compound No.** | **Compound structure** | **Compound No.** | **Compound structure** |
|---|---|---|---|
| A.01 | 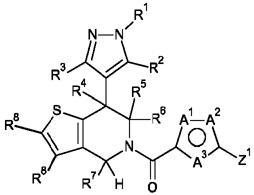 | A.07 | 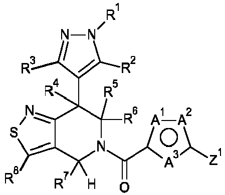 |
| A.02 | 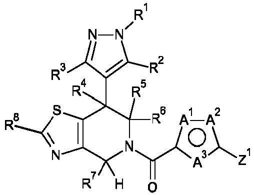 | A.08 | 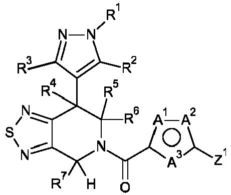 |
| A.03 | 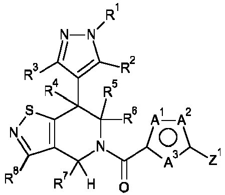 | A.09 | 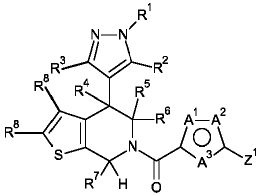 |
| A.04 | 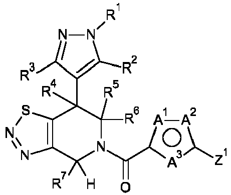 | A.10 | 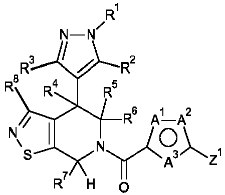 |
| A.05 | 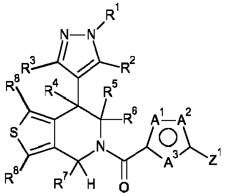 | A.11 | 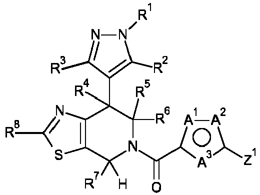 |
| A.06 | 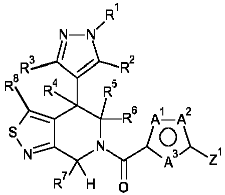 | A.12 | 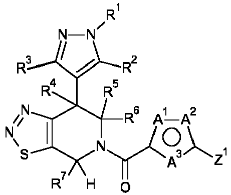 |

Even more preferably, the compound of formula (I) is selected from compounds A.01, A.02, A.03, A.04, A.05, A.06, A.07, A.08, A.09, A.10, A.11 and A.12 as defined in Table A above, wherein R⁸ is selected from the group consisting of hydrogen, chlorine, bromine, methyl and ethyl, preferably R⁸ is selected from the group consisting of hydrogen, chlorine and methyl, more preferably R⁸ is hydrogen, and R¹, R², R³, R⁴, R⁵, R⁶, R⁷, A¹, A², A³ and Z¹ are as defined for the compounds of formula (I) according to the present invention.

The presence of one or more possible asymmetric carbon atoms in any of the compounds of formula (I), (I-A), (II-A), (II-B) and (II-C) according to the invention means that the compounds may occur in chiral isomeric forms, i.e., enantiomeric or diastereomeric forms.

More preferably, the compound of formula (I) according to the invention is selected from compounds listed in any one of Tables B-1 to B-10, C-1 to C-102 or from compounds (P-1) to (P-39) listed in Table T1.

Even more preferably, the compound of formula (I) according to the invention is selected from compounds (P-1) to (P-39) listed in Table T1.

According to a fifth aspect of the invention, there is provided an intermediate compound of formula (III) or a salt thereof: 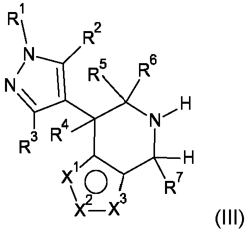 wherein R¹, R², R³, R⁴, R⁵, R⁶, R⁷, X¹, X² and X³ correspond to the same definitions as for the compounds of formula (I) according to the present invention.

The intermediate compounds of formula (III) possess the same definitions for R¹, R², R³, R⁴, R⁵, R⁶, R⁷, X¹, X² and X³ as for the compounds of formula (I) according to the invention and their corresponding preferences.

The presence of one or more possible asymmetric carbon atoms in a compound of formula (III) according to the invention means that the compounds may occur in chiral isomeric forms, i.e., enantiomeric or diastereomeric forms.

According to a sixth aspect of the invention, there is provided an intermediate compound of formula (IV): 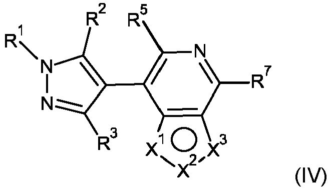 wherein R¹, R², R³, R⁵, R⁷, X¹, X² and X³ correspond to the same definitions as for the compounds of formula (I) according to the present invention.

The intermediate compounds of formula (IV) possess the same definitions for R¹, R², R³, R⁵, R⁷, X¹, X² and X³ as for the compounds of formula (I) according to the invention and their corresponding preferences.

The presence of one or more possible asymmetric carbon atoms in a compound of formula (IV) according to the invention means that the compounds may occur in chiral isomeric forms, i.e., enantiomeric or diastereomeric forms.

The compounds of formula (I) according to the present invention can be made as shown in the following Schemes 1 to 12, in which, unless otherwise stated, the definition of each variable is as defined above for a compound of formula (I).

In particular, compounds of formula (I), wherein R⁴ and R⁶ are hydrogen and R⁵ is hydrogen or methyl, can be made as shown in the following Schemes 1 to 7, in which, unless otherwise stated, the definition of each variable is as defined above for a compound of formula (I).

In any of the Schemes 1 to 12 below, the presence of one or more possible asymmetric carbon atoms in a compound of formula (I) according to the invention means that the compounds may occur in chiral isomeric forms, i.e., enantiomeric or diastereomeric forms.

Compounds of formula (I) may be prepared by a person skilled in the art following known methods. More specifically, compounds of formula (I) may be prepared from compounds of formula (III) or a salt thereof, wherein R¹, R², R³, R⁴, R⁵, R⁶, R⁷, X¹, X² and X³ are as defined above for the compound of formula (I) by reaction with a compound of formula (V), wherein A¹, A², A³ and Z¹ are as defined above for the compound of formula (I). This reaction is shown in Scheme 1.

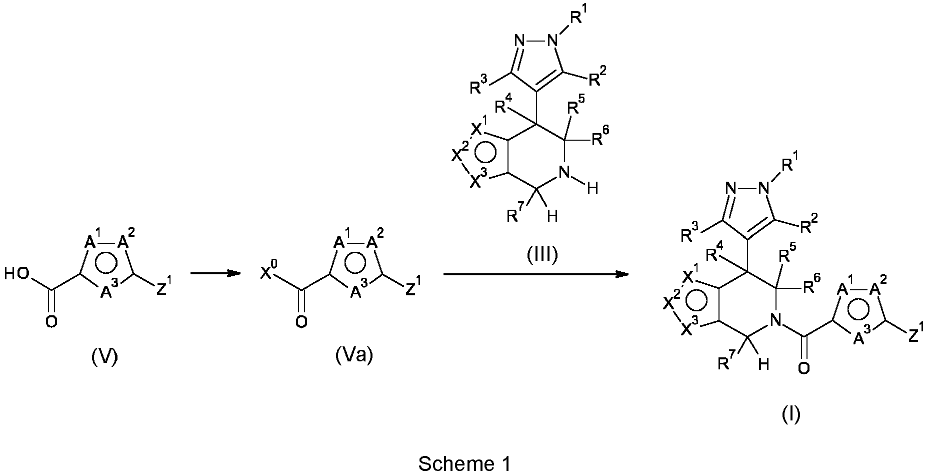

In Scheme 1, compounds of formula (V), wherein A¹, A², A³ and Z¹ are as defined above for the compound of formula (I), are activated to compounds of formula (Va) by methods known to a person skilled in the art and described, for example, in Tetrahedron 2005, 61 (46), 10827-10852. For example, compounds of formula (Va), where X⁰ is halogen, are formed by treatment of compounds of formula (V) with, for example, oxalyl chloride or thionyl chloride in the presence of catalytic quantities of N,N-dimethylformamide (DMF) in inert solvents such as methylene dichloride or tetrahydrofuran (THF) at temperatures from 20°C to 100°C, preferably 25°C. Treatment of compounds of formula (Va) with compounds of formula (III), wherein R¹, R², R³, R⁴, R⁵, R⁶, R⁷, R⁸, X¹, X² and X³ are as defined above for the compound of formula (I), optionally in the presence of a base, e.g. triethylamine or pyridine, leads to compounds of formula (I). Alternatively, compounds of formula (I) may be prepared by treatment of compounds of formula (V) with dicyclohexyl carbodiimide (DCC), 1-ethyl-3-(3-dimethylaminopropyl)carbodiimide (EDC) or 1-[bis(dimethylamino)methylene]-1H-1,2,3-triazolo[4,5-b]pyridinium 3-oxide hexafluorophosphate (HATU) to give the activated compound of formula (Va), wherein X° is X⁰¹, X⁰² or X⁰³ as set forth below, in an inert solvent, e.g. pyridine, DMF, acetonitrile, CH₂Cl₂ or THF, optionally in the presence of a base, e.g. triethylamine, at temperatures from 30°C to 180°C. Finally, a compound of formula (V) can also be activated by reaction with a coupling reagent such as propanephosphonic acid anhydride (T3P) to provide compounds of formula (Va), wherein X° is X⁰⁴ as set forth below, as described for example in Synthesis 2013, 45, 1569. Further reaction with the compound of formula (III) or a salt thereof leads to compounds of formula (I).

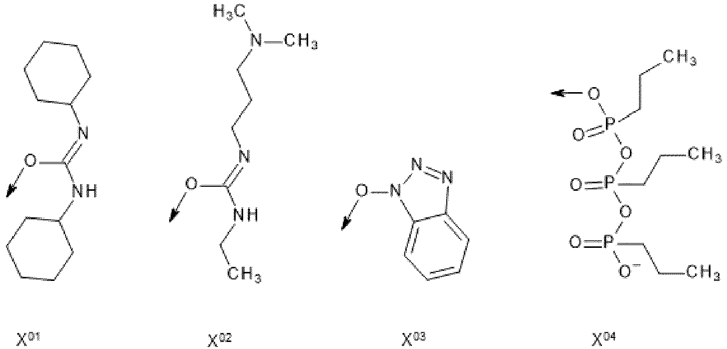

Compounds of formula (Illa), wherein R⁴ and R⁶ are hydrogen, R⁵ is hydrogen or methyl and R¹, R², R³, R⁷, X¹, X² and X³ are as defined above for the compound of formula (I), may be prepared by a person skilled in the art following known methods.

For example, compounds of formula (Illa), wherein R⁴ and R⁶ are hydrogen, R⁵ is hydrogen or methyl and R¹, R², R³, R⁷, X¹, X² and X³ are as defined above for the compound of formula (I), may be prepared from compounds of formula (IVa), wherein R⁵ is hydrogen or methyl, and R¹, R², R³, R⁷, X¹, X² and X³ are as defined above for the compound of formula (I), by treatment with a reducing agent such as NaBH₃CN and an acid, for example hydrochloric acid, or acetic acid in a protic solvent such as methanol or ethanol and the like. Such reactions are well known in the literature and analogous reactions have been described for example in Deng, Zeping et al, CN103772278, and Synthesis 1979, 4, 281-3. Alternatively, compounds of formula (Illa) may be prepared from compounds of formula (IV) by reduction with hydrogen in the presence of a suitable metal catalyst, such as Pd, Ir, Rh with a suitable ligand, e.g. diphosphine [1,2-bis(diphenylphosphino)ethane (dppe), 1,3-bis(diphenylphosphino)propane (dppp) or 1,4-bis(diphenylphosphino)butane (dppb)]. Similar reactions have been reported for example in Reaction Kinetics and Catalysis Letters 2007, 92, 99-104. This reaction is shown in Scheme 2.

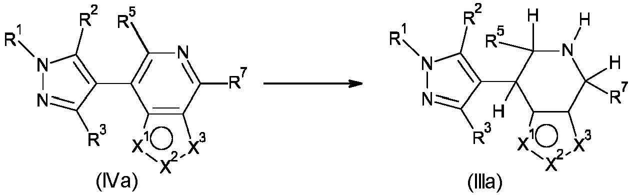

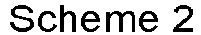

Alternatively, compounds of formula (Illa) may be prepared as shown in Scheme 4.

As shown in Scheme 3, compounds of formula (Illb), wherein R⁴, R⁶ and R⁷ are hydrogen, R⁵ is hydrogen or methyl and R¹, R², R³, X¹, X² and X³ are as defined above for the compound of formula (I), can be converted to compounds of formula (VII), wherein R⁴, R⁶ and R⁷ are hydrogen, R⁵ is hydrogen or methyl and R¹, R², R³, X¹, X² and X³ are as defined above for the compound of formula (I), by treatment with a compound of formula (VI), wherein X⁰ is a leaving group, such as halogen, and R⁰ is C₁-C₄alkyl, by methods known to a person skilled in the art and by those described in Scheme 1. Alternatively, compounds of formula (VII) may be prepared by treatment with an anhydride of formula (R⁰CO)₂O, wherein R⁰ is C₁-C₄ alkyl, in an inert solvent such as methylene chloride, THF or 2-methyl-THF, optionally in the presence of a base, such as triethylamine or dimethylaminopyridine at temperatures between 0°C and 60°C. Compounds of formula (VII) are then metalated with a base, for example an alkyl metal base, such as tert-butyl lithium, and an additive such as *N*,*N*,*N'*,*N'*-tetramethylethylendiamine (TMEDA) at low temperature, for example - 78°C to room temperature, in an inert polar solvent such as THF or 2-methyl-THF. Subsequent treatment of the anion of formula (VII) formed under such conditions with an electrophile of formula R^{X}-X⁰, wherein X⁰ is as previously defined and R^{X} is C₁-C₄ alkyl, C₁-C₄ alkylcarbonyl, C₁-C₄ alkoxycarbonyl, N-methoxy-N-methyl-carbonyl, C₁-C₄ alkylaminocarbonyl, di(C₁-C₄ alkylamino)carbonyl or C₃-C₆ cycloalkyl, wherein the C₃-C₆-cycloalkyl is optionally substituted by 1, 2 or 3 substituents independently selected from halogen, cyano, C₁-C₄ alkyl, C₁-C₄ haloalkyl and C₁-C₄ alkoxy, yields compounds of formula (Vlla), wherein R⁴ and R⁶ are hydrogen, R⁵ is hydrogen or methyl, R⁰ is C₁-C₄ alkyl and R¹, R², R³, R⁷, X¹, X² and X³ are as defined above for the compound of formula (I). This reaction is shown in Scheme 3.

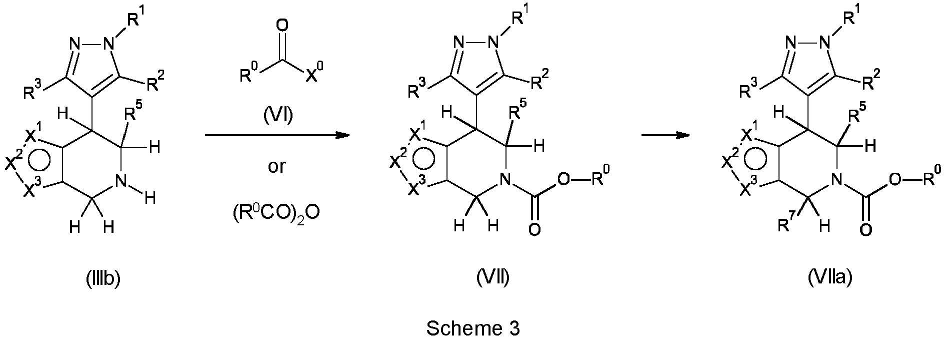

Compounds of formula (Vlla) may be converted to compounds of formula (Illa), wherein R⁴ and R⁶ are hydrogen, R⁵ is hydrogen or methyl and R¹, R², R³, R⁷, X¹, X² and X³ are as defined above for the compound of formula (I), by methods known to a person skilled in the art. For example, compounds of formula (Vlla), wherein R⁰ is tert-butyl, may be treated with an organic or inorganic acid such as trifluoroacetic acid or HCl to give compounds of formula (Illa). This reaction is shown in Scheme 4.

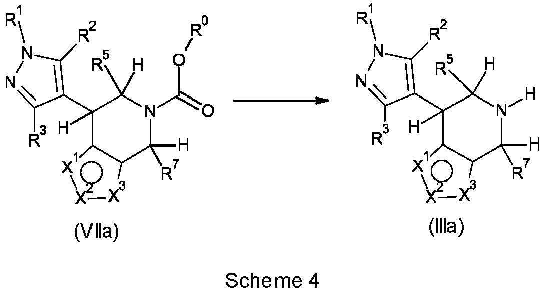

Compounds of formula (IVa), wherein R⁵ is hydrogen or methyl and R¹, R², R³, R⁷, X¹, X² and X³ are as defined above for the compound of formula (I), may be prepared by reacting compounds of formula (IX), wherein R', R² and R³ are as defined above for the compound of formula (I) and X⁰ is halogen, preferably chlorine, bromine or iodine, with compounds of formula (VIII), wherein R⁵ is hydrogen or methyl and R⁷, X¹, X² and X³ are as defined above for the compound of formula (I), by means of a C-C bond formation reaction typically under palladium-catalyzed (alternatively nickel-catalyzed) cross-coupling conditions. This reaction is shown in Scheme 5.

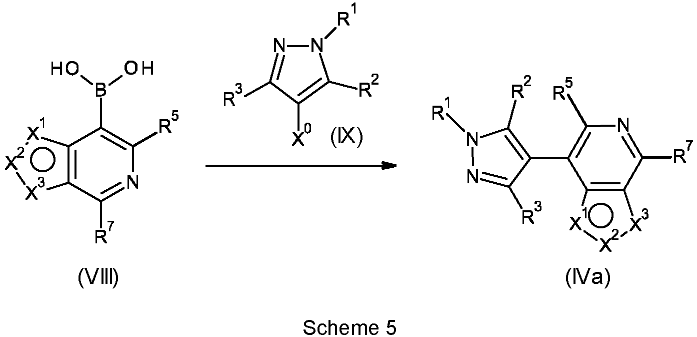

Suzuki-Miyaura cross-coupling reactions between compounds of formula (VIII) and compound of formula (IX) are well known to a person skilled in the art and are usually carried out in the presence of a palladium catalyst, such as tetrakis(triphenylphosphine)-palladium(0) or [1,1'-bis(diphenylphosphino)ferrocene]palladium(II) dichloride dichloromethane complex, and a base, such as sodium or potassium carbonate, in a solvent, such as N,N-dimethylformamide, dioxane or dioxane-water mixtures, at temperatures between room temperature and 160°C, optionally under microwave heating conditions, and preferably under inert atmosphere. Such reactions have been reviewed for example in J. Organomet. Chem. 1999, 576, 147-168. A person skilled in the art will also recognize that the reaction can be reversed, i.e. by reacting a compound of formula (XI), wherein R¹, R² and R³ are as defined above for the compound of formula (I), with a compound of formula (X), wherein R⁵ is hydrogen or methyl, R⁷, X¹, X² and X³ are as defined above for the compound of formula (I) and X⁰ is halogen, preferably chlorine, bromine or iodine, to provide a compound of formula (lVa), wherein R⁵ is hydrogen or methyl and R¹, R², R³, R⁷, X¹, X² and X³ are as defined above for the compound of formula (I). This reaction is shown in Scheme 6.

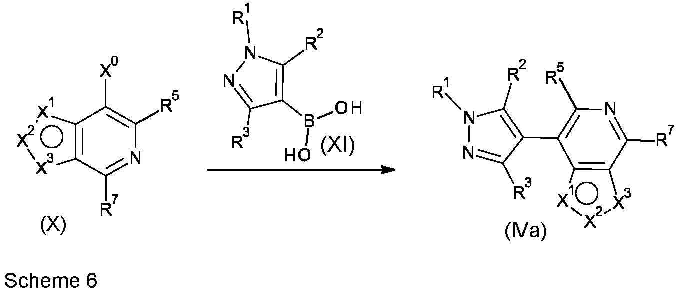

A further cross-coupling chemistry, namely C-H activation, can also be used to prepare compounds of formula (lVa), wherein R⁵ is hydrogen or methyl and R¹, R², R³, R⁷, X¹, X² and X³ are as defined above for the compound of formula (I). This reaction is shown in Scheme 7.

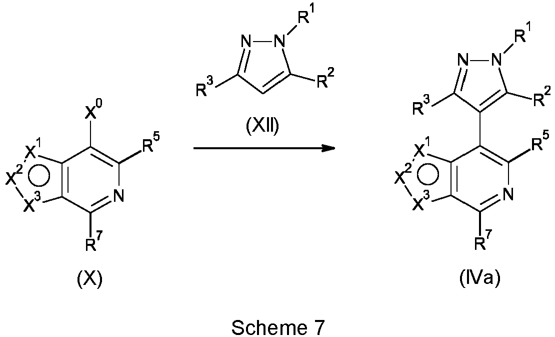

As shown in Scheme 7, compounds of formula (X), wherein R⁵ is hydrogen or methyl, R⁷, X¹, X² and X³ are as defined above for the compound of formula (I) and X⁰ is halogen, preferably chlorine, bromine or iodine, are reacted with compounds of formula (XII), wherein R¹, R² and R³ are as defined above for the compound of formula (I), in the presence of a palladium catalyst, typically palladium acetate Pd(OAc)₂, a suitable ligand, for example 1,10-phenanthroline, in the presence of a base such as cesium carbonate or potassium carbonate, in inert solvents such as chlorobenzene, toluene or xylene at temperatures between room temperature and 180°C, optionally under microwave heating conditions, preferably under inert atmosphere. Similar reactions have been reported in the literature for example *in* Chemical Science 2013, 4, 2374-2379.

Also, compounds of formula (III) may be prepared from compounds of formula (XIII). This reaction is shown in Scheme 8.

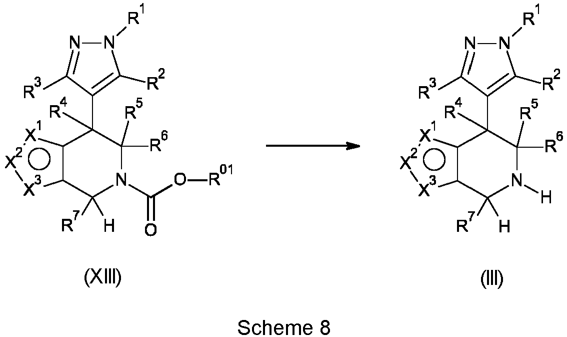

As shown in Scheme 8, compounds of formula (III) may be prepared by a person skilled in the art by a carbamate deprotection reaction of compounds of formula (XIII), wherein R¹, R², R³, R⁴, R⁵, R⁶, R⁷, X¹, X² and X³ are as defined above for the compound of formula (I) and R⁰¹ may be a member of a common carbamate protecting group substituent, for example methyl, *tert*-butyl, allyl, 2,2,2-trichloroethyl or benzyl. For example, when R⁰¹ is methyl, a suitable solvent such as dichloromethane and a suitable reagent such as iodotrimethylsilane may be employed to afford the product upon heating at temperatures between room temperature and 200°C, preferably between 20°C and the boiling point of the reaction mixture as described, for example, in J. Am. Chem. Soc. 1992, 114, 5959. The compounds of formula (III) thus obtained are converted to compounds of formula (I) as shown in Scheme 1.

Compounds of formula (XIII), wherein R¹, R², R³, R⁴, R⁵, R⁶, R¹, X¹, X² and X³ are as defined above for the compound of formula (I) and wherein R⁰¹ is as described above, may be formed by a Pictet-Spengler reaction between an aldehyde (including formaldehyde in its various forms) of formula (XV), wherein R⁷ is as defined above for the compound of formula (I), and a compound of formula (XIV), wherein R¹, R², R³, R⁴, R⁵, R⁶, X¹, X² and X³ are as defined above for the compound of formula (I) and wherein R⁰¹ is as described above, by combination with an acid in a suitable solvent, for example as described in Tetrahedron 1987, 43, 439. This reaction is shown in Scheme 9.

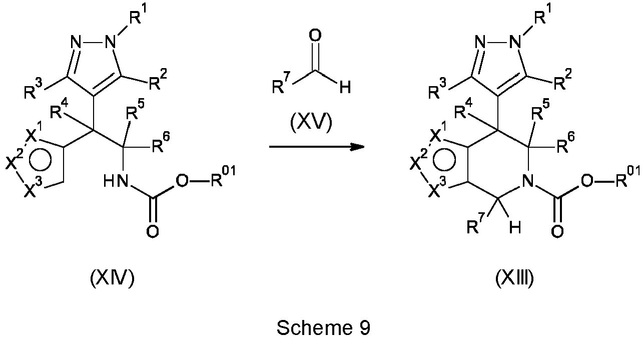

Compounds of formula (XIV), wherein R¹, R², R³, R⁴, R⁵, R⁶, X¹, X² and X³ are as defined above for the compound of formula (I) and wherein R⁰¹ is as described above, may be prepared by a reaction between amines of formula (XVI), wherein R¹, R², R³, R⁴, R⁵, R⁶, X¹, X² and X³ are as defined above for the compound of formula (I), and a suitable protecting reagent such as methyl chloroformate, optionally in the presence of a base such as triethylamine or pyridine, in a suitable solvent such as dichloromethane at temperatures between -20°C and the boiling point of the mixture, as for example described in Org. Biomolec.Chem. 2016, 14, 6853. This reaction is shown in Scheme 10.

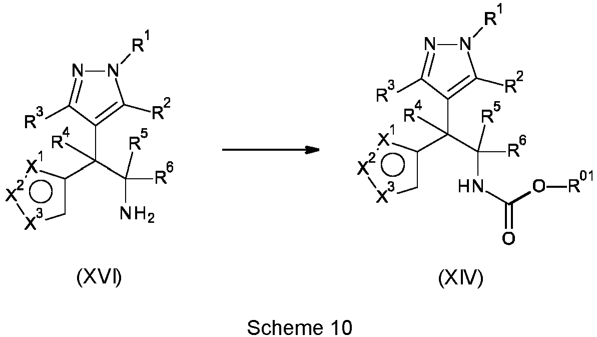

Compounds of formula (XVI), or salts thereof, wherein R¹, R², R³, R⁴, R⁵, R⁶, X¹, X² and X³ are as defined above for the compound of formula (I), may be prepared by a person skilled in the art by a reaction between nitriles of formula (XVII), wherein R¹, R², R³, R⁴, X¹, X² and X³ are as defined above for the compound of formula (I), and a suitable nucleophile such as (dimethyl sulfide)dihydroboron (BMS) in a suitable aprotic solvent such as tetrahydrofuran, for example as described in J. Org. Chem. 1981 47, 3153. Alternatively, Grignard reagents R⁵MgBr or R⁶MgBr, wherein R⁵ and R⁶ are as defined above for the compound of formula (I), may be added as nucleophiles to compounds of formula (XVII), sequentially or simultaneously, to allow more highly substituted amines of formula (XVI) to be prepared. Such Grignard additions to nitriles are carried out in an inert solvent such as diethyl ether, tert-butylmethyl ether, and cyclopentyl methyl ether in the presence of a Lewis acid such as Ti(O-'Pr)₄ (see Synlett 2007, (4), 652-654). This reaction is shown in Scheme 11.

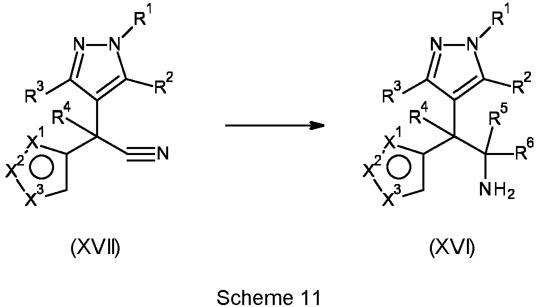

Compounds of formula (XVII), wherein R¹, R², R³, R⁴, X¹, X² and X³ are as defined above for the compound of formula (I), may be prepared by a person skilled in the art following known methods. More specifically, compounds of formula (XVII), and intermediates thereof, may be prepared from compounds of formula (XVIII) as shown in Scheme 12.

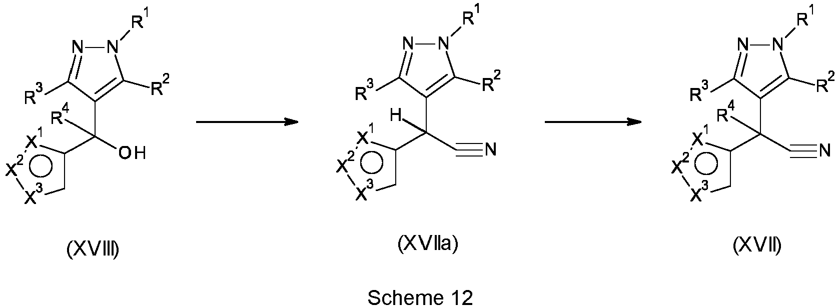

For example, compounds of formula (XVII), wherein R¹, R², R³, R⁴, X¹, X² and X³ are as defined above for the compound of formula (I) and R⁴ is different from hydrogen, may be prepared by a person skilled in the art by deprotonation of compound of formula (XVlla), wherein R⁴ is hydrogen and R¹, R², R³, X¹, X² and X³ are as defined above for the compound of formula (I), using a strong base such as n-butyl lithium or sodium hydride at cryogenic temperatures in an inert solvent such as tetrahydrofuran, followed by addition of a suitable alkylating agent R⁴-X, wherein X is halogen, for example iodomethane.

Compounds of formula (XVlla), wherein R⁴ is hydrogen and R¹, R², R³, X¹, X² and X³ are as defined above for the compound of formula (I), may be prepared from alcohols of formula (XVIII) by treatment with cyanotrimethylsilane (TMSCN) in the presence of a base such as lithium carbonate in a nonpolar solvent such as dichloromethane at temperatures between 0°C and the boiling point of the reaction mixture. Such transformations are well known in the literature under a variety of conditions, for example as described in Org. Lett. 2008, 10, 4570 and references therein. This reaction is shown in Scheme 12.

Compounds of formula (III) are commercially available or are readily prepared by compounds known in the state of the art. Compounds of formula (XVIII) may be prepared by methods known to a person skilled in the art.

Salts of compounds of formula (I) may be prepared in a manner known *per se.* Thus, for example, acid addition salts of compounds of formula (I) are obtained by treatment with a suitable acid or a suitable ion exchanger reagent and salts with bases are obtained by treatment with a suitable base or with a suitable ion exchanger reagent.

Salts of compounds of formula (I) can be converted in the customary manner into the free compounds (I), acid addition salts, for example, by treatment with a suitable basic compound or with a suitable ion exchanger reagent and salts with bases, for example, by treatment with a suitable acid or with a suitable ion exchanger reagent.

Salts of compounds of formula (I) can be converted in a manner known *per se* into other salts of compounds of formula (I), acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula (I), which have salt-forming properties, can be obtained in free form or in the form of salts.

The compounds of formula (I) and, where appropriate, the tautomer's thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule, the invention relates to the pure isomers and also to all isomer mixtures which are possible and is to be understood in each case in this sense hereinabove and herein below, even when stereochemical details are not mentioned specifically in each case.

Diastereomeric mixtures or racemic mixtures of compounds of formula (I), in free form or in salt form, which can be obtained depending on which starting materials and procedures have been chosen can be separated in a known manner into the pure diastereomers or racemates on the basis of the physicochemical differences of the components, for example by fractional crystallization, distillation and/or chromatography.

Enantiomeric mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl cellulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

It is advantageous to isolate or synthesize in each case the biologically more effective isomer, for example enantiomer or diastereomer, or isomer mixture, for example enantiomer mixture or diastereomer mixture, if the individual components have a different biological activity.

As an example, compounds with more than one asymmetric carbon atoms may exist in diastereomeric forms which can be optionally separated using for example supercritical fluid chromatography (SFC) chromatography with chiral colums. Such diastereomers can show a different fungicidal activity profile, but all isomers and diastereomers form part of this invention.

The compounds of formula (I) (also shown for compounds of formula (I-A) of the present invention exhibit three asymmetric carbon atoms, wherein the star (*) indicates the asymmetric carbon atom, such there are eight stereoisomers available. These eight stereoisomers consist of four sets of enantiomers.

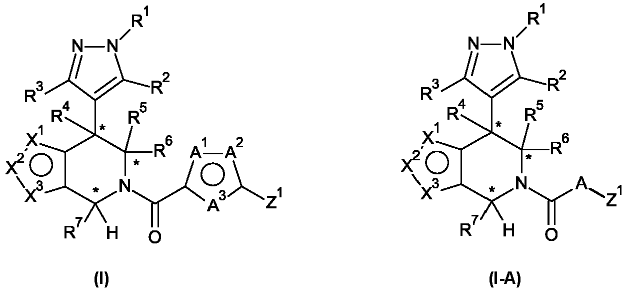

A person skilled in the art is well aware that above diastereomeres and enantiomers of formula (I) and formula (I-A), wherein R¹, R², R³, R⁴, R⁵, R⁶, R⁷, X¹, X², X³, A (or A¹, A², and A³) and Z¹ are as defined for formula (I) are covered by the claims.

The relationship between enantiomers and diastereomers of compounds of formula (I-A), wherein R¹ is methyl, R², R³, R⁴, R⁵, R⁶ are hydrogen, R⁷ is methyl, X¹ and X² are CH and X³ is S and A is A10 is shown below.

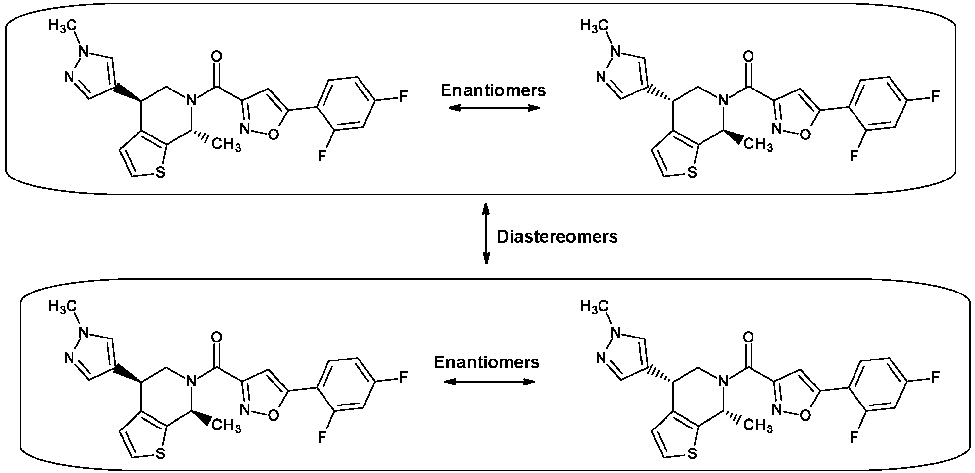

The compounds of formula (I) and, where appropriate, the tautomers thereof, in each case in free form or in salt form, can, if appropriate, also be obtained in the form of hydrates and/or include other solvents, for example those which may have been used for the crystallization of compounds which are present in solid form.

As already indicated, surprisingly, it has now been found that the compounds of formula (I) of the present invention have, for practical purposes, a very advantageous level of biological activity for protecting plants against diseases that are caused by fungi.

The compounds of formula (I) according to the invention can be used in the agricultural sector and related fields of use, e.g., as active ingredients for controlling plant pests or on non-living materials for the control of spoilage microorganisms or organisms potentially harmful to man. The novel compounds are distinguished by excellent activity at low rates of application, by being well tolerated by plants and by being environmentally safe. They have very useful curative, preventive and systemic properties and can be used for protecting numerous cultivated plants. The compounds of formula (I) can be used to inhibit or destroy the pests that occur on plants or parts of plants (fruit, blossoms, leaves, stems, tubers, roots) of different crops of useful plants, while at the same time protecting also those parts of the plants that grow later, e.g., from phytopathogenic microorganisms.

The present invention further relates to a method for controlling or preventing infestation of plants or plant propagation material and/or harvested food crops susceptible to microbial attack by treating plants or plant propagation material and/or harvested food crops wherein an effective amount a compound of formula (I) according to the invention is applied to the plants, to parts thereof or the locus thereof.

It is also possible to use a compound of formula (I) according to the invention as a fungicide. The term "fungicide" as used herein means a compound that controls, modifies, or prevents the growth of fungi. The term "fungicidally effective amount" where used means the quantity of such a compound or combination of such compounds that is capable of producing an effect on the growth of fungi. Controlling or modifying effects include all deviation from natural development, such as killing, retardation and the like, and prevention includes barrier or other defensive formation in or on a plant to prevent fungal infection.

As used herein, the term "controlling" refers to reducing the number of pests, eliminating pests and/or preventing further pest damage such that damage to a plant or to a plant derived product is reduced.

The term "preventing" when used in context of infestation of plants or plant propagartion material and /or harvest food crops refers to the avoidance of a symptom due to microbial attack or fungal infections (growth of fungi).

It may also be possible to use compounds of formula (I) according to the invention as dressing agents for the treatment of plant propagation material, e.g., seed, such as fruits, tubers or grains, or plant cuttings, for the protection against fungal infections as well as against phytopathogenic fungi occurring in the soil. The propagation material can be treated with a composition comprising a compound of formula (I) before planting: seed, for example, can be dressed before being sown. The active compounds of formula (I) can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore, the compounds of formula (I) according to the invention can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

The compounds of formula (I) according to the invention are for example, effective against fungi and fungal vectors of disease as well as phytopathogenic bacteria and viruses. These fungi and fungal vectors of disease as well as phytopathogenic bacteria and viruses are for example:
Absidia corymbifera, Alternaria spp. including Alternaria solani, Aphanomyces spp., Ascochyta spp., Aspergillus spp. including A. flavus, A. fumigatus, A. nidulans, A. niger, A. terrus, Aureobasidium spp. including A. pullulans, Blastomyces dermatitidis, Blumeria graminis, Bremia lactucae, Botryosphaeria spp. including B. dothidea, B. obtusa, Botryotinia fuckeliana, Botrytis spp. inclusing Botrytis cinerea, Candida spp. including C. albicans, C. glabrata, C. krusei, C. lusitaniae, C. parapsilosis, C. tropicalis, Cephaloascus fragrans, Ceratocystis spp., Cercospora spp. including Cercospora arachidicola, Cercospora kikuchii,Cercospora sojina, Cercosporidium personatum, Cladosporium spp. spp. including Cladosporium cucumerinum, Claviceps purpurea, Coccidioides immitis, Cochliobolus spp., Colletotrichum spp. including Colletotrichum musae, Colletotrichum asianum, Corynespora cassiicola, Cryptococcus neoformans, Diaporthe spp., Didymella spp. spp. including Didymella bryoniae, Drechslera spp., Elsinoe spp., Epidermophyton spp., Erwinia amylovora, Erysiphe spp. including Erysiphe cichoracearum, Eutypa lata, Fusarium spp. including Fusarium culmorum, Fusarium graminearum, Fusarium langsethiae, Fusarium moniliforme, Fusarium oxysporum, Fusarium proliferatum, Fusarium subglutinans, Fusarium solani, Gaeumannomyces graminis, Gibberella fujikuroi, Gloeodes pomigena, Gloeosporium musarum, Glomerella cingulate, Glomerella lagenarium, Guignardia bidwellii, Gymnosporangium juniperi-virginianae, Helminthosporium spp., Hemileia spp., Histoplasma spp. including H. capsulatum, Laetisaria fuciformis, Leptographium lindbergi, Leveillula taurica, Lophodermium seditiosum, Microdochium nivale, Microsporum spp., Monilinia spp., Mucor spp., Mycosphaerella spp. including Mycosphaerella graminicola, Mycosphaerella pomi, Oncobasidium theobromaeon, Ophiostoma piceae, Paracoccidioides spp., Penicillium spp. including P. digitatum, P. italicum, Petriellidium spp., Peronosclerospora spp. Including P. maydis, P. philippinensis and P. sorghi, Peronospora spp., Phaeosphaeria nodorum, Phakopsora pachyrhizi, Phellinus igniarus, Phialophora spp., Phoma spp., Phomopsis viticola, Phytophthora spp. including P. infestans, Plasmopara spp. including Plasmopara halstedii, Plasmopara viticola, Pleospora spp., Podosphaera spp. including P. leucotricha, Polymyxa graminis, Polymyxa betae, Pseudocercosporella herpotrichoides, Pseudomonas spp., Pseudoperonospora spp. including P. cubensis, P. humuli, Pseudopeziza tracheiphila, Puccinia Spp. including Puccinia hordei, Puccinia recondita, Puccinia striiformis, Puccinia triticina, Pyrenopeziza spp., Pyrenophora spp. spp. including Pyrenophora teres, Pyricularia spp. including Pyricularia oryzae, Pythium spp. including P. ultimum, Ramularia spp., Rhizoctonia spp. spp. including Rhizoctonia solani, Rhizomucor pusillus, Rhizopus arrhizus, Rhynchosporium spp., Scedosporium spp. including S. apiospermum and S. prolificans, Schizothyrium pomi, Sclerotinia spp. including Sclerotinia sclerotiorum, Sclerotium spp., Septoria spp., including Septoria nodorum, Septoria tritici, Sphaerotheca macularis, Sphaerotheca fusca (Sphaerotheca fuliginea), Sporothorix spp., Stagonospora nodorum, Stemphylium spp., . Stereum hirsutum, Thanatephorus cucumeris, Thielaviopsis basicola, Tilletia spp., Trichoderma spp. including T. harzianum, T. pseudokoningii, T. viride, Trichophyton spp., Typhula spp., Uncinula necator, Urocystis spp., Ustilago spp., Venturia spp. including Venturia inaequalis, Verticillium spp., and Xanthomonas spp.

The compounds of formula (I) according to the invention may be used for example on turf, ornamentals, such as flowers, shrubs, broad-leaved trees or evergreens, for example conifers, as well as for tree injection, pest management and the like.

Examples of target crops and/or useful plants to be protected according to the present invention are for example perennial and annual crops, such as berry plants for example blackberries, blueberries, cranberries, raspberries and strawberries; cereals for example barley, maize (corn), millet, oats, rice, rye, sorghum triticale and wheat; fibre plants for example cotton, flax, hemp, jute and sisal; field crops for example sugar and fodder beet, coffee, hops, mustard, oilseed rape (canola), poppy, sugar cane, sunflower, tea and tobacco; fruit trees for example apple, apricot, avocado, banana, cherry, citrus, nectarine, peach, pear and plum; grasses for example Bermuda grass, bluegrass, bentgrass, centipede grass, fescue, ryegrass, St. Augustine grass and Zoysia grass; herbs such as basil, borage, chives, coriander, lavender, lovage, mint, oregano, parsley, rosemary, sage and thyme; legumes for example beans, lentils, peas and soya beans; nuts for example almond, cashew, ground nut, hazelnut, peanut, pecan, pistachio and walnut; palms for example oil palm; ornamentals for example flowers, shrubs and trees; other trees, for example cacao, coconut, olive and rubber; vegetables for example asparagus, aubergine, broccoli, cabbage, carrot, cucumber, garlic, lettuce, marrow, melon, okra, onion, pepper, potato, pumpkin, rhubarb, spinach and tomato; and vines for example grapes.

The term "useful plants" is to be understood as also including useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield@ summer rape (Canola). Examples of crops that have been rendered tolerant to herbicides or classes of herbicides by genetic engineering methods include glyphosate- and glufosinate-resistant maize varieties commercially available under the trade names RoundupReady^{®}, Herculex I^{®} and LibertyLink^{®}.

The term "useful plants" is to be understood as also including useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard^{®} (maize variety that expresses a CryIA(b) toxin); YieldGard Rootworm^{®} (maize variety that expresses a CryllIB(b1) toxin); YieldGard Plus^{®} (maize variety that expresses a CryIA(b) and a CryllIB(b1) toxin); Starlink^{®} (maize variety that expresses a Cry9(c) toxin); Herculex I^{®} (maize variety that expresses a CrylF(a2) toxin and the enzyme phosphinothricine N-acetyltransferase (PAT) to achieve tolerance to the herbicide glufosinate ammonium); NuCOTN 33B^{®} (cotton variety that expresses a CryIA(c) toxin); Bollgard I^{®} (cotton variety that expresses a CryIA(c) toxin); Bollgard II^{®} (cotton variety that expresses a CryIA(c) and a CrylIA(b) toxin); VIPCOT^{®} (cotton variety that expresses a VIP toxin); NewLeaf^{®} (potato variety that expresses a CryllIA toxin); Nature-Gard^{®} Agrisure^{®} GT Advantage (GA21 glyphosate-tolerant trait), Agrisure^{®} CB Advantage (Bt11 corn borer (CB) trait), Agrisure^{®} RW (corn rootworm trait) and Protecta^{®}.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins from Bacillus cereus or Bacillus popilliae; or insecticidal proteins from Bacillus thuringiensis, such as d-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3 hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

Further, in the context of the present invention there are to be understood by □-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO93/07278, WO95/34656, EP-A-0 427 529, EP-A-451 878 and WO 03/052073.

The processes for the preparation of such transgenic plants are generally known to a person skilled in the art and are described, for example, in the publications mentioned above. Cryl-type deoxyribonucleic acids and their preparation are known, for example, from WO 95/34656, EP-A-0 367 474, EP-A-0 401 979 and WO90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and butterflies (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); Nature¬Gard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. Bt11 Maize from Syngenta Seeds SAS, Chemin de l'Hobit 27, F-31 790 St. Sauveur, France, registration number C/FR/96/05/10. Genetically modified Zea mays which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. Bt176 Maize from Syngenta Seeds SAS, Chemin de l'Hobit 27, F-31 790 St. Sauveur, France, registration number C/FR/96/05/10. Genetically modified Zea mays which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. MIR604 Maize from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. MON 863 Maize from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. IPC 531 Cotton from Monsanto Europe S.A. 270-272 Avenue de Tervuren, B-1150 Brussels, Belgium, registration number C/ES/96/02.
6. 1507 Maize from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. NK603 × MON 810 Maize from Monsanto Europe S.A. 270-272 Avenue de Tervuren, B 1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

The compounds of formula (I) according to the invention may be used in controlling or preventing phytopathogenic diseases, especially phytopathogenic fungi such as Alternaria solani, Blumeria graminis, Botryotinia fuckeliana, Botrytis cinerea, Cercospora arachidicola, Cercospora kikuchii, Cercospora sojina, Cladosporium cucumerinum, Colletotrichum lagenarium, Corynespora cassiicola, Didymella bryoniae, Fusarium spp., Glomerella lagenarium, Leptosphaeria spp., Leveillula taurica, Microdochium nivale, Plasmopara viticola, Puccinia recondita, Pyrenophora teres, Pyricularia oryzae, Rhizoctonia solani, Sclerotinia sclerotiorum, Septoria nodorum, Septoria tritici, Sphaerotheca fuliginea, Uncinula necator and Venturia inaequalis. In an embodiment of the present invention, the compounds of formula (I) according to the invention may be used in controlling or preventing phytopathogenic diseases, especially phytopathogenic fungi such as Septoria tritici, Pyrenophora teres, Puccinia recondita and Blumeria graminis in cereals; Cercospora arachidicola and Sclerotinia sclerotiorum in field crops; Alternaria solani in fruits and vegetables, e.g. tomatoes and potatoes; Botrytis cinerea in fruits, vegetables and field crops, e.g. strawberries, tomatoes, sunflower, legumes and grapes; Glomerella lagenarium in vegetables, e.g. cucumbers; Uncinula necator in vegetables, e.g. grapes; Venturia inaequalis in fruits, e.g. apples; Rhizoctonia solani in vegetables, e.g. potatoes; Cladosporium cucumerinum, Didymella bryoniae and Sphaerotheca fuliginea in vegetables, e.g. cucumbers; Leveillula taurica in cucumbers and solanaceous vegetables; Fusarium spp. in cereals and vegetables; Leptosphaeria spp. in cereals.

The term "locus" as used herein means fields in or on which plants are growing, or where seeds of cultivated plants are sown, or where seed will be placed into the soil. It includes soil, seeds, and seedlings, as well as established vegetation.

The term "plants" refers to all physical parts of a plant, including seeds, seedlings, saplings, roots, tubers, stems, stalks, foliage, and fruits.

The term "plant propagation material" is understood to denote generative parts of the plant, such as seeds, which can be used for the multiplication of the latter, and vegetative material, such as cuttings or tubers, for example potatoes. There can be mentioned for example seeds (in the strict sense), roots, fruits, tubers, bulbs, rhizomes and parts of plants. Germinated plants and young plants which are to be transplanted after germination or after emergence from the soil, may also be mentioned. These young plants can be protected before transplantation by a total or partial treatment by immersion. Preferably "plant propagation material" is understood to denote seeds.

The compounds of formula (I) according to the invention may be used in unmodified form or, preferably, together with the adjuvants conventionally employed in the art of formulation. To this end they may be conve¬niently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the pre¬vailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants, e.g. for agricultural use, can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

Suspension concentrates are aqueous formulations in which finely divided solid particles of the active compound are suspended. Such formulations include anti-settling agents and dispersing agents and may further include a wetting agent to enhance activity as well an anti-foam and a crystal growth inhibitor. In use, these concentrates are diluted in water and normally applied as a spray to the area to be treated. The amount of active ingredient may range from 0.5% to 95% of the concentrate.

Wettable powders are in the form of finely divided particles which disperse readily in water or other liquid carriers. The particles contain the active ingredient retained in a solid matrix. Typical solid matrices include fuller's earth, kaolin clays, silicas and other readily wet organic or inorganic solids. Wettable powders normally contain from 5% to 95% of the active ingredient plus a small amount of wetting, dispersing or emulsifying agent.

Emulsifiable concentrates are homogeneous liquid compositions dispersible in water or other liquid and may consist entirely of the active compound with a liquid or solid emulsifying agent, or may also contain a liquid carrier, such as xylene, heavy aromatic naphthas, isophorone and other non-volatile organic solvents. In use, these concentrates are dispersed in water or other liquid and normally applied as a spray to the area to be treated. The amount of active ingredient may range from 0.5% to 95% of the concentrate.

Granular formulations include both extrudates and relatively coarse particles and are usually applied without dilution to the area in which treatment is required. Typical carriers for granular formulations include sand, fuller's earth, attapulgite clay, bentonite clays, montmorillonite clay, vermiculite, perlite, calcium carbonate, brick, pumice, pyrophyllite, kaolin, dolomite, plaster, wood flour, ground corn cobs, ground peanut hulls, sugars, sodium chloride, sodium sulphate, sodium silicate, sodium borate, magnesia, mica, iron oxide, zinc oxide, titanium oxide, antimony oxide, cryolite, gypsum, diatomaceous earth, calcium sulphate and other organic or inorganic materials which absorb or which can be coated with the active compound. Granular formulations normally contain 5% to 25% of active ingredients which may include surface-active agents such as heavy aromatic naphthas, kerosene and other petroleum fractions, or vegetable oils; and/or stickers such as dextrins, glue or synthetic resins.

Dusts are free-flowing admixtures of the active ingredient with finely divided solids such as talc, clays, flours and other organic and inorganic solids which act as dispersants and carriers.

Microcapsules are typically droplets or granules of the active ingredient enclosed in an inert porous shell which allows escape of the enclosed material to the surroundings at controlled rates. Encapsulated droplets are typically 1 to 50 microns in diameter. The enclosed liquid typically constitutes 50 to 95% of the weight of the capsule and may include solvent in addition to the active compound. Encapsulated granules are generally porous granules with porous membranes sealing the granule pore openings, retaining the active species in liquid form inside the granule pores. Granules typically range from 1 millimetre to 1 centimetre and preferably 1 to 2 millimetres in diameter. Granules are formed by extrusion, agglomeration or prilling, or are naturally occurring. Examples of such materials are vermiculite, sintered clay, kaolin, attapulgite clay, sawdust and granular carbon. Shell or membrane materials include natural and synthetic rubbers, cellulosic materials, styrene-butadiene copolymers, polyacrylonitriles, polyacrylates, polyesters, polyamides, polyureas, polyurethanes and starch xanthates.

Other useful formulations for agrochemical applications include simple solutions of the active ingredient in a solvent in which it is completely soluble at the desired concentration, such as acetone, alkylated naphthalenes, xylene and other organic solvents. Pressurised sprayers, wherein the active ingredient is dispersed in finely-divided form as a result of vaporisation of a low boiling dispersant solvent carrier, may also be used.

Suitable agricultural adjuvants and carriers that are useful in formulating the compositions of the invention in the formulation types described above are well known to a person skilled in the art.

Liquid carriers that can be employed include, for example, water, toluene, xylene, petroleum naphtha, crop oil, acetone, methyl ethyl ketone, cyclohexanone, acetic anhydride, acetonitrile, acetophenone, amyl acetate, 2-butanone, chlorobenzene, cyclohexane, cyclohexanol, alkyl acetates, diacetonalcohol, 1,2-dichloropropane, diethanolamine, p diethylbenzene, diethylene glycol, diethylene glycol abietate, diethylene glycol butyl ether, diethylene glycol ethyl ether, diethylene glycol methyl ether, N,N-dimethyl formamide, dimethyl sulfoxide, 1,4-dioxane, dipropylene glycol, dipropylene glycol methyl ether, dipropylene glycol dibenzoate, diproxitol, alkyl pyrrolidinone, ethyl acetate, 2-ethyl hexanol, ethylene carbonate, 1,1,1-trichloroethane, 2-heptanone, alpha pinene, d-limonene, ethylene glycol, ethylene glycol butyl ether, ethylene glycol methyl ether, gamma-butyrolactone, glycerol, glycerol diacetate, glycerol monoacetate, glycerol triacetate, hexadecane, hexylene glycol, isoamyl acetate, isobornyl acetate, isooctane, isophorone, isopropyl benzene, isopropyl myristate, lactic acid, laurylamine, mesityl oxide, methoxy-propanol, methyl isoamyl ketone, methyl isobutyl ketone, methyl laurate, methyl octanoate, methyl oleate, methylene chloride, m-xylene, n-hexane, n-octylamine, octadecanoic acid, octyl amine acetate, oleic acid, oleylamine, o-xylene, phenol, polyethylene glycol (PEG400), propionic acid, propylene glycol, propylene glycol monomethyl ether, p-xylene, toluene, triethyl phosphate, triethylene glycol, xylene sulfonic acid, paraffin, mineral oil, trichloroethylene, perchloroethylene, ethyl acetate, amyl acetate, butyl acetate, methanol, ethanol, isopropanol, and higher molecular weight alcohols such as amyl alcohol, tetrahydrofurfuryl alcohol, hexanol, octanol, etc., ethylene glycol, propylene glycol, glycerine and N-methyl-2-pyrrolidinone. Water is generally the carrier of choice for the dilution of concentrates.

Suitable solid carriers include, for example, talc, titanium dioxide, pyrophyllite clay, silica, attapulgite clay, kieselguhr, chalk, diatomaxeous earth, lime, calcium carbonate, bentonite clay, fuller's earth, cotton seed hulls, wheat flour, soybean flour, pumice, wood flour, walnut shell flour and lignin.

A broad range of surface-active agents are advantageously employed in both said liquid and solid compositions, especially those designed to be diluted with carrier before application. These agents, when used, normally comprise from 0.1% to 15% by weight of the formulation. They can be anionic, cationic, non-ionic or polymeric in character and can be employed as emulsifying agents, wetting agents, suspending agents or for other purposes. Typical surface active agents include salts of alkyl sulfates, such as diethanolammonium lauryl sulphate; alkylarylsulfonate salts, such as calcium dodecylbenzenesulfonate; alkylphenol-alkylene oxide addition products, such as nonylphenol-C.sub. 18 ethoxylate; alcohol-alkylene oxide addition products, such as tridecyl alcohol-C.sub. 16 ethoxylate; soaps, such as sodium stearate; alkylnaphthalenesulfonate salts, such as sodium dibutylnaphthalenesulfonate; dialkyl esters of sulfosuccinate salts, such as sodium di(2 ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; and salts of mono and dialkyl phosphate esters.

Other adjuvants commonly utilized in agricultural compositions include crystallisation inhibitors, viscosity modifiers, suspending agents, spray droplet modifiers, pigments, antioxidants, foaming agents, antifoaming agents, light-blocking agents, compatibilizing agents, antifoam agents, sequestering agents, neutralising agents and buffers, corrosion inhibitors, dyes, odorants, spreading agents, penetration aids, micronutrients, emollients, lubricants and sticking agents.

In addition, further, other biocidally active ingredients or compositions may be combined with the compositions of the invention and used in the methods of the invention and applied simultaneously or sequentially with the compositions of the invention. When applied simultaneously, these further active ingredients may be formulated together with the compositions of the invention or mixed in, for example, the spray tank. These further biocidally active ingredients may be fungicides, herbicides, insecticides, bactericides, acaricides, nematicides and/or plant growth regulators.

Pesticidal agents are referred to herein using their common name are known, for example, from "The Pesticide Manual", 15th Ed., British Crop Protection Council 2009.

In addition, the compositions of the invention may also be applied with one or more systemically acquired resistance inducers ("SAR" inducer). SAR inducers are known and described in, for example, United States Patent No. US 6,919,298 and include, for example, salicylates and the commercial SAR inducer acibenzolar-S-methyl.

The compounds of formula (I) according to the invention are normally used in the form of agrochemical compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula (I) according to the invention may be used in the form of (fungicidal) compositions for controlling or protecting against phytopathogenic microorganisms, comprising as active ingredient at least one compound of formula (I) or of at least one preferred individual compound as defined herein, in free form or in agrochemically usable salt form, and at least one of the above-mentioned adjuvants.

The invention therefore provides a composition, preferably a fungicidal composition, comprising at least one compound of formula (I) according to the invention, an agriculturally acceptable carrier and optionally an adjuvant. An agricultural acceptable carrier is for example a carrier that is suitable for agricultural use. Agricultural carriers are well known in the art. Preferably, said composition may comprise at least one or more pesticidally-active compounds, for example an additional fungicidal active ingredient in addition to the compound of formula (I).

The compound of formula (I) according to the invention may be the sole active ingredient of a composition or it may be admixed with one or more additional active ingredients such as a pesticide, fungicide, synergist, herbicide or plant growth regulator where appropriate. An additional active ingredient may, in some cases, result in unexpected synergistic activities.

Examples of suitable additional active ingredients include the following: acycloamino acid fungicides, aliphatic nitrogen fungicides, amide fungicides, anilide fungicides, antibiotic fungicides, aromatic fungicides, arsenical fungicides, aryl phenyl ketone fungicides, benzamide fungicides, benzanilide fungicides, benzimidazole fungicides, benzothiazole fungicides, botanical fungicides, bridged diphenyl fungicides, carbamate fungicides, carbanilate fungicides, conazole fungicides, copper fungicides, dicarboximide fungicides, dinitrophenol fungicides, dithiocarbamate fungicides, dithiolane fungicides, furamide fungicides, furanilide fungicides, hydrazide fungicides, imidazole fungicides, mercury fungicides, morpholine fungicides, organophosphorous fungicides, organotin fungicides, oxathiin fungicides, oxazole fungicides, phenylsulfamide fungicides, polysulfide fungicides, pyrazole fungicides, pyridine fungicides, pyrimidine fungicides, pyrrole fungicides, quaternary ammonium fungicides, quinoline fungicides, quinone fungicides, quinoxaline fungicides, strobilurin fungicides, sulfonanilide fungicides, thiadiazole fungicides, thiazole fungicides, thiazolidine fungicides, thiocarbamate fungicides, thiophene fungicides, triazine fungicides, triazole fungicides, triazolopyrimidine fungicides, urea fungicides, valinamide fungicides, and zinc fungicides.

Examples of suitable additional active ingredients include the following: petroleum oils, 1,1-bis(4-chlorophenyl)-2-ethoxyethanol, 2,4-dichlorophenyl benzenesulfonate, 2-fluoro-N-methyl-N-1-naphthylacetamide, 4-chlorophenyl phenylsulfone, acetoprole, aldoxycarb, amidithion, amidothioate, amiton, amiton hydrogen oxalate, amitraz, aramite, arsenous oxide, azobenzene, azothoate, benomyl, benoxa-fos, benzyl benzoate, bixafen, brofenvalerate, bromocyclen, bromophos, bromopropylate, buprofezin, butocarboxim, butoxycarboxim, butylpyridaben, calcium polysulfide, camphechlor, carbanolate, carbophenothion, cymiazole, chinomethionat, chlorbenside, chlordimeform, chlordimeform hydrochloride, chlorfenethol, chlorfenson, chlorfensulfide, chlorobenzilate, chloromebuform, chloromethiuron, chloropropylate, chlorthiophos, cinerin I, cinerin II, cinerins, closantel, coumaphos, crotamiton, crotoxyphos, cufraneb, cyanthoate, DCPM, DDT, demephion, demephion-O, demephion-S, demeton-methyl, demeton-O, demeton-O-methyl, demeton-S, demeton-S-methyl, demeton-S-methylsulfon, dichlofluanid, dichlorvos, dicliphos, dienochlor, dimefox, dinex, dinex-diclexine, dinocap-4, dinocap-6, dinocton, dinopenton, dinosulfon, dinoterbon, dioxathion, diphenyl sulfone, disulfiram, DNOC, dofenapyn, doramectin, endothion, eprinomectin, ethoate-methyl, etrimfos, fenazaflor, fenbutatin oxide, fenothiocarb, fenpyrad, fenpyroximate, fenpyrazamine, fenson, fentrifanil, flubenzimine, flucycloxuron, fluenetil, fluorbenside, FMC 1137, formetanate, formetanate hydrochloride, formparanate, gamma-HCH, glyodin, halfenprox, hexadecyl cyclopropanecarboxylate, isocarbophos, jasmolin I, jasmolin II, jodfenphos, lindane, malonoben, mecarbam, mephosfolan, mesulfen, methacrifos, methyl bromide, metolcarb, mexacarbate, milbemycin oxime, mipafox, monocrotophos, morphothion, moxidectin, naled, 4-chloro-2-(2-chloro-2-methyl-propyl)-5-[(6-iodo-3-pyridyl)methoxy]pyridazin-3-one, nifluridide, nikkomycins, nitrilacarb, nitrilacarb 1:1 zinc chloride complex, omethoate, oxydeprofos, oxydisulfoton, pp'-DDT, parathion, permethrin, phenkapton, phosalone, phosfolan, phosphamidon, polychloroterpenes, polynactins, proclonol, promacyl, propoxur, prothidathion, prothoate, pyrethrin I, pyrethrin II, pyrethrins, pyridaphenthion, pyrimitate, quinalphos, quintiofos, R-1492, phosglycin, rotenone, schradan, sebufos, selamectin, sophamide, SSI-121, sulfiram, sulfluramid, sulfotep, sulfur, diflovidazin, tau-fluvalinate, TEPP, terbam, tetradifon, tetrasul, thiafenox, thiocarboxime, thiofanox, thiometon, thioquinox, thuringiensin, triamiphos, triarathene, triazophos, triazuron, trifenofos, trinactin, vamidothion, vaniliprole, bethoxazin, copper dioctanoate, copper sulfate, cybutryne, dichlone, dichlorophen, endothal, fentin, hydrated lime, nabam, quinoclamine, quinonamid, simazine, triphenyltin acetate, triphenyltin hydroxide, crufomate, piperazine, thiophanate, chloralose, fenthion, pyridin-4-amine, strychnine, 1-hydroxy-1H-pyridine-2-thione, 4-(quinoxalin-2-ylamino)benzenesulfonamide, 8-hydroxyquinoline sulfate, bronopol, copper hydroxide, cresol, dipyrithione, dodicin, fenaminosulf, formaldehyde, hydrargaphen, kasugamycin, kasugamycin hydrochloride hydrate, nickel bis(dimethyldithiocarbamate), nitrapyrin, octhilinone, oxolinic acid, oxytetracycline, potassium hydroxyquinoline sulfate, probenazole, streptomycin, streptomycin sesquisulfate, tecloftalam, thiomersal, Adoxophyes orana GV, Agrobacterium radiobacter, Amblyseius spp., Anagrapha falcifera NPV, Anagrus atomus, Aphelinus abdominalis, Aphidius colemani, Aphidoletes aphidimyza, Autographa californica NPV, Bacillus sphaericus Neide, Beauveria brongniartii, Chrysoperla carnea, Cryptolaemus montrouzieri, Cydia pomonella GV, Dacnusa sibirica, Diglyphus isaea, Encarsia formosa, Eretmocerus eremicus, Heterorhabditis bacteriophora and H. megidis, Hippodamia convergens, Leptomastix dactylopii, Macrolophus caliginosus, Mamestra brassicae NPV, Metaphycus helvolus, Metarhizium anisopliae var. acridum, Metarhizium anisopliae var. anisopliae, Neodiprion sertifer NPV and N. lecontei NPV, Orius spp., Paecilomyces fumosoroseus, Phytoseiulus persimilis, Steinernema bibionis, Steinernema carpocapsae, Steinernema feltiae, Steinernema glaseri, Steinernema riobrave, Steinernema riobravis, Steinernema scapterisci, Steinernema spp., Trichogramma spp., Typhlodromus occidentalis, Verticillium lecanii, apholate, bisazir, busulfan, dimatif, hemel, hempa, metepa, methiotepa, methyl apholate, morzid, penfluron, tepa, thiohempa, thiotepa, tretamine, uredepa, (E)-dec-5-en-1-yl acetate with (E)-dec-5-en-1-ol, (E)-tridec-4-en-1-yl acetate, (E)-6-methylhept-2-en-4-ol, (E,Z)-tetradeca-4,10-dien-1-yl acetate, (Z)-dodec-7-en-1-yl acetate, (Z)-hexadec-11-enal, (Z)-hexadec-11-en-1-yl acetate, (Z)-hexadec-13-en-11-yn-1-yl acetate, (Z)-icos-13-en-10-one, (Z)-tetradec-7-en-1-al, (Z)-tetradec-9-en-1-ol, (Z)-tetradec-9-en-1-yl acetate, (7E,9Z)-dodeca-7,9-dien-1-yl acetate, (9Z,11E)-tetradeca-9,11-dien-1-yl acetate, (9Z,12E)-tetradeca-9,12-dien-1-yl acetate, 14-methyloctadec-1-ene, 4-methylnonan-5-ol with 4-methylnonan-5-one, alpha-multistriatin, brevicomin, codlelure, codlemone, cuelure, disparlure, dodec-8-en-1-yl acetate, dodec-9-en-1-yl acetate, dodeca-8, 10-dien-1-yl acetate, dominicalure, ethyl 4-methyloctanoate, eugenol, frontalin, grandlure, grandlure I, grandlure II, grandlure III, grandlure IV, hexalure, ipsdienol, ipsenol, japonilure, lineatin, litlure, looplure, medlure, megatomoic acid, methyl eugenol, muscalure, octadeca-2,13-dien-1-yl acetate, octadeca-3,13-dien-1-yl acetate, orfralure, oryctalure, ostramone, siglure, sordidin, sulcatol, tetradec-11-en-1-yl acetate, trimedlure, trimedlure A, trimedlure B1, trimedlure B2, trimedlure C, trunc-call, 2-(octylthio)ethanol, butopyronoxyl, butoxy(polypropylene glycol), dibutyl adipate, dibutyl phthalate, dibutyl succinate, diethyltoluamide, dimethyl carbate, dimethyl phthalate, ethyl hexanediol, hexamide, methoquin-butyl, methylneodecanamide, oxamate, picaridin, 1-dichloro-1-nitroethane, 1,1-dichloro-2,2-bis(4-ethylphenyl)ethane, 1,2-dichloropropane with 1,3-dichloropropene, 1-bromo-2-chloroethane, 2,2,2-trichloro-1-(3,4-dichlorophenyl)ethyl acetate, 2,2-dichlorovinyl 2-ethylsulfinylethyl methyl phosphate, 2-(1,3-dithiolan-2-yl)phenyl dimethylcarbamate, 2-(2-butoxyethoxy)ethyl thiocyanate, 2-(4,5-dimethyl-1,3-dioxolan-2-yl)phenyl methylcarbamate, 2-(4-chloro-3,5-xylyloxy)ethanol, 2-chlorovinyl diethyl phosphate, 2-imidazolidone, 2-isovalerylindan-1,3-dione, 2-methyl(prop-2-ynyl)aminophenyl methylcarbamate, 2-thiocyanatoethyl laurate, 3-bromo-1-chloroprop-1-ene, 3-methyl-1-phenylpyrazol-5-yl dimethylcarbamate, 4-methyl(prop-2-ynyl)amino-3,5-xylyl methylcarbamate, 5,5-dimethyl-3-oxocyclohex-1-enyl dimethylcarbamate, acethion, acrylonitrile, aldrin, allosamidin, allyxycarb, alpha-ecdysone, aluminium phosphide, aminocarb, anabasine, athidathion, azamethiphos, Bacillus thuringiensis delta endotoxins, barium hexafluorosilicate, barium polysulfide, barthrin, Bayer 22/190, Bayer 22408, beta-cyfluthrin, beta-cypermethrin, bioethanomethrin, biopermethrin, bis(2-chloroethyl) ether, borax, bromfenvinfos, bromo-DDT, bufencarb, butacarb, butathiofos, butonate, calcium arsenate, calcium cyanide, carbon disulfide, carbon tetrachloride, cartap hydrochloride, cevadine, chlorbicyclen, chlordane, chlordecone, chloroform, chloropicrin, chlorphoxim, chlorprazophos, cis-resmethrin, cismethrin, clocythrin, copper acetoarsenite, copper arsenate, copper oleate, coumithoate, cryolite, CS 708, cyanofenphos, cyanophos, cyclethrin, cythioate, d-tetramethrin, DAEP, dazomet, decarbofuran, diamidafos, dicapthon, dichlofenthion, dicresyl, dicyclanil, dieldrin, diethyl 5-methylpyrazol-3-yl phosphate, dilor, dimefluthrin, dimetan, dimethrin, dimethylvinphos, dimetilan, dinoprop, dinosam, dinoseb, diofenolan, dioxabenzofos, dithicrofos, DSP, ecdysterone, El 1642, EMPC, EPBP, etaphos, ethiofencarb, ethyl formate, ethylene dibromide, ethylene dichloride, ethylene oxide, EXD, fenchlorphos, fenethacarb, fenitrothion, fenoxacrim, fenpirithrin, fensulfothion, fenthion-ethyl, flucofuron, fosmethilan, fospirate, fosthietan, furathiocarb, furethrin, guazatine, guazatine acetates, sodium tetrathiocarbonate, halfenprox, HCH, HEOD, heptachlor, heterophos, HHDN, hydrogen cyanide, hyquincarb, IPSP, isazofos, isobenzan, isodrin, isofenphos, isolane, isoprothiolane, isoxathion, juvenile hormone I, juvenile hormone II, juvenile hormone III, kelevan, kinoprene, lead arsenate, leptophos, lirimfos, lythidathion, m-cumenyl methylcarbamate, magnesium phosphide, mazidox, mecarphon, menazon, mercurous chloride, mesulfenfos, metam, metam-potassium, metam-sodium, methanesulfonyl fluoride, methocrotophos, methoprene, methothrin, methoxychlor, methyl isothiocyanate, methylchloroform, methylene chloride, metoxadiazone, mirex, naftalofos, naphthalene, NC-170, nicotine, nicotine sulfate, nithiazine, nornicotine, O-5-dichloro-4-iodophenyl O-ethyl ethylphosphonothioate, O,O-diethyl O-4-methyl-2-oxo-2H-chromen-7-yl phosphorothioate, O,O-diethyl O-6-methyl-2-propylpyrimidin-4-yl phosphorothioate, O,O,O',O'-tetrapropyl dithiopyrophosphate, oleic acid, para-dichlorobenzene, parathion-methyl, pentachlorophenol, pentachlorophenyl laurate, PH 60-38, phenkapton, phosnichlor, phosphine, phoxim-methyl, pirimetaphos, polychlorodicyclopentadiene isomers, potassium arsenite, potassium thiocyanate, precocene I, precocene II, precocene III, primidophos, profluthrin, promecarb, prothiofos, pyrazophos, pyresmethrin, quassia, quinalphos-methyl, quinothion, rafoxanide, resmethrin, rotenone, kadethrin, ryania, ryanodine, sabadilla, schradan, sebufos, SI-0009, thiapronil, sodium arsenite, sodium cyanide, sodium fluoride, sodium hexafluorosilicate, sodium pentachlorophenoxide, sodium selenate, sodium thiocyanate, sulcofuron, sulcofuron-sodium, sulfuryl fluoride, sulprofos, tar oils, tazimcarb, TDE, tebupirimfos, temephos, terallethrin, tetrachloroethane, thicrofos, thiocyclam, thiocyclam hydrogen oxalate, thionazin, thiosultap, thiosultap-sodium, tralomethrin, transpermethrin, triazamate, trichlormetaphos-3, trichloronat, trimethacarb, tolprocarb, triclopyricarb, triprene, veratridine, veratrine, XMC, zetamethrin, zinc phosphide, zolaprofos, meperfluthrin, tetramethylfluthrin, bis(tributyltin) oxide, bromoacetamide, ferric phosphate, niclosamide-olamine, tributyltin oxide, pyrimorph, trifenmorph, 1,2-dibromo-3-chloropropane, 1,3-dichloropropene, 3,4-dichlorotetrahydrothiophene 1,1-dioxide, 3-(4-chlorophenyl)-5-methylrhodanine, 5-methyl-6-thioxo-1,3,5-thiadiazinan-3-ylacetic acid, 6-isopentenylaminopurine, anisiflupurin, benclothiaz, cytokinins, DCIP, furfural, isamidofos, kinetin, Myrothecium verrucaria composition, tetrachlorothiophene, xylenols, zeatin, potassium ethylxanthate, acibenzolar, acibenzolar-S-methyl, Reynoutria sachalinensis extract, alpha-chlorohydrin, antu, barium carbonate, bisthiosemi, brodifacoum, bromadiolone, bromethalin, chlorophacinone, cholecalciferol, coumachlor, coumafuryl, coumatetralyl, crimidine, difenacoum, difethialone, diphacinone, ergocalciferol, flocoumafen, fluoroacetamide, flupropadine, flupropadine hydrochloride, norbormide, phosacetim, phosphorus, pindone, pyrinuron, scilliroside, sodium fluoroacetate, thallium sulfate, warfarin, 2-(2-butoxyethoxy)ethyl piperonylate, 5-(1,3-benzodioxol-5-yl)-3-hexylcyclohex-2-enone, farnesol with nerolidol, verbutin, MGK 264, piperonyl butoxide, piprotal, propyl isomer, S421, sesamex, sesasmolin, sulfoxide, anthraquinone, copper naphthenate, copper oxychloride, dicyclopentadiene, thiram, zinc naphthenate, ziram, imanin, ribavirin, chloroinconazide, mercuric oxide, thiophanate-methyl, azaconazole, bitertanol, bromuconazole, cyproconazole, difenoconazole, diniconazole, epoxiconazole, fenbuconazole, fluquinconazole, flusilazole, flutriafol, furametpyr, hexaconazole, imazalil, imibenconazole, ipconazole, metconazole, myclobutanil, paclobutrazole, pefurazoate, penconazole, prothioconazole, pyrifenox, prochloraz, propiconazole, pyrisoxazole, simeconazole, tebuconazole, tetraconazole, triadimefon, triadimenol, triflumizole, triticonazole, ancymidol, fenarimol, nuarimol, bupirimate, dimethirimol, ethirimol, dodemorph, fenpropidin, fenpropimorph, spiroxamine, tridemorph, cyprodinil, mepanipyrim, pyrimethanil, fenpiclonil, fludioxonil, benalaxyl, furalaxyl, metalaxyl, R-metalaxyl, ofurace, oxadixyl, carbendazim, debacarb, fuberidazole, thiabendazole, chlozolinate, dichlozoline, myclozoline, procymidone, vinclozoline, boscalid, carboxin, fenfuram, flutolanil, mepronil, oxycarboxin, penthiopyrad, thifluzamide, dodine, iminoctadine, azoxystrobin, dimoxystrobin, enestroburin, fenaminstrobin, flufenoxystrobin, fluoxastrobin, kresoxim-methyl, metominostrobin, trifloxystrobin, orysastrobin, picoxystrobin, pyraclostrobin, pyrametostrobin, pyraoxystrobin, ferbam, mancozeb, maneb, metiram, propineb, zineb, captafol, captan, fluoroimide, folpet, tolylfluanid, bordeaux mixture, copper oxide, mancopper, oxine-copper, nitrothal-isopropyl, edifenphos, iprobenphos, phosdiphen, tolclofos-methyl, anilazine, benthiavalicarb, blasticidin-S, chloroneb, chlorothalonil, cyflufenamid, cymoxanil, cyclobutrifluram, diclocymet, diclomezine, dicloran, diethofencarb, dimethomorph, flumorph, dithianon, ethaboxam, etridiazole, famoxadone, fenamidone, fenoxanil, ferimzone, fluazinam, flumetylsulforim,fluopicolide, fluoxytioconazole, flusulfamide, fluxapyroxad, fenhexamid, fosetylaluminium, hymexazol, iprovalicarb, cyazofamid, methasulfocarb, metrafenone, pencycuron, phthalide, polyoxins, propamocarb, pyribencarb, proquinazid, pyroquilon, pyriofenone, quinoxyfen, quintozene, tiadinil, triazoxide, tricyclazole, triforine, validamycin, valifenalate, zoxamide, mandipropamid, flubeneteram, isopyrazam, sedaxane, benzovindiflupyr, pydiflumetofen, 3-difluoromethyl-1-methyl-1H-pyrazole-4-carboxylic acid (3',4',5'-trifluoro-biphenyl-2-yl)-amide, isoflucypram, isotianil, dipymetitrone, 6-ethyl-5,7-dioxo-pyrrolo[4,5][1,4]dithiino[1,2-c]isothiazole-3-carbonitrile, 2-(difluoromethyl)-N-[3-ethyl-1,1-dimethyl-indan-4-yl]pyridine-3-carboxamide, 4-(2,6-difluorophenyl)-6-methyl-5-phenyl-pyridazine-3-carbonitrile, (R)-3-(difluoromethyl)-1-methyl-N-[1,1,3-trimethylindan-4-yl]pyrazole-4-carboxamide, 4-(2-bromo-4-fluoro-phenyl)-N-(2-chloro-6-fluoro-phenyl)-2,5-dimethyl-pyrazol-3-amine, 4-(2-bromo-4-fluorophenyl)-N-(2-chloro-6-fluorophenyl)-1,3-dimethyl-1H-pyrazol-5-amine, fluindapyr, coumethoxystrobin (jiaxiangjunzhi), Ivbenmixianan, dichlobentiazox, mandestrobin, 3-(4,4-difluoro-3,4-dihydro-3,3-dimethylisoquinolin-1-yl)quinolone, 2-[2-fluoro-6-[(8-fluoro-2-methyl-3-quinolyl)oxy]phenyl]propan-2-ol, oxathiapiprolin, tert-butyl N-[6-[[[(1-methyltetrazol-5-yl)-phenylmethylene]amino]oxymethyl]-2-pyridyl]carbamate, pyraziflumid, inpyrfluxam, trolprocarb, mefentrifluconazole, ipfentrifluconazole, 2-(difluoromethyl)-N-[(3R)-3-ethyl-1,1-dimethyl-indan-4-yl]pyridine-3-carboxamide, N'-(2,5-dimethyl-4-phenoxy-phenyl)-N-ethyl-N-methyl-formamidine, N'-[4-(4,5-dichlorothiazol-2-yl)oxy-2,5-dimethyl-phenyl]-N-ethyl-N-methyl-formamidine, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate, but-3-ynyl N-[6-[[(Z)-[(1-methyltetrazol-5-yl)-phenyl-methylene]amino]oxymethyl]-2-pyridyl]carbamate, methyl N-[[5-[4-(2,4-dimethylphenyl)triazol-2-yl]-2-methyl-phenyl]methyl]carbamate, 3-chloro-6-methyl-5-phenyl-4-(2,4,6-trifluorophenyl)pyridazine, pyridachlometyl, 3-(difluoromethyl)-1-methyl-N-[1,1,3-trimethylindan-4-yl]pyrazole-4-carboxamide, 1-[2-[[1-(4-chlorophenyl)pyrazol-3-yl]oxymethyl]-3-methyl-phenyl]-4-methyl-tetrazol-5-one, 1-methyl-4-[3-methyl-2-[[2-methyl-4-(3,4,5-trimethylpyrazol-1-yl)phenoxy]methyl]phenyl]tetrazol-5-one, aminopyrifen, ametoctradin, amisulbrom, penflufen, (Z,2E)-5-[1-(4-chlorophenyl)pyrazol-3-yl]oxy-2-methoxyimino-N,3-dimethyl-pent-3-enamide, florylpicoxamid, fenpicoxamid, metarylpicoxamid, tebufloquin, ipflufenoquin, quinofumelin, isofetamid, ethyl 1-[[4-[[2-(trifluoromethyl)-1,3-dioxolan-2-yl]methoxy]phenyl]methyl]pyrazole-3-carboxylate (may be prepared from the methods described in WO2020/056090), ethyl 1-[[4-[(Z)-2-ethoxy-3,3,3-trifluoro-prop-1-enoxy]phenyl]methyl]pyrazole-3-carboxylate (may be prepared from the methods described in WO2020/056090), methyl N-[[4-[1-(4-cyclopropyl-2,6-difluoro-phenyl)pyrazol-4-yl]-2-methylphenyl]methyl]carbamate (may be prepared from the methods described in WO2020/097012), methyl N-[[4-[1-(2,6-difluoro-4-isopropyl-phenyl)pyrazol-4-yl]-2-methyl-phenyl]methyl]carbamate (may be prepared from the methods described in WO2020/097012), 6-chloro-3-(3-cyclopropyl-2-fluoro-phenoxy)-N-[2-(2,4-dimethylphenyl)-2,2-difluoro-ethyl]-5-methyl-pyridazine-4-carboxamide (may be prepared from the methods described in WO2020/109391), 6-chloro-N-[2-(2-chloro-4-methyl-phenyl)-2,2-difluoro-ethyl]-3-(3-cyclopropyl-2-fluoro-phenoxy)-5-methyl-pyridazine-4-carboxamide (may be prepared from the methods described in WO2020/109391), 6-chloro-3-(3-cyclopropyl-2-fluoro-phenoxy)-N-[2-(3,4-dimethylphenyl)-2,2-difluoro-ethyl]-5-methyl-pyridazine-4-carboxamide (may be prepared from the methods described in WO2020/109391),N-[2-[2,4-dichloro-phenoxy]phenyl]-3-(difluoromethyl)-1-methyl-pyrazole-4-carboxamide, N-[2-[2-chloro-4-(trifluoromethyl)phenoxy]phenyl]-3-(difluoromethyl)-1-methyl-pyrazole-4-carboxamide, benzothiostrobin, phenamacril, 5-amino-1,3,4-thiadiazole-2-thiol zinc salt (2:1), fluopyram, flufenoxadiazam, flutianil, fluopimomide, pyrapropoyne, picarbutrazox, 2-(difluoromethyl)-N-(3-ethyl-1,1-dimethyl-indan-4-yl)pyridine-3-carboxamide, 2-(difluoromethyl)-N-((3R)-1,1,3- trimethylindan-4-yl) pyridine-3-carboxamide, 4-[[6-[2-(2,4-difluorophenyl)-1,1-difluoro-2-hydroxy-3-(1,2,4-triazol-1-yl)propyl]-3-pyridyl]oxy]benzonitrile, metyltetraprole, 2-(difluoromethyl)-N-((3R)-1,1,3- trimethylindan-4-yl) pyridine-3-carboxamide, α-(1,1-dimethylethyl)-α- [4'-(trifluoromethoxy) [1,1'- biphenyl]-4-yl]-5-pyrimidinemethanol, fluoxapiprolin, enoxastrobin, methyl (Z)-3-methoxy-2-[2-methyl-5-[4-(trifluoromethyl)triazol-2-yl]phenoxy]prop-2-enoate, methyl (Z)-3-methoxy-2-[2-methyl-5-(4-propyltriazol-2-yl)phenoxy]prop-2-enoate, methyl (Z)-2-[5-(3-isopropylpyrazol-1-yl)-2-methyl-phenoxy]-3-methoxy-prop-2-enoate, methyl (Z)-3-methoxy-2-[2-methyl-5-(3-propylpyrazol-1-yl)phenoxy]prop-2-enoate, methyl (Z)-3-methoxy-2-[2-methyl-5-[3-(trifluoromethyl)pyrazol-1-yl]phenoxy]prop-2-enoate (these compounds may be prepared from the methods described in WO2020/079111), methyl (Z)-2-(5-cyclohexyl-2-methyl-phenoxy)-3-methoxy-prop-2-enoate, methyl (Z)-2-(5-cyclopentyl-2-methyl-phenoxy)-3-methoxy-prop-2-enoate (these compounds may be prepared from the methods described in WO2020/193387), 4-[[6-[2-(2,4-difluorophenyl)-1,1-difluoro-2-hydroxy-3-(1,2,4-triazol-1-yl)propyl]-3-pyridyl]oxy] benzonitrile, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile, trinexapac, coumoxystrobin, zhongshengmycin, thiodiazole copper, zinc thiazole, amectotractin, iprodione, seboctylamine, N'-[5-bromo-2-methyl-6-[(1S)-1-methyl-2-propoxy-ethoxy]-3-pyridyl]-N-ethyl-N-methyl-formamidine, N'-[5-bromo-2-methyl-6-[(1R)-1-methyl-2-propoxy-ethoxy]-3-pyridyl]-N-ethyl-N-methyl-formamidine, N'-[5-bromo-2-methyl-6-(1-methyl-2-propoxy-ethoxy)-3-pyridyl]-N-ethyl-N-methyl-formamidine, N'-[5-chloro-2-methyl-6-(1-methyl-2-propoxy-ethoxy)-3-pyridyl]-N-ethyl-N-methyl-formamidine, N'-[5-bromo-2-methyl-6-(1-methyl-2-propoxy-ethoxy)-3-pyridyl]-N-isopropyl-N-methyl-formamidine (these compounds may be prepared from the methods described in WO2015/155075); N'-[5-bromo-2-methyl-6-(2-propoxypropoxy)-3-pyridyl]-N-ethyl-N-methyl-formamidine (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine, N'-[4-(1-cyclopropyl-2,2,2-trifluoro-1-hydroxy-ethyl)-5-methoxy-2-methyl-phenyl]-N-isopropyl-N-methyl-formamidine (these compounds may be prepared from the methods described in WO2018/228896); N-ethyl-N'-[5-methoxy-2-methyl-4-[(2-trifluoromethyl)oxetan-2-yl]phenyl]-N-methyl-formamidine, N-ethyl-N'-[5-methoxy-2-methyl-4-[(2-trifuoromethyl)tetrahydrofuran-2-yl]phenyl]-N-methyl-formamidine (these compounds may be prepared from the methods described in WO2019/110427); N-[(1R)-1-benzyl-3-chloro-1-methyl-but-3-enyl]-8-fluoro-quinoline-3-carboxamide, N-[(1S)-1-benzyl-3-chloro-1-methyl-but-3-enyl]-8-fluoro-quinoline-3-carboxamide, N-[(1R)-1-benzyl-3,3,3-trifluoro-1-methyl-propyl]-8-fluoro-quinoline-3-carboxamide, N-[(1S)-1-benzyl-3,3,3-trifluoro-1-methyl-propyl]-8-fluoro-quinoline-3-carboxamide, N-[(1R)-1-benzyl-1,3-dimethylbutyl]-7,8-difluoro-quinoline-3-carboxamide, N-[(1S)-1-benzyl-1,3-dimethyl-butyl]-7,8-difluoro-quinoline-3-carboxamide, 8-fluoro-N-[(1R)-1-[(3-fluorophenyl)methyl]-1,3-dimethyl-butyl]quinoline-3-carboxamide, 8-fluoro-N-[(1S)-1-[(3-fluorophenyl)methyl]-1,3-dimethyl-butyl]quinoline-3-carboxamide, N-[(1R)-1-benzyl-1,3-dimethyl-butyl]-8-fluoro-quinoline-3-carboxamide, N-[(1S)-1-benzyl-1,3-dimethyl-butyl]-8-fluoro-quinoline-3-carboxamide, N-((1R)-1-benzyl-3-chloro-1-methyl-but-3-enyl)-8-fluoro-quinoline-3-carboxamide, N-((1S)-1-benzyl-3-chloro-1-methyl-but-3-enyl)-8-fluoro-quinoline-3-carboxamide (these compounds may be prepared from the methods described in WO2017/153380); 1-(6,7-dimethylpyrazolo[1,5-a]pyridin-3-yl)-4,4,5-trifluoro-3,3-dimethyl-isoquinoline, 1-(6,7-dimethylpyrazolo[1,5-a]pyridin-3-yl)-4,4,6-trifluoro-3,3-dimethyl-isoquinoline, 4,4-difluoro-3,3-dimethyl-1-(6-methylpyrazolo[1,5-a]pyridin-3-yl)isoquinoline, 4,4-difluoro-3,3-dimethyl-1-(7-methylpyrazolo[1,5-a]pyridin-3-yl)isoquinoline, 1-(6-chloro-7-methyl-pyrazolo[1,5-a]pyridin-3-yl)-4,4-difluoro-3,3-dimethyl-isoquinoline (these compounds may be prepared from the methods described in WO2017/025510); 1-(4,5-dimethylbenzimidazol-1-yl)-4,4,5-trifluoro-3,3-dimethyl-isoquinoline, 1-(4,5-dimethylbenzimidazol-1-yl)-4,4-difluoro-3,3-dimethyl-isoquinoline, 6-chloro-4,4-difluoro-3,3-dimethyl-1-(4-methylbenzimidazol-1-yl)isoquinoline, 4,4-difluoro-1-(5-fluoro-4-methyl-benzimidazol-1-yl)-3,3-dimethyl-isoquinoline, 3-(4,4-difluoro-3,3-dimethyl-1-isoquinolyl)-7,8-dihydro-6H-cyclopenta[e]benzimidazole (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide, N,2-dimethoxy-N-[[4-[5-(trifluoromethyl)-1,2,4-oxadiazol-3-yl]phenyl]methyl]propanamide, N-ethyl-2-methyl-N-[[4-[5-(trifluoromethyl)-1,2,4-oxadiazol-3-yl]phenyl]methyl]propanamide, 1-methoxy-3-methyl-1-[[4-[5-(trifluoromethyl)-1,2,4-oxadiazol-3-yl]phenyl]methyl]urea, 1,3-dimethoxy-1-[[4-[5-(trifluoromethyl)-1,2,4-oxadiazol-3-yl]phenyl]methyl]urea, 3-ethyl-1-methoxy-1-[[4-[5-(trifluoromethyl)-1,2,4-oxadiazol-3-yl]phenyl]methyl]urea, N-[[4-[5-(trifluoromethyl)-1,2,4-oxadiazol-3-yl]phenyl]methyl]propanamide, 4,4-dimethyl-2-[[4-[5-(trifluoromethyl)-1,2,4-oxadiazol-3-yl]phenyl]methyl]isoxazolidin-3-one, 5,5-dimethyl-2-[[4-[5-(trifluoromethyl)-1,2,4-oxadiazol-3-yl]phenyl]methyl]isoxazolidin-3-one, ethyl 1-[[4-[5-(trifluoromethyl)-1,2,4-oxadiazol-3-yl]phenyl]methyl]pyrazole-4-carboxylate, N,N-dimethyl-1-[[4-[5-(trifluoromethyl)-1,2,4-oxadiazol-3-yl]phenyl]methyl]-1,2,4-triazol-3-amine (these compounds may be prepared from the methods described in WO2017/055473, WO2017/055469, WO2017/093348 and WO2017/118689); 2-[6-(4-chlorophenoxy)-2-(trifluoromethyl)-3-pyridyl]-1-(1,2,4-triazol-1-yl)propan-2-ol (this compound may be prepared from the methods described in WO2017/029179); 2-[6-(4-bromophenoxy)-2-(trifluoromethyl)-3-pyridyl]-1-(1,2,4-triazol-1-yl)propan-2-ol (this compound may be prepared from the methods described in WO2017/029179); 3-[2-(1-chlorocyclopropyl)-3-(2-fluorophenyl)-2-hydroxy-propyl]imidazole-4-carbonitrile (this compound may be prepared from the methods described in WO2016/156290); 3-[2-(1-chlorocyclopropyl)-3-(3-chloro-2-fluoro-phenyl)-2-hydroxy-propyl]imidazole-4-carbonitrile (this compound may be prepared from the methods described in WO2016/156290); (4-phenoxyphenyl)methyl 2-amino-6-methyl-pyridine-3-carboxylate (this compound may be prepared from the methods described in WO2014/006945); 2,6-Dimethyl-1H,5H-[1,4]dithiino[2,3-c:5,6-c']dipyrrole-1,3,5,7(2H,6H)-tetrone (this compound may be prepared from the methods described in WO2011/138281) N-methyl-4-[5-(trifluoromethyl)-1,2,4-oxadiazol-3-yl]benzenecarbothioamide; N-methyl-4-[5-(trifluoromethyl)-1,2,4-oxadiazol-3-yl]benzamide; (Z,2E)-5-[1-(2,4-dichlorophenyl)pyrazol-3-yl]oxy-2-methoxyimino-N,3-dimethyl-pent-3-enamide (this compound may be prepared from the methods described in WO2018/153707); N'-(2-chloro-5-methyl-4-phenoxy-phenyl)-N-ethyl-N-methyl-formamidine; N'-[2-chloro-4-(2-fluorophenoxy)-5-methyl-phenyl]-N-ethyl-N-methyl-formamidine (this compound may be prepared from the methods described in WO2016/202742); 2-(difluoromethyl)-N-[(3S)-3-ethyl-1,1-dimethyl-indan-4-yl]pyridine-3-carboxamide (this compound may be prepared from the methods described in WO2014/095675); (5-methyl-2-pyridyl)-[4-[5-(trifluoromethyl)-1,2,4-oxadiazol-3-yl]phenyl]methanone, (3-methylisoxazol-5-yl)-[4-[5-(trifluoromethyl)-1,2,4-oxadiazol-3-yl]phenyl]methanone (these compounds may be prepared from the methods described in WO2017/220485); 2-oxo-N-propyl-2-[4-[5-(trifluoromethyl)-1,2,4-oxadiazol-3-yl]phenyl]acetamide (this compound may be prepared from the methods described in WO2018/065414); ethyl 1-[[5-[5-(trifluoromethyl)-1,2,4-oxadiazol-3-yl]-2-thienyl]methyl]pyrazole-4-carboxylate (this compound may be prepared from the methods described in WO2018/158365); 2,2-difluoro-N-methyl-2-[4-[5-(trifluoromethyl)-1,2,4-oxadiazol-3-yl]phenyl]acetamide, N-[(E)-methoxyiminomethyl]-4-[5-(trifluoromethyl)-1,2,4-oxadiazol-3-yl]benzamide, N-[(Z)-methoxyiminomethyl]-4-[5-(trifluoromethyl)-1,2,4-oxadiazol-3-yl]benzamide, N-[N-methoxy-methyl-carbonimidoyl]-4-[5-(trifluoromethyl)-1,2,4-oxadiazol-3-yl]benzamide (these compounds may be prepared from the methods described in WO2018/202428).

The compounds of the invention may also be used in combination with anthelmintic agents. Such anthelmintic agents include, compounds selected from the macrocyclic lactone class of compounds such as ivermectin, avermectin, abamectin, emamectin, eprinomectin, doramectin, selamectin, moxidectin, nemadectin and milbemycin derivatives as described in EP0357460, EP0444964 and EP0594291. Additional anthelmintic agents include semisynthetic and biosynthetic avermectin/milbemycin derivatives such as those described in US5015630, WO9415944 and WO9522552. Additional anthelmintic agents include the benzimidazoles such as albendazole, cambendazole, fenbendazole, flubendazole, mebendazole, oxfendazole, oxibendazole, parbendazole, and other members of the class. Additional anthelmintic agents include imidazothiazoles and tetrahydropyrimidines such as tetramisole, levamisole, pyrantel pamoate, oxantel or morantel. Additional anthelmintic agents include flukicides, such as triclabendazole and clorsulon and the cestocides, such as praziquantel and epsiprantel.

The compounds of the invention may be used in combination with derivatives and analogues of the paraherquamide/marcfortine class of anthelmintic agents, as well as the antiparasitic oxazolines such as those disclosed in US5478855, US4639771 and DE19520936.

The compounds of the invention may be used in combination with derivatives and analogues of the general class of dioxomorpholine antiparasitic agents as described in WO9615121 and also with anthelmintic active cyclic depsipeptides such as those described in WO9611945, WO9319053, WO 9325543, EP0626375, EP0382173, WO9419334, EP0382173, and EP0503538.

The compounds of the invention may be used in combination with other ectoparasiticides; for example, fipronil; pyrethroids; organophosphates; insect growth regulators such as lufenuron; ecdysone agonists such as tebufenozide and the like; neonicotinoids such as imidacloprid and the like.

The compounds of the invention may be used in combination with terpene alkaloids, for example those described in WO95/19363 or WO04/72086, particularly the compounds disclosed therein.

Other examples of such biologically active compounds that the compounds of the invention may be used in combination with include but are not restricted to the following:
Organophosphates: acephate, azamethiphos, azinphos-ethyl, azinphos- methyl, bromophos, bromophos-ethyl, cadusafos, chlorethoxyphos, chlorpyrifos, chlorfenvinphos, chlormephos, demeton, demeton-S-methyl, demeton-S-methyl sulphone, dialifos, diazinon, dichlorvos, dicrotophos, dimethoate, disulfoton, ethion, ethoprophos, etrimfos, famphur, fenamiphos, fenitrothion, fensulfothion, fenthion, flupyrazofos, fonofos, formothion, fosthiazate, heptenophos, isazophos, isothioate, isoxathion, malathion, methacriphos, methamidophos, methidathion, methyl- parathion, mevinphos, monocrotophos, naled, omethoate, oxydemeton-methyl, paraoxon, parathion, parathion-methyl, phenthoate, phosalone, phosfolan, phosphocarb, phosmet, phosphamidon, phorate, phoxim, pirimiphos, pirimiphos- methyl, profenofos, propaphos, proetamphos, prothiofos, pyraclofos, pyridapenthion, quinalphos, sulprophos, temephos, terbufos, tebupirimfos, tetrachlorvinphos, thimeton, triazophos, trichlorfon, vamidothion.

Carbamates: alanycarb, aldicarb, 2-sec-butylphenyl methylcarbamate, benfuracarb, carbaryl, carbofuran, carbosulfan, cloethocarb, ethiofencarb, fenoxycarb, fenthiocarb, furathiocarb, HCN-801, isoprocarb, indoxacarb, methiocarb, methomyl, 5-methyl-m-cumenylbutyryl(methyl)carbamate, oxamyl, pirimicarb, propoxur, thiodicarb, thiofanox, triazamate, UC-51717.

Pyrethroids: acrinathin, allethrin, alphametrin, 5-benzyl-3-furylmethyl (E)-(1R)-cis-2,2-dimethyl-3-(2-oxothiolan-3-ylidenemethyl)cyclopropanecarboxylate, bifenthrin, beta-cyfluthrin, cyfluthrin, a-cypermethrin, beta-cypermethrin, bioallethrin, bioallethrin((S)-cyclopentylisomer), bioresmethrin, bifenthrin, NCI-85193, cycloprothrin, cyhalothrin, cythithrin, cyphenothrin, deltamethrin, empenthrin, esfenvalerate, ethofenprox, fenfluthrin, fenpropathrin, fenvalerate, flucythrinate, flumethrin, fluvalinate (D-isomer), imiprothrin, cyhalothrin, lambda-cyhalothrin, permethrin, phenothrin, prallethrin, pyrethrins (natural products), resmethrin, tetramethrin, transfluthrin, theta-cypermethrin, silafluofen, t-fluvalinate, tefluthrin, tralomethrin, zeta-cypermethrin.

Arthropod growth regulators: a) chitin synthesis inhibitors: benzoylureas: chlorfluazuron, diflubenzuron, fluazuron, flucycloxuron, flufenoxuron, hexaflumuron, lufenuron, novaluron, teflubenzuron, triflumuron, buprofezin, diofenolan, hexythiazox, etoxazole, chlorfentazine; b) ecdysone antagonists: halofenozide, methoxyfenozide, tebufenozide; c) juvenoids: pyriproxyfen, methoprene (including S-methoprene), fenoxycarb; d) lipid biosynthesis inhibitors: spirodiclofen.

Other antiparasitics: acequinocyl, amitraz, AKD-1022, ANS-118, azadirachtin, Bacillus thuringiensis, bensultap, bifenazate, binapacryl, bromopropylate, BTG-504, BTG-505, camphechlor, cartap, chlorobenzilate, chlordimeform, chlorfenapyr, chromafenozide, clothianidine, cyromazine, diacloden, diafenthiuron, DBI-3204, dinactin, dihydroxymethyldihydroxypyrrolidine, dinobuton, dinocap, endosulfan, ethiprole, ethofenprox, fenazaquin, flumite, MTI- 800, fenpyroximate, fluacrypyrim, flubenzimine, flubrocythrinate, flufenzine, flufenprox, fluproxyfen, halofenprox, hydramethylnon, IKI-220, kanemite, NC-196, neem guard, nidinorterfuran, nitenpyram, SD-35651, WL-108477, pirydaryl, propargite, protrifenbute, pymethrozine, pyridaben, pyrimidifen, NC-1111, R-195,RH-0345, RH-2485, RYI-210, S-1283, S-1833, SI-8601, silafluofen, silomadine, spinosad, tebufenpyrad, tetradifon, tetranactin, thiacloprid, thiocyclam, thiamethoxam, tolfenpyrad, triazamate, triethoxyspinosyn, trinactin, verbutin, vertalec, YI-5301.

Biological agents: Bacillus thuringiensis ssp aizawai, kurstaki, Bacillus thuringiensis delta endotoxin, baculovirus, entomopathogenic bacteria, virus and fungi.

Bactericides: chlortetracycline, oxytetracycline, streptomycin.

Other biological agents: enrofloxacin, febantel, penethamate, moloxicam, cefalexin, kanamycin, pimobendan, clenbuterol, omeprazole, tiamulin, benazepril, pyriprole, cefquinome, florfenicol, buserelin, cefovecin, tulathromycin, ceftiour, carprofen, metaflumizone, praziquarantel, triclabendazole.

The following mixtures of the compounds of Formula (I) with active ingredients are preferred. The abbreviation "TX" means one compound selected from the group consisting of the compounds of formula (I), (I-A), (II-A), (II-B) and (II-C) and compounds of formula (I) selected from one compound as represented in Table A, Table B-1 to B-10, Table C-1 to C-102 or from one compound (P-1) to (P-39) listed in Table T1 (below).
a compound selected from the group of substances consisting of petroleum oils + TX, 1,1-bis(4-chlorophenyl)-2-ethoxyethanol + TX, 2,4-dichlorophenyl benzenesulfonate + TX, 2-fluoro-N-methyl-N-1-naphthylacetamide + TX, 4-chlorophenyl phenyl sulfone + TX, acetoprole + TX, aldoxycarb + TX, amidithion + TX, amidothioate + TX, amiton + TX, amiton hydrogen oxalate + TX, amitraz + TX, aramite + TX, arsenous oxide + TX, azobenzene + TX, azothoate + TX, benomyl + TX, benoxafos + TX, benzyl benzoate + TX, bixafen + TX, brofenvalerate + TX, bromocyclen + TX, bromophos + TX, bromopropylate + TX, buprofezin + TX, butocarboxim + TX, butoxycarboxim + TX, butylpyridaben + TX, calcium polysulfide + TX, camphechlor + TX, carbanolate + TX, carbophenothion + TX, cymiazole + TX, chinomethionat + TX, chlorbenside + TX, chlordimeform + TX, chlordimeform hydrochloride + TX, chlorfenethol + TX, chlorfenson + TX, chlorfensulfide + TX, chlorobenzilate + TX, chloromebuform + TX, chloromethiuron + TX, chloropropylate + TX, chlorthiophos + TX, cinerin I + TX, cinerin II + TX, cinerins + TX, closantel + TX, coumaphos + TX, crotamiton + TX, crotoxyphos + TX, cufraneb + TX, cyanthoate + TX, DCPM + TX, DDT + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, dichlofluanid + TX, dichlorvos + TX, dicliphos + TX, dienochlor + TX, dimefox + TX, dinex + TX, dinex-diclexine + TX, dinocap-4 + TX, dinocap-6 + TX, dinocton + TX, dinopenton + TX, dinosulfon + TX, dinoterbon + TX, dioxathion + TX, diphenyl sulfone + TX, disulfiram + TX, DNOC + TX, dofenapyn + TX, doramectin + TX, endothion + TX, eprinomectin + TX, ethoate-methyl + TX, etrimfos + TX, fenazaflor + TX, fenbutatin oxide + TX, fenothiocarb + TX, fenpyrad + TX, fenpyroximate + TX, fenpyrazamine + TX, fenson + TX, fentrifanil + TX, flubenzimine + TX, flucycloxuron + TX, fluenetil + TX, fluorbenside + TX, FMC 1137 + TX, formetanate + TX, formetanate hydrochloride + TX, formparanate + TX, gamma-HCH + TX, glyodin + TX, halfenprox + TX, hexadecyl cyclopropanecarboxylate + TX, isocarbophos + TX, jasmolin I + TX, jasmolin II + TX, jodfenphos + TX, lindane + TX, malonoben + TX, mecarbam + TX, mephosfolan + TX, mesulfen + TX, methacrifos + TX, methyl bromide + TX, metolcarb + TX, mexacarbate + TX, milbemycin oxime + TX, mipafox + TX, monocrotophos + TX, morphothion + TX, moxidectin + TX, naled + TX, 4-chloro-2-(2-chloro-2-methyl-propyl)-5-[(6-iodo-3-pyridyl)methoxy]pyridazin-3-one + TX, nifluridide + TX, nikkomycins + TX, nitrilacarb + TX, nitrilacarb 1:1 zinc chloride complex + TX, omethoate + TX, oxydeprofos + TX, oxydisulfoton + TX, pp'-DDT + TX, parathion + TX, permethrin + TX, phenkapton + TX, phosalone + TX, phosfolan + TX, phosphamidon + TX, polychloroterpenes + TX, polynactins + TX, proclonol + TX, promacyl + TX, propoxur + TX, prothidathion + TX, prothoate + TX, pyrethrin I + TX, pyrethrin II + TX, pyrethrins + TX, pyridaphenthion + TX, pyrimitate + TX, quinalphos + TX, quintiofos + TX, R-1492 + TX, phosglycin + TX, rotenone + TX, schradan + TX, sebufos + TX, selamectin + TX, sophamide + TX, SSI-121 + TX, sulfiram + TX, sulfluramid + TX, sulfotep + TX, sulfur + TX, diflovidazin + TX, tau-fluvalinate + TX, TEPP + TX, terbam + TX, tetradifon + TX, tetrasul + TX, thiafenox + TX, thiocarboxime + TX, thiofanox + TX, thiometon + TX, thioquinox + TX, thuringiensin + TX, triamiphos + TX, triarathene + TX, triazophos + TX, triazuron + TX, trifenofos + TX, trinactin + TX, vamidothion + TX, vaniliprole + TX, bethoxazin + TX, copper dioctanoate + TX, copper sulfate + TX, cybutryne + TX, dichlone + TX, dichlorophen + TX, endothal + TX, fentin + TX, hydrated lime + TX, nabam + TX, quinoclamine + TX, quinonamid + TX, simazine + TX, triphenyltin acetate + TX, triphenyltin hydroxide + TX, crufomate + TX, piperazine + TX, thiophanate + TX, chloralose + TX, fenthion + TX, pyridin-4-amine + TX, strychnine + TX, 1-hydroxy-1H-pyridine-2-thione + TX, 4-(quinoxalin-2-ylamino)benzenesulfonamide + TX, 8-hydroxyquinoline sulfate + TX, bronopol + TX, copper hydroxide + TX, cresol + TX, dipyrithione + TX, dodicin + TX, fenaminosulf + TX, formaldehyde + TX, hydrargaphen + TX, kasugamycin + TX, kasugamycin hydrochloride hydrate + TX, nickel bis(dimethyldithiocarbamate) + TX, nitrapyrin + TX, octhilinone + TX, oxolinic acid + TX, oxytetracycline + TX, potassium hydroxyquinoline sulfate + TX, probenazole + TX, streptomycin + TX, streptomycin sesquisulfate + TX, tecloftalam + TX, thiomersal + TX, Adoxophyes orana GV + TX, Agrobacterium radiobacter + TX, Amblyseius spp. + TX, Anagrapha falcifera NPV + TX, Anagrus atomus + TX, Aphelinus abdominalis + TX, Aphidius colemani + TX, Aphidoletes aphidimyza + TX, Autographa californica NPV + TX, Bacillus sphaericus Neide + TX, Beauveria brongniartii + TX, Chrysoperla carnea + TX, Cryptolaemus montrouzieri + TX, Cydia pomonella GV + TX, Dacnusa sibirica + TX, Diglyphus isaea + TX, Encarsia formosa + TX, Eretmocerus eremicus + TX, Heterorhabditis bacteriophora and H. megidis + TX, Hippodamia convergens + TX, Leptomastix dactylopii + TX, Macrolophus caliginosus + TX, Mamestra brassicae NPV + TX, Metaphycus helvolus + TX, Metarhizium anisopliae var. acridum + TX, Metarhizium anisopliae var. anisopliae + TX, Neodiprion sertifer NPV and N. lecontei NPV + TX, Orius spp. + TX, Paecilomyces fumosoroseus + TX, Phytoseiulus persimilis + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Trichogramma spp. + TX, Typhlodromus occidentalis + TX, Verticillium lecanii + TX, apholate + TX, bisazir + TX, busulfan + TX, dimatif + TX, hemel + TX, hempa + TX, metepa + TX, methiotepa + TX, methyl apholate + TX, morzid + TX, penfluron + TX, tepa + TX, thiohempa + TX, thiotepa + TX, tretamine + TX, uredepa + TX, (E)-dec-5-en-1-yl acetate with (E)-dec-5-en-1-ol + TX, (E)-tridec-4-en-1-yl acetate + TX, (E)-6-methylhept-2-en-4-ol + TX, (E,Z)-tetradeca-4,10-dien-1-yl acetate + TX, (Z)-dodec-7-en-1-yl acetate + TX, (Z)-hexadec-11-enal + TX, (Z)-hexadec-11-en-1-yl acetate + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, 14-methyloctadec-1-ene + TX, 4-methylnonan-5-ol with 4-methylnonan-5-one + TX, alpha-multistriatin + TX, brevicomin + TX, codlelure + TX, codlemone + TX, cuelure + TX, disparlure + TX, dodec-8-en-1-yl acetate + TX, dodec-9-en-1-yl acetate + TX, dodeca-8 + TX, 10-dien-1-yl acetate + TX, dominicalure + TX, ethyl 4-methyloctanoate + TX, eugenol + TX, frontalin + TX, grandlure + TX, grandlure I + TX, grandlure II + TX, grandlure III + TX, grandlure IV + TX, hexalure + TX, ipsdienol + TX, ipsenol + TX, japonilure + TX, lineatin + TX, litlure + TX, looplure + TX, medlure + TX, megatomoic acid + TX, methyl eugenol + TX, muscalure + TX, octadeca-2,13-dien-1-yl acetate + TX, octadeca-3,13-dien-1-yl acetate + TX, orfralure + TX, oryctalure + TX, ostramone + TX, siglure + TX, sordidin + TX, sulcatol + TX, tetradec-11-en-1-yl acetate + TX, trimedlure + TX, trimedlure A + TX, trimedlure B₁ + TX, trimedlure B₂ + TX, trimedlure C + TX, trunc-call + TX, 2-(octylthio)ethanol + TX, butopyronoxyl + TX, butoxy(polypropylene glycol) + TX, dibutyl adipate + TX, dibutyl phthalate + TX, dibutyl succinate + TX, diethyltoluamide + TX, dimethyl carbate + TX, dimethyl phthalate + TX, ethyl hexanediol + TX, hexamide + TX, methoquin-butyl + TX, methylneodecanamide + TX, oxamate + TX, picaridin + TX, 1-dichloro-1-nitroethane + TX, 1,1-dichloro-2,2-bis(4-ethylphenyl)ethane + TX, 1,2-dichloropropane with 1,3-dichloropropene + TX, 1-bromo-2-chloroethane + TX, 2,2,2-trichloro-1-(3,4-dichlorophenyl)ethyl acetate + TX, 2,2-dichlorovinyl 2-ethylsulfinylethyl methyl phosphate + TX, 2-(1,3-dithiolan-2-yl)phenyl dimethylcarbamate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-chlorovinyl diethyl phosphate + TX, 2-imidazolidone + TX, 2-isovalerylindan-1,3-dione + TX, 2-methyl(prop-2-ynyl)aminophenyl methylcarbamate + TX, 2-thiocyanatoethyl laurate + TX, 3-bromo-1-chloroprop-1-ene + TX, 3-methyl-1-phenylpyrazol-5-yl dimethylcarbamate + TX, 4-methyl(prop-2-ynyl)amino-3,5-xylyl methylcarbamate + TX, 5,5-dimethyl-3-oxocyclohex-1-enyl dimethylcarbamate + TX, acethion + TX, acrylonitrile + TX, aldrin + TX, allosamidin + TX, allyxycarb + TX, alpha-ecdysone + TX, aluminium phosphide + TX, aminocarb + TX, anabasine + TX, athidathion + TX, azamethiphos + TX, Bacillus thuringiensis delta endotoxins + TX, barium hexafluorosilicate + TX, barium polysulfide + TX, barthrin + TX, Bayer 22/190 + TX, Bayer 22408 + TX, beta-cyfluthrin + TX, beta-cypermethrin + TX, bioethanomethrin + TX, biopermethrin + TX, bis(2-chloroethyl) ether + TX, borax + TX, bromfenvinfos + TX, bromo-DDT + TX, bufencarb + TX, butacarb + TX, butathiofos + TX, butonate + TX, calcium arsenate + TX, calcium cyanide + TX, carbon disulfide + TX, carbon tetrachloride + TX, cartap hydrochloride + TX, cevadine + TX, chlorbicyclen + TX, chlordane + TX, chlordecone + TX, chloroform + TX, chloropicrin + TX, chlorphoxim + TX, chlorprazophos + TX, cis-resmethrin + TX, cismethrin + TX, clocythrin + TX, copper acetoarsenite + TX, copper arsenate + TX, copper oleate + TX, coumithoate + TX, cryolite + TX, CS 708 + TX, cyanofenphos + TX, cyanophos + TX, cyclethrin + TX, cythioate + TX, d-tetramethrin + TX, DAEP + TX, dazomet + TX, decarbofuran + TX, diamidafos + TX, dicapthon + TX, dichlofenthion + TX, dicresyl + TX, dicyclanil + TX, dieldrin + TX, diethyl 5-methylpyrazol-3-yl phosphate + TX, dilor + TX, dimefluthrin + TX, dimetan + TX, dimethrin + TX, dimethylvinphos + TX, dimetilan + TX, dinoprop + TX, dinosam + TX, dinoseb + TX, diofenolan + TX, dioxabenzofos + TX, dithicrofos + TX, DSP + TX, ecdysterone + TX, El 1642 + TX, EMPC + TX, EPBP + TX, etaphos + TX, ethiofencarb + TX, ethyl formate + TX, ethylene dibromide + TX, ethylene dichloride + TX, ethylene oxide + TX, EXD + TX, fenchlorphos + TX, fenethacarb + TX, fenitrothion + TX, fenoxacrim + TX, fenpirithrin + TX, fensulfothion + TX, fenthion-ethyl + TX, flucofuron + TX, fosmethilan + TX, fospirate + TX, fosthietan + TX, furathiocarb + TX, furethrin + TX, guazatine + TX, guazatine acetates + TX, sodium tetrathiocarbonate + TX, halfenprox + TX, HCH + TX, HEOD + TX, heptachlor + TX, heterophos + TX, HHDN + TX, hydrogen cyanide + TX, hyquincarb + TX, IPSP + TX, isazofos + TX, isobenzan + TX, isodrin + TX, isofenphos + TX, isolane + TX, isoprothiolane + TX, isoxathion + TX, juvenile hormone I + TX, juvenile hormone II + TX, juvenile hormone III + TX, kelevan + TX, kinoprene + TX, lead arsenate + TX, leptophos + TX, lirimfos + TX, lythidathion + TX, m-cumenyl methylcarbamate + TX, magnesium phosphide + TX, mazidox + TX, mecarphon + TX, menazon + TX, mercurous chloride + TX, mesulfenfos + TX, metam + TX, metam-potassium + TX, metam-sodium + TX, methanesulfonyl fluoride + TX, methocrotophos + TX, methoprene + TX, methothrin + TX, methoxychlor + TX, methyl isothiocyanate + TX, methylchloroform + TX, methylene chloride + TX, metoxadiazone + TX, mirex + TX, naftalofos + TX, naphthalene + TX, NC-170 + TX, nicotine + TX, nicotine sulfate + TX, nithiazine + TX, nornicotine + TX, O-5-dichloro-4-iodophenyl O-ethyl ethylphosphonothioate + TX, O,O-diethyl O-4-methyl-2-oxo-2H-chromen-7-yl phosphorothioate + TX, O,O-diethyl O-6-methyl-2-propylpyrimidin-4-yl phosphorothioate + TX, O,O,O',O'-tetrapropyl dithiopyrophosphate + TX, oleic acid + TX, para-dichlorobenzene + TX, parathion-methyl + TX, pentachlorophenol + TX, pentachlorophenyl laurate + TX, PH 60-38 + TX, phenkapton + TX, phosnichlor + TX, phosphine + TX, phoxim-methyl + TX, pirimetaphos + TX, polychlorodicyclopentadiene isomers + TX, potassium arsenite + TX, potassium thiocyanate + TX, precocene I + TX, precocene II + TX, precocene III + TX, primidophos + TX, profluthrin + TX, promecarb + TX, prothiofos + TX, pyrazophos + TX, pyresmethrin + TX, quassia + TX, quinalphos-methyl + TX, quinothion + TX, rafoxanide + TX, resmethrin + TX, rotenone + TX, kadethrin + TX, ryania + TX, ryanodine + TX, sabadilla) + TX, schradan + TX, sebufos + TX, SI-0009 + TX, thiapronil + TX, sodium arsenite + TX, sodium cyanide + TX, sodium fluoride + TX, sodium hexafluorosilicate + TX, sodium pentachlorophenoxide + TX, sodium selenate + TX, sodium thiocyanate + TX, sulcofuron + TX, sulcofuron-sodium + TX, sulfuryl fluoride + TX, sulprofos + TX, tar oils + TX, tazimcarb + TX, TDE + TX, tebupirimfos + TX, temephos + TX, terallethrin + TX, tetrachloroethane + TX, thicrofos + TX, thiocyclam + TX, thiocyclam hydrogen oxalate + TX, thionazin + TX, thiosultap + TX, thiosultap-sodium + TX, tralomethrin + TX, transpermethrin + TX, triazamate + TX, trichlormetaphos-3 + TX, trichloronat + TX, trimethacarb + TX, tolprocarb + TX, triclopyricarb + TX, triprene + TX, veratridine + TX, veratrine + TX, XMC + TX, zetamethrin + TX, zinc phosphide + TX, zolaprofos + TX, and meperfluthrin + TX, tetramethylfluthrin + TX, bis(tributyltin) oxide + TX, bromoacetamide + TX, ferric phosphate + TX, niclosamide-olamine + TX, tributyltin oxide + TX, pyrimorph + TX, trifenmorph + TX, 1,2-dibromo-3-chloropropane + TX, 1,3-dichloropropene + TX, 3,4-dichlorotetrahydrothiophene 1,1-dioxide + TX, 3-(4-chlorophenyl)-5-methylrhodanine + TX, 5-methyl-6-thioxo-1,3,5-thiadiazinan-3-ylacetic acid + TX, 6-isopentenylaminopurine + TX, 2-fluoro-N-(3-methoxyphenyl)-9H-purin-6-amine + TX, benclothiaz + TX, cytokinins + TX, DCIP + TX, furfural + TX, isamidofos + TX, kinetin + TX, Myrothecium verrucaria composition + TX, tetrachlorothiophene + TX, xylenols + TX, zeatin + TX, potassium ethylxanthate + TX, acibenzolar + TX, acibenzolar-S-methyl + TX, Reynoutria sachalinensis extract + TX, alpha-chlorohydrin + TX, antu + TX, barium carbonate + TX, bisthiosemi + TX, brodifacoum + TX, bromadiolone + TX, bromethalin + TX, chlorophacinone + TX, cholecalciferol + TX, coumachlor + TX, coumafuryl + TX, coumatetralyl + TX, crimidine + TX, difenacoum + TX, difethialone + TX, diphacinone + TX, ergocalciferol + TX, flocoumafen + TX, fluoroacetamide + TX, flupropadine + TX, flupropadine hydrochloride + TX, norbormide + TX, phosacetim + TX, phosphorus + TX, pindone + TX, pyrinuron + TX, scilliroside + TX, sodium fluoroacetate + TX, thallium sulfate + TX, warfarin + TX, 2-(2-butoxyethoxy)ethyl piperonylate + TX, 5-(1,3-benzodioxol-5-yl)-3-hexylcyclohex-2-enone + TX, farnesol with nerolidol + TX, verbutin + TX, MGK 264 + TX, piperonyl butoxide + TX, piprotal + TX, propyl isomer + TX, S421 + TX, sesamex + TX, sesasmolin + TX, sulfoxide + TX, anthraquinone + TX, copper naphthenate + TX, copper oxychloride + TX, dicyclopentadiene + TX, thiram + TX, zinc naphthenate + TX, ziram + TX, imanin + TX, ribavirin + TX, mercuric oxide + TX, thiophanate-methyl + TX, azaconazole + TX, bitertanol + TX, bromuconazole + TX, cyproconazole + TX, difenoconazole + TX, diniconazole + TX, epoxiconazole + TX, fenbuconazole + TX, fluquinconazole + TX, flusilazole + TX, flutriafol + TX, furametpyr + TX, hexaconazole + TX, imazalil + TX, imibenconazole + TX, ipconazole + TX, metconazole + TX, myclobutanil + TX, paclobutrazole + TX, pefurazoate + TX, penconazole + TX, prothioconazole + TX, pyrifenox + TX, prochloraz + TX, propiconazole + TX, pyrisoxazole + TX, simeconazole + TX, tebuconazole + TX, tetraconazole + TX, triadimefon + TX, triadimenol + TX, triflumizole + TX, triticonazole + TX, ancymidol + TX, fenarimol + TX, nuarimol + TX, bupirimate + TX, dimethirimol + TX, ethirimol + TX, dodemorph + TX, fenpropidin + TX, fenpropimorph + TX, spiroxamine + TX, tridemorph + TX, cyprodinil + TX, mepanipyrim + TX, pyrimethanil + TX, fenpiclonil + TX, fludioxonil + TX, benalaxyl + TX, furalaxyl + TX, metalaxyl -+ TX, Rmetalaxyl + TX, ofurace + TX, oxadixyl + TX, carbendazim + TX, debacarb + TX, fuberidazole + TX, thiabendazole + TX, chlozolinate + TX, dichlozoline + TX, myclozoline + TX, procymidone + TX, vinclozoline + TX, boscalid + TX, carboxin + TX, fenfuram + TX, flutolanil + TX, mepronil + TX, oxycarboxin + TX, penthiopyrad + TX, thifluzamide + TX, dodine + TX, iminoctadine + TX, azoxystrobin + TX, dimoxystrobin + TX, enestroburin + TX, fenaminstrobin + TX, flufenoxystrobin + TX, fluoxastrobin + TX, kresoxim-methyl + TX, metominostrobin + TX, trifloxystrobin + TX, orysastrobin + TX, picoxystrobin + TX, pyraclostrobin + TX, pyrametostrobin + TX, pyraoxystrobin + TX, ferbam + TX, mancozeb + TX, maneb + TX, metiram + TX, propineb + TX, zineb + TX, captafol + TX, captan + TX, fluoroimide + TX, folpet + TX, tolylfluanid + TX, bordeaux mixture + TX, copper oxide + TX, mancopper + TX, oxine-copper + TX, nitrothal-isopropyl + TX, edifenphos + TX, iprobenphos + TX, phosdiphen + TX, tolclofos-methyl + TX, anilazine + TX, benthiavalicarb + TX, blasticidin-S + TX, chloroneb + TX, chlorothalonil + TX, cyflufenamid + TX, cymoxanil + TX, cyclobutrifluram + TX, diclocymet + TX, diclomezine + TX, dicloran + TX, diethofencarb + TX, dimethomorph + TX, flumorph + TX, dithianon + TX, ethaboxam + TX, etridiazole + TX, famoxadone + TX, fenamidone + TX, fenoxanil + TX, ferimzone + TX, fluazinam + TX, fluopicolide + TX, flusulfamide + TX, fluxapyroxad + TX, fenhexamid + TX, fosetyl-aluminium + TX, hymexazol + TX, iprovalicarb + TX, cyazofamid + TX, methasulfocarb + TX, metrafenone + TX, pencycuron + TX, phthalide + TX, polyoxins + TX, propamocarb + TX, pyribencarb + TX, proquinazid + TX, pyroquilon + TX, pyriofenone + TX, quinoxyfen + TX, quintozene + TX, tiadinil + TX, triazoxide + TX, tricyclazole + TX, triforine + TX, validamycin + TX, valifenalate + TX, zoxamide + TX, mandipropamid + TX, flubeneteram + TX, isopyrazam + TX, sedaxane + TX, benzovindiflupyr + TX, pydiflumetofen + TX, 3-difluoromethyl-1-methyl-1H-pyrazole-4-carboxylic acid (3',4',5'-trifluoro-biphenyl-2-yl)-amide + TX, isoflucypram + TX, isotianil + TX, dipymetitrone + TX, 6-ethyl-5,7-dioxo-pyrrolo[4,5][1,4]dithiino[1,2-c]isothiazole-3-carbonitrile + TX, 2-(difluoromethyl)-N-[3-ethyl-1,1-dimethyl-indan-4-yl]pyridine-3-carboxamide + TX, 4-(2,6-difluorophenyl)-6-methyl-5-phenyl-pyridazine-3-carbonitrile + TX, (R)-3-(difluoromethyl)-1-methyl-N-[1,1,3-trimethylindan-4-yl]pyrazole-4-carboxamide + TX, 4-(2-bromo-4-fluoro-phenyl)-N-(2-chloro-6-fluoro-phenyl)-2,5-dimethyl-pyrazol-3-amine + TX, 4- (2- bromo- 4- fluorophenyl) - N- (2- chloro- 6- fluorophenyl) - 1, 3- dimethyl- 1H- pyrazol- 5-amine + TX, fluindapyr + TX, coumethoxystrobin (jiaxiangjunzhi) + TX, Ivbenmixianan + TX, dichlobentiazox + TX, mandestrobin + TX, 3-(4,4-difluoro-3,4-dihydro-3,3-dimethylisoquinolin-1-yl)quinolone + TX, 2-[2-fluoro-6-[(8-fluoro-2-methyl-3-quinolyl)oxy]phenyl]propan-2-ol + TX, oxathiapiprolin + TX, tert-butyl N-[6-[[[(1-methyltetrazol-5-yl)-phenyl-methylene]amino]oxymethyl]-2-pyridyl]carbamate + TX, pyraziflumid + TX, inpyrfluxam + TX, trolprocarb + TX, mefentrifluconazole + TX, ipfentrifluconazole+ TX, 2-(difluoromethyl)-N-[(3R)-3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine + TX, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate + TX, but-3-ynyl N-[6-[[(Z)-[(1-methyltetrazol-5-yl)-phenyl-methylene]amino]oxymethyl]-2-pyridyl]carbamate + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine + TX, pyridachlometyl + TX, 3-(difluoromethyl)-1-methyl-N-[1,1,3-trimethylindan-4-yl]pyrazole-4-carboxamide + TX, 1-[2-[[1-(4-chlorophenyl)pyrazol-3-yl]oxymethyl]-3-methyl-phenyl]-4-methyl-tetrazol-5-one + TX, 1-methyl-4-[3-methyl-2-[[2-methyl-4-(3,4,5-trimethylpyrazol-1-yl)phenoxy]methyl]phenyl]tetrazol-5-one + TX, aminopyrifen + TX, ametoctradin + TX, amisulbrom + TX, penflufen + TX, (Z,2E)-5-[1-(4-chlorophenyl)pyrazol-3-yl]oxy-2-methoxyimino-N,3-dimethyl-pent-3-enamide + TX, florylpicoxamid + TX, fenpicoxamid + TX, tebufloquin + TX, ipflufenoquin + TX, quinofumelin + TX, isofetamid + TX, N-[2-[2,4-dichloro-phenoxy]phenyl]-3-(difluoromethyl)-1-methylpyrazole-4-carboxamide + TX, N-[2-[2-chloro-4-(trifluoromethyl)phenoxy]phenyl]-3-(difluoromethyl)-1-methyl-pyrazole-4-carboxamide + TX, benzothiostrobin + TX, phenamacril + TX, 5-amino-1,3,4-thiadiazole-2-thiol zinc salt (2:1) + TX, fluopyram + TX, flutianil + TX, fluopimomide + TX, pyrapropoyne + TX, picarbutrazox + TX, 2-(difluoromethyl)-N-(3-ethyl-1,1-dimethyl-indan-4-yl)pyridine-3-carboxamide + TX, 2- (difluoromethyl) - N- ((3R) - 1, 1, 3- trimethylindan- 4- yl) pyridine- 3- carboxamide + TX, 4-[[6-[2-(2,4-difluorophenyl)-1,1-difluoro-2-hydroxy-3-(1,2,4-triazol-1-yl)propyl]-3-pyridyl]oxy]benzonitrile + TX, metyltetraprole + TX, 2- (difluoromethyl) - N- ((3R) - 1, 1, 3- trimethylindan- 4- yl) pyridine- 3- carboxamide + TX, a- (1, 1- dimethylethyl) - a- [4'- (trifluoromethoxy) [1, 1'- biphenyl] - 4- yl] -5- pyrimidinemethanol + TX, fluoxapiprolin + TX, enoxastrobin + TX, 4-[[6-[2-(2,4-difluorophenyl)-1,1-difluoro-2-hydroxy-3-(1,2,4-triazol-1-yl)propyl]-3-pyridyl]oxy] benzonitrile + TX, 4-[[6-[2-(2,4-difluorophenyl)-1,1-difluoro-2-hydroxy-3-(5-sulfanyl-1 ,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile + TX, trinexapac + TX, coumoxystrobin + TX, zhongshengmycin + TX, thiodiazole copper + TX, zinc thiazole + TX, amectotractin + TX, iprodione + TX, N-octyl-N'-[2-(octylamino)ethyl]ethane-1,2-diamine + TX; N'-[5-bromo-2-methyl-6-[(1S)-1-methyl-2-propoxy-ethoxy]-3-pyridyl]-N-ethyl-N-methyl-formamidine + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine + TX (these compounds may be prepared from the methods described in WO2015/155075); N'-[5-bromo-2-methyl-6-(2-propoxypropoxy)-3-pyridyl]-N-ethyl-N-methyl-formamidine + TX (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine+ TX, N'-[4-(1-cyclopropyl-2,2,2-trifluoro-1-hydroxyethyl)-5-methoxy-2-methyl-phenyl]-N-isopropyl-N-methyl-formamidine + TX (these compounds may be prepared from the methods described in WO2018/228896); N-ethyl-N'-[5-methoxy-2-methyl-4-[2-trifluoromethyl)oxetan-2-yl]phenyl]-N-methyl-formamidine + TX, N-ethyl-N'-[5-methoxy-2-methyl-4-[2-trifuoromethyl)tetrahydrofuran-2-yl]phenyl]-N-methyl-formamidine + TX (these compounds may be prepared from the methods described in WO2019/110427); N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-3,3,3-trifluoro-1-methyl-propyl]-8-fluoro-quinoline-3-carboxamide + TX, N-[(1R)-1-benzyl-1,3-dimethyl-butyl]-7,8-difluoro-quinoline-3-carboxamide + TX, N-[(1S)-1-benzyl-1,3-dimethyl-butyl]-7,8-difluoro-quinoline-3-carboxamide + TX, 8-fluoro-N-[(1R)-1-[(3-fluorophenyl)methyl]-1,3-dimethyl-butyl]quinoline-3-carboxamide + TX, 8-fluoro-N-[(1S)-1-[(3-fluorophenyl)methyl]-1,3-dimethyl-butyl]quinoline-3-carboxamide + TX, N-[(1R)-1-benzyl-1,3-dimethyl-butyl]-8-fluoro-quinoline-3-carboxamide + TX, N-[(1S)-1-benzyl-1,3-dimethyl-butyl]-8-fluoro-quinoline-3-carboxamide + TX, N-((1R)-1-benzyl-3-chloro-1-methyl-but-3-enyl)-8-fluoro-quinoline-3-carboxamide + TX, N-((1S)-1-benzyl-3-chloro-1-methyl-but-3-enyl)-8-fluoro-quinoline-3-carboxamide + TX (these compounds may be prepared from the methods described in WO2017/153380); 1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 1-(6-chloro-7-methyl-pyrazolo[1,5-a]pyridin-3-yl)-4,4-difluoro-3,3-dimethyl-isoquinoline + TX (these compounds may be prepared from the methods described in WO2017/025510); 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 6-chloro-4,4-difluoro-3,3-dimethyl-1-(4-methylbenzimidazol-1-yl)isoquinoline + TX, 4,4-difluoro-1-(5-fluoro-4-methyl-benzimidazol-1-yl)-3,3-dimethyl-isoquinoline + TX, 3-(4,4-difluoro-3,3-dimethyl-1-isoquinolyl)-7,8-dihydro-6H-cyclopenta[e]benzimidazole + TX (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide + TX, N,2-dimethoxy-N-[[4-[5-(trifluoromethyl)-1,2,4-oxadiazol-3-yl]phenyl]methyl]propanamide + TX, N-ethyl-2-methyl-N-[[4-[5-(trifluoromethyl)-1,2,4-oxadiazol-3-yl]phenyl]methyl]propanamide + TX, 1-methoxy-3-methyl-1-[[4-[5-(trifluoromethyl)-1,2,4-oxadiazol-3-yl]phenyl]methyl]urea + TX, 1,3-dimethoxy-1-[[4-[5-(trifluoromethyl)-1,2,4-oxadiazol-3-yl]phenyl]methyl]urea + TX, 3-ethyl-1-methoxy-1-[[4-[5-(trifluoromethyl)-1,2,4-oxadiazol-3-yl]phenyl]methyl]urea + TX, N-[[4-[5-(trifluoromethyl)-1,2,4-oxadiazol-3-yl]phenyl]methyl]propanamide + TX, 4,4-dimethyl-2-[[4-[5-(trifluoromethyl)-1,2,4-oxadiazol-3-yl]phenyl]methyl]isoxazolidin-3-one + TX, 5,5-dimethyl-2-[[4-[5-(trifluoromethyl)-1,2,4-oxadiazol-3-yl]phenyl]methyl]isoxazolidin-3-one + TX, ethyl 1-[[4-[5-(trifluoromethyl)-1,2,4-oxadiazol-3-yl]phenyl]methyl]pyrazole-4-carboxylate + TX, N,N-dimethyl-1-[[4-[5-(trifluoromethyl)-1,2,4-oxadiazol-3-yl]phenyl]methyl]-1,2,4-triazol-3-amine + TX. The compounds in this paragraph may be prepared from the methods described in WO2017/055473, WO2017/055469, WO2017/093348 and WO2017/118689; 2-[6-(4-chlorophenoxy)-2-(trifluoromethyl)-3-pyridyl]-1-(1,2,4-triazol-1-yl)propan-2-ol + TX (this compound may be prepared from the methods described in WO2017/029179); 2-[6-(4-bromophenoxy)-2-(trifluoromethyl)-3-pyridyl]-1-(1,2,4-triazol-1-yl)propan-2-ol + TX (this compound may be prepared from the methods described in WO2017/029179); 3-[2-(1-chlorocyclopropyl)-3-(2-fluorophenyl)-2-hydroxy-propyl]imidazole-4-carbonitrile + TX (this compound may be prepared from the methods described in WO2016/156290); 3-[2-(1-chlorocyclopropyl)-3-(3-chloro-2-fluoro-phenyl)-2-hydroxy-propyl]imidazole-4-carbonitrile + TX (this compound may be prepared from the methods described in WO2016/156290); (4-phenoxyphenyl)methyl 2-amino-6-methyl-pyridine-3-carboxylate + TX (this compound may be prepared from the methods described in WO2014/006945); 2,6-Dimethyl-1H,5H-[1,4]dithiino[2,3-c:5,6-c']dipyrrole-1,3,5,7(2H,6H)-tetrone + TX (this compound may be prepared from the methods described in WO2011/138281); N-methyl-4-[5-(trifluoromethyl)-1,2,4-oxadiazol-3-yl]benzenecarbothioamide + TX; N-methyl-4-[5-(trifluoromethyl)-1,2,4-oxadiazol-3-yl]benzamide + TX; (Z,2E)-5-[1-(2,4-dichlorophenyl)pyrazol-3-yl]oxy-2-methoxyimino-N,3-dimethyl-pent-3-enamide + TX (this compound may be prepared from the methods described in WO2018/153707); N'-(2-chloro-5-methyl-4-phenoxy-phenyl)-N-ethyl-N-methyl-formamidine + TX; N'-[2-chloro-4-(2-fluorophenoxy)-5-methyl-phenyl]-N-ethyl-N-methyl-formamidine + TX (this compound may be prepared from the methods described in WO2016/202742); 2-(difluoromethyl)-N-[(3S)-3-ethyl-1,1-dimethyl-indan-4-yl]pyridine-3-carboxamide + TX (this compound may be prepared from the methods described in WO2014/095675); (5-methyl-2-pyridyl)-[4-[5-(trifluoromethyl)-1,2,4-oxadiazol-3-yl]phenyl]methanone + TX, (3-methylisoxazol-5-yl)-[4-[5-(trifluoromethyl)-1,2,4-oxadiazol-3-yl]phenyl]methanone + TX (these compounds may be prepared from the methods described in WO2017/220485); 2-oxo-N-propyl-2-[4-[5-(trifluoromethyl)-1,2,4-oxadiazol-3-yl]phenyl]acetamide + TX (this compound may be prepared from the methods described in WO2018/065414); ethyl 1-[[5-[5-(trifluoromethyl)-1,2,4-oxadiazol-3-yl]-2-thienyl]methyl]pyrazole-4-carboxylate + TX (this compound may be prepared from the methods described in WO2018/158365) ; 2,2-difluoro-N-methyl-2-[4-[5-(trifluoromethyl)-1,2,4-oxadiazol-3-yl]phenyl]acetamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[N-methoxy-C-methyl-carbonimidoyl]-4-[5-(trifluoromethyl)-1,2,4-oxadiazol-3-yl]benzamide + TX (these compounds may be prepared from the methods described in WO2018/202428), chloroinconazide + TX, flumetylsulforim + TX, fluoxytioconazole + TX, flufenoxadiazam +TX, metarylpicoxamid +TX.

The references in brackets behind the active ingredients, e.g. *[*3878-19-1*]* refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula (I), (I-A), (II-A), (II-B) and (II-C)and compounds of formula (I) selected from one compound as represented in Table A, Table B-1 to B-10, Table C-1 to C-102 or from one compound (P-1) to (P-39) listed in Table T1 (below) is preferably in a mixing ratio of from 100:1 to 1:100, especially from 50:1 to 1:50, more especially in a ratio of from 20:1 to 1:20, even more especially from 10:1 to 1:10, still more preferably from 2:1 to 1:2. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of formula (I), (I-A), (II-A), (II-B) and (II-C) or compounds of formula (I) selected from one compound as represented in Table A, Table B-1 to B-10, Table C-1 to C-102 or from one compound (P-1) to (P-39) listed in Table T1 (below), and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying a compound of formula (I), (I-A), (II-A), (II-B) and (II-C) or compounds of formula (I) selected from one compound as represented in Table A, Table B-1 to B-10, Table C-1 to C-102 or from one compound (P-1) to (P-39) listed in Table T1 (below), and the active ingredient(s) as described above, is not essential for working the present invention.The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds (I) for the preparation of these compositions are also a subject of the invention.

Another aspect of the invention is related to the use of a compound of formula (I) according to the invention or of a preferred individual compound as defined herein, of a composition comprising at least one compound of formula (I) or at least one preferred individual compound as defined herein, or of a fungicidal or insecticidal mixture comprising at least one compound of formula (I) or at least one preferred individual compound as defined herein, in admixture with other fungicides or insecticides as described above, for controlling or preventing infestation of plants, e.g. useful plants such as crop plants, propagation material thereof, e.g. seeds, harvested crops, e.g. harvested food crops, or non-living materials by insects or by phytopathogenic microorganisms, preferably fungal organisms.

A further aspect of invention is related to a method of controlling or preventing an infestation of plants, e.g. useful plants such as crop plants, propagation material thereof, e.g. seeds, harvested crops, e.g. harvested food crops, or of non-living materials by phytopathogenic or spoilage microorganisms or organisms potentially harmful to man, especially fungal organisms, which comprises the application of a compound of formula (I) according to the invention or of a preferred individual compound as defined herein as active ingredient to the plants, to parts of the plants or to the locus thereof, to the propagation material thereof, or to any part of the non-living materials.

Controlling or preventing means reducing infestation by insects or by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, or insects which comprises the application of a compound of formula (I) according to the invention, or an agrochemical composition which contains at least one compound of formula (I), is foliar application. The frequency of application and the rate of application will depend on the risk of infestation by the corresponding pathogen or insect. However, the compounds of formula (I) according to the invention can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula (I) may also be applied to seeds (coating) by impregn¬ting the seeds or tubers either with a liquid formulation of the fungicide or coating them with a solid formulation.

A formulation, e.g. a composition containing the compound of formula (I) according to the invention and, if desired, a solid or liquid adjuvant or monomers for encapsulating the compound of formula (I), may be prepared in a known manner, typically by intimately mixing and/or grinding the compound with extenders, for example solvents, solid carriers and, optionally, surface active compounds (surfactants).

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient dosages are from 10mg to 1g of active substance per kg of seeds.

When the combinations of the present invention are used for treating seed, rates of 0.001 to 50 g of a compound of formula (I) per kg of seed, preferably from 0.01 to 10g per kg of seed are generally sufficient.

The term "g a.i./ha" as used herein refer to the application rate given in gramm [g ] of active ingredient [a.i.] per unit of surface [ha]. The unit hectare (symbol ha) is the metric unit of area that equals a square with 100 m side (1 hm²) or 10,000 square meters. Hectare is a commonly used unit of area in the metric system.

Suitably, a composition comprising a compound of formula (I) according to the present invention is applied either preventative, meaning prior to disease development or curative, meaning after disease development.

The compositions of the invention may be employed in any conventional form, for example in the form of a twin pack, a powder for dry seed treatment (DS), an emulsion for seed treatment (ES), a flowable concentrate for seed treatment (FS), a solution for seed treatment (LS), a water dispersible powder for seed treatment (WS), a capsule suspension for seed treatment (CF), a gel for seed treatment (GF), an emulsion concen¬trate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP) or any technically feasible formulation in combination with agriculturally acceptable adjuvants.

Such compositions may be produced in conventional manner, e.g. by mixing the active ingre¬dients with appropriate formulation inerts (diluents, solvents, fillers and optionally other formulating ingredients such as surfactants, biocides, anti-freeze, stickers, thickeners and compounds that provide adjuvancy effects). Also conventional slow release formulations may be employed where long lasting efficacy is intended. Particularly formulations to be applied in spraying forms, such as water dispersible concentrates (e.g. EC, SC, DC, OD, SE, EW, EO and the like), wettable powders and granules, may contain surfactants such as wetting and dispersing agents and other compounds that provide adjuvancy effects, e.g. the ondensation product of formaldehyde with naphthalene sulphonate, an alkylarylsulphonate, a lignin sulphonate, a fatty alkyl sulphate, and ethoxylated alkylphenol and an ethoxylated fatty alcohol.

A seed dressing formulation is applied in a manner known per se to the seeds employing the combination of the invention and a diluent in suitable seed dressing formulation form, e.g. as an aqueous suspension or in a dry powder form having good adherence to the seeds. Such seed dressing formulations are known in the art. Seed dressing formulations may contain the single active ingredients or the combination of active ingredients in encapsulated form, e.g. as slow release capsules or microcapsules.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of formula (I) according to the invention optionally together with other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

Whereas it is preferred to formulate commercial products as concentrates, the end user will normally use dilute formulations.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The disclosure in the present application makes available each and every combination of embodiments disclosed herein.

The compounds according to the following Tables B-1 to B-10 may be prepared according to the methods described above. The examples which follow are intended to illustrate the invention and show preferred compounds of formula (I). In any of Tables B-1 to B-10 below, the presence of one or more possible asymmetric carbon atoms in a compound of formula (I) according to the invention means that the compounds may occur in chiral isomeric forms, i.e., enantiomeric or diastereomeric forms.
**Table B-1:** This table discloses 102 compounds B-1.001 to B-1.102 of formula (la) according to the invention, wherein X² and X³ are CR⁸ and R⁸ is hydrogen: 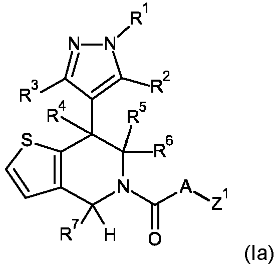 wherein R¹, R², R³, R⁴, R⁵, R⁶, R⁷, A and Z¹ are as defined in Tables C-1 to C-102 below.
   For example, compound B-1.003 has the following structure: 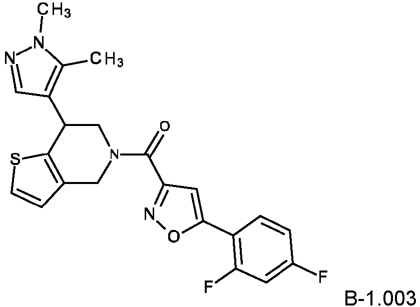
**Table B-2**: This table discloses 102 compounds B-2.001 to B-2.102 of formula (Ib) according to the invention, wherein X² is CH, and X³ is CCl: 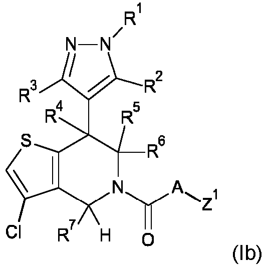 wherein R¹, R², R³, R⁴, R⁵, R⁶, R⁷, A and Z¹ are as defined in Tables C-1 to C-102 below.
**Table B-3:** This table discloses 102 compounds B-3.001 to B-3.102 of formula (Ic) according to the invention, wherein X² is CCI, and X² is CH: 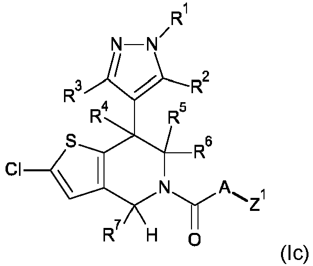 wherein R¹, R², R³, R⁴, R⁵, R⁶, R⁷, A and Z¹ are as defined in Tables C-1 to C-102 below.
**Table B-4**: This table discloses 102 compounds B-4.001 to B-4.102 formula (Id) according to the invention, wherein X² and X³ are CCl: 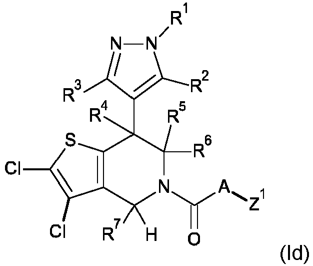 wherein R¹, R², R³, R⁴, R⁵, R⁶, R⁷, A and Z¹ are as defined in Tables C-1 to C-102 below.
**Table B-5:** This table discloses 102 compounds B-5.001 to B-5.102 of formula (le) according to the invention, wherein X¹ and X³ are CR⁸ and R⁸ is hydrogen: 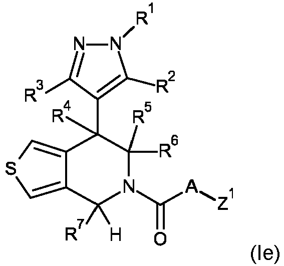 wherein R¹, R², R³, R⁴, R⁵, R⁶, R⁷, A and Z¹ are as defined in Tables C-1 to C-102 below.
**Table B-6**: This table discloses 102 compounds B-6.001 to B-6.102 of formula (If) according to the invention, wherein X¹ and X² are CR⁸ and R⁸ is hydrogen: 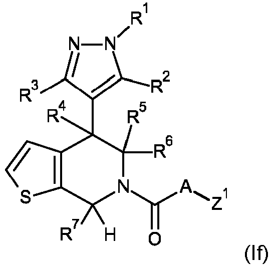 wherein R¹, R², R³, R⁴, R⁵, R⁶, R⁷, A and Z¹ are as defined in Tables C-1 to C-102 below.
**Table B-7:** This table discloses 102 compounds B-7.001 to B-7.102 of formula (Ig) according to the invention, wherein X¹ and CH, and X² is CCl: 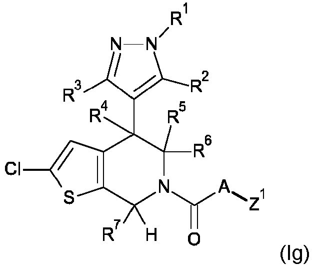 wherein R¹, R², R³, R⁴, R⁵, R⁶, R⁷, A and Z¹ are as defined in Tables C-1 to C-102 below.
**Table B-8:** This table discloses 102 compounds B-8.001 to B-8.102 of formula (Ih) according to the invention, wherein X² are CR⁸ and R⁸ is hydrogen: 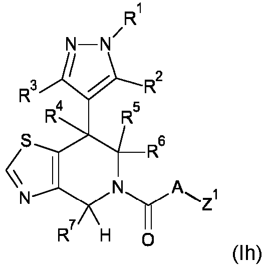 wherein R¹, R², R³, R⁴, R⁵, R⁶, R⁷, A and Z¹ are as defined in Tables C-1 to C-102 below.
**Table B-9:** This table discloses 102 compounds B-9.001 to B-9.102 of formula (li) according to the invention, wherein X² is CR⁸ and R⁸ is chlorine: 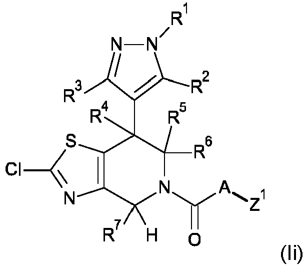 wherein R¹, R², R³, R⁴, R⁵, R⁶, R⁷, A and Z¹ are as defined in Tables C-1 to C-102 below.
**Table B-10:** This table discloses 102 compounds B-10.001 to B-10.102 of formula (li) according to the invention: 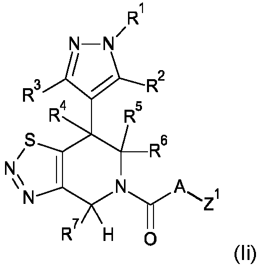 wherein R¹, R², R³, R⁴, R⁵, R⁶, R⁷, A and Z¹ are as defined in Tables C-1 to C-102 below.
**Table C-1:** This table provides compounds wherein R¹ is methyl, R², R³, R⁴, R⁵, R⁶ and R⁷ are hydrogen, A is A1 and Z¹ is 2,4-difluorophenyl.
**Table C-2:** This table provides compounds wherein R¹ is methyl, R², R³, R⁴, R⁵, R⁶ and R⁷ are hydrogen, A is A4 and Z¹ is 2,4-difluorophenyl.
**Table C-3:** This table provides compounds wherein R¹ is methyl, R², R³, R⁴, R⁵, R⁶ and R⁷ are hydrogen, A is A9 and Z¹ is 2,4-difluorophenyl.
**Table C-4:** This table provides compounds wherein R¹ is methyl, R², R³, R⁴, R⁵, R⁶ and R⁷ are hydrogen, A is A10 and Z¹ is 2,4-difluorophenyl.
**Table C-5:** This table provides compounds wherein R¹ is methyl, R² is methyl, R³, R⁴, R⁵, R⁶ and R⁷ are hydrogen, A is A1 and Z¹ is 2,4-difluorophenyl.
**Table C-6:** This table provides compounds wherein R¹ is methyl, R² is methyl, R³, R⁴, R⁵, R⁶ and R⁷ are hydrogen, A is A4 and Z¹ is 2,4-difluorophenyl.
**Table C-7:** This table provides compounds wherein R¹ is methyl, R² is methyl, R³, R⁴, R⁵, R⁶ and R⁷ are hydrogen, A is A9 and Z¹ is 2,4-difluorophenyl.
**Table C-8:** This table provides compounds wherein R¹ is methyl, R² is methyl, R³, R⁴, R⁵, R⁶ and R⁷ are hydrogen, A is A10 and Z¹ is 2,4-difluorophenyl.
**Table C-9:** This table provides compounds wherein R¹ is methyl, R² is methyl, R³ is methyl, R⁴, R⁵, R⁶ and R⁷ are hydrogen, A is A1 and Z¹ is 2,4-difluorophenyl.
**Table C-10:** This table provides compounds wherein R¹ is methyl, R² is methyl, R³ is methyl, R⁴, R⁵, R⁶ and R⁷ are hydrogen, A is A4 and Z¹ is 2,4-difluorophenyl.
**Table C-11:** This table provides compounds wherein R¹ is methyl, R² is methyl, R³ is methyl, R⁴, R⁵, R⁶ and R⁷ are hydrogen, A is A9 and Z¹ is 2,4-difluorophenyl.
**Table C-12:** This table provides compounds wherein R¹ is methyl, R² is methyl, R³ is methyl, R⁴, R⁵, R⁶ and R⁷ are hydrogen, A is A10 and Z¹ is 2,4-difluorophenyl.
**Table C-13:** This table provides compounds wherein R¹ is methyl, R⁴ is methyl, R², R³, R⁵, R⁶ and R⁷ are hydrogen, A is A1 and Z¹ is 2,4-difluorophenyl.
**Table C-14:** This table provides compounds wherein R¹ is methyl, R⁴ is methyl, R², R³, R⁵, R⁶ and R⁷ are hydrogen, A is A4 and Z¹ is 2,4-difluorophenyl.
**Table C-15:** This table provides compounds wherein R¹ is methyl, R⁴ is methyl, R², R³, R⁵, R⁶ and R⁷ are hydrogen, A is A9 and Z¹ is 2,4-difluorophenyl.
**Table C-16:** This table provides compounds wherein R¹ is methyl, R⁴ is methyl, R², R³, R⁵, R⁶ and R⁷ are hydrogen, A is A10 and Z¹ is 2,4-difluorophenyl.
**Table C-17:** This table provides compounds wherein R¹ is methyl, R⁷ is methyl, R², R³, R⁴, R⁵ and R^{6 7} are hydrogen, A is A1 and Z¹ is 2,4-difluorophenyl.
**Table C-18:** This table provides compounds wherein R¹ is methyl, R⁷ is methyl, R², R³, R⁴, R⁵ and R^{6 7} are hydrogen, A is A4 and Z¹ is 2,4-difluorophenyl.
**Table C-19:** This table provides compounds wherein R¹ is methyl, R⁷ is methyl, R², R³, R⁴, R⁵ and R^{6 7} are hydrogen, A is A9 and Z¹ is 2,4-difluorophenyl.
**Table C-20:** This table provides compounds wherein R¹ is methyl, R⁷ is methyl, R², R³, R⁴, R⁵ and R^{6 7} are hydrogen, A is A10 and Z¹ is 2,4-difluorophenyl.
**Table C-21:** This table provides compounds wherein R¹ is methyl, R⁴ is methyl, R⁷ is methyl, R², R³, R⁵ and R⁶ are hydrogen, A is A1 and Z¹ is 2,4-difluorophenyl.
**Table C-22:** This table provides compounds wherein R¹ is methyl, R⁴ is methyl, R⁷ is methyl, R², R³, R⁵ and R⁶ are hydrogen, A is A4 and Z¹ is 2,4-difluorophenyl.
**Table C-23:** This table provides compounds wherein R¹ is methyl, R⁴ is methyl, R⁷ is methyl, R², R³, R⁵ and R⁶ are hydrogen, A is A9 and Z¹ is 2,4-difluorophenyl.
**Table C-24:** This table provides compounds wherein R¹ is methyl, R⁴ is methyl, R⁷ is methyl, R², R³, R⁵ and R⁶ are hydrogen, A is A10 and Z¹ is 2,4-difluorophenyl.
**Table C-25:** This table provides compounds wherein R¹ is methyl, R² is chlorine, and R³, R⁴, R⁵, R⁶ and R⁷ are hydrogen, A is A4 and Z¹ is 2,4-difluorophenyl.
**Table C-26:** This table provides compounds wherein R¹ is methyl, R² is chlorine, and R³, R⁴, R⁵, R⁶ and R⁷ are hydrogen, A is A9 and Z¹ is 2,4-difluorophenyl.
**Table C-27:** This table provides compounds wherein R¹ is methyl, R² is chlorine, and R³, R⁴, R⁵, R⁶ and R⁷ are hydrogen, A is A10 and Z¹ is 2,4-difluorophenyl.
**Table C-28:** This table provides compounds wherein R¹ and R⁴ are methyl, R² is chlorine, and R³, R⁵, R⁶ and R⁷ are hydrogen, A is A4 and Z¹ is 2,4-difluorophenyl.
**Table C-29:** This table provides compounds wherein R¹ and R⁴ are methyl, R² is chlorine, and R³, R⁵, R⁶ and R⁷ are hydrogen, A is A9 and Z¹ is 2,4-difluorophenyl.
**Table C-30:** This table provides compounds wherein R¹ and R⁴ are methyl, R² is chlorine, and R³, R⁵, R⁶ and R⁷ are hydrogen, A is A10 and Z¹ is 2,4-difluorophenyl.
**Table C-31:** This table provides compounds wherein R¹ and R⁷ are methyl, R² is chlorine, and R³, R⁴, R⁵, and R⁶ are hydrogen, A is A4 and Z¹ is 2,4-difluorophenyl.
**Table C-32:** This table provides compounds wherein R¹ and R⁷ are methyl, R² is chlorine, and R³, R⁴, R⁵, and R⁶ are hydrogen, A is A9 and Z¹ is 2,4-difluorophenyl.
**Table C-33:** This table provides compounds wherein R¹ and R⁷ are methyl, R² is chlorine, and R³, R⁴, R⁵, and R⁶ are hydrogen, A is A10 and Z¹ is 2,4-difluorophenyl.
**Table C-34:** This table provides compounds wherein R', R⁴ and R⁷ are methyl, R² is chlorine, and R³, R⁵, and R⁶ are hydrogen, A is A4 and Z¹ is 2,4-difluorophenyl.
**Table C-35:** This table provides compounds wherein R¹, R⁴ and R⁷ are methyl, R² is chlorine, and R³, R⁵, and R⁶ are hydrogen, A is A9 and Z¹ is 2,4-difluorophenyl.
**Table C-36:** This table provides compounds wherein R¹, R⁴ and R⁷ are methyl, R² is chlorine, and R³, R⁵, and R⁶ are hydrogen, A is A10 and Z¹ is 2,4-difluorophenyl.
**Table C-37:** This table provides compounds wherein R¹ is methyl, R² is fluorine, and R³, R⁴, R⁵, R⁶ and R⁷ are hydrogen, A is A4 and Z¹ is 2,4-difluorophenyl.
**Table C-38:** This table provides compounds wherein R¹ is methyl, R² is fluorine, and R³, R⁴, R⁵, R⁶ and R⁷ are hydrogen, A is A9 and Z¹ is 2,4-difluorophenyl.
**Table C-39:** This table provides compounds wherein R¹ is methyl, R² is fluorine, and R³, R⁴, R⁵, R⁶ and R⁷ are hydrogen, A is A10 and Z¹ is 2,4-difluorophenyl.
**Table C-40:** This table provides compounds wherein R¹ and R⁴ are methyl, R² is fluorine, and R³, R⁵, R⁶ and R⁷ are hydrogen, A is A4 and Z¹ is 2,4-difluorophenyl.
**Table C-41:** This table provides compounds wherein R¹ and R⁴ are methyl, R² is fluorine, and R³, R⁵, R⁶ and R⁷ are hydrogen, A is A9 and Z¹ is 2,4-difluorophenyl.
**Table C-42:** This table provides compounds wherein R¹ and R⁴ are methyl, R² is fluorine, and R³, R⁵, R⁶ and R⁷ are hydrogen, A is A10 and Z¹ is 2,4-difluorophenyl.
**Table C-43:** This table provides compounds wherein R¹ and R⁷ are methyl, R² is fluorine, and R³, R⁴, R⁵, and R⁶ are hydrogen, A is A4 and Z¹ is 2,4-difluorophenyl.
**Table C-44:** This table provides compounds wherein R¹ and R⁷ are methyl, R² is fluorine, and R³, R⁴, R⁵, and R⁶ are hydrogen, A is A9 and Z¹ is 2,4-difluorophenyl.
**Table C-45:** This table provides compounds wherein R¹ and R⁷ are methyl, R² is fluorine, and R³, R⁴, R⁵, and R⁶ are hydrogen, A is A10 and Z¹ is 2,4-difluorophenyl.
**Table C-46:** This table provides compounds wherein R¹, R⁴ and R⁷ are methyl, R² is fluorine, and R³, R⁵, and R⁶ are hydrogen, A is A4 and Z¹ is 2,4-difluorophenyl.
**Table C-47:** This table provides compounds wherein R¹, R⁴ and R⁷ are methyl, R² is fluorine, and R³, R⁵, and R⁶ are hydrogen, A is A9 and Z¹ is 2,4-difluorophenyl.
**Table C-48:** This table provides compounds wherein R¹, R⁴ and R⁷ are methyl, R² is fluorine, and R³, R⁵, and R⁶ are hydrogen, A is A10 and Z¹ is 2,4-difluorophenyl.
**Table C-49:** This table provides compounds wherein R¹ and R² is methyl, R⁴ is ethyl, R³, R⁵, and R⁶ are hydrogen, A is A4 and Z¹ is 2,4-difluorophenyl.
**Table C-50:** This table provides compounds wherein R¹ and R² is methyl, R⁴ is ethyl, R³, R⁵, and R⁶ are hydrogen, A is A9 and Z¹ is 2,4-difluorophenyl.
**Table C-51:** This table provides compounds wherein R¹ and R² is methyl, R⁴ is ethyl, R³, R⁵, and R⁶ are hydrogen, A is A10 and Z¹ is 2,4-difluorophenyl.
**Table C-52:** This table provides compounds wherein R¹ is methyl, R⁴ is ethyl, R², R³, R⁵, and R⁶ are hydrogen, A is A4 and Z¹ is 2,4-difluorophenyl.
**Table C-53:** This table provides compounds wherein R¹ is methyl, R⁴ is ethyl, R², R³, R⁵, and R⁶ are hydrogen, A is A9 and Z¹ is 2,4-difluorophenyl.
**Table C-54:** This table provides compounds wherein R¹ is methyl, R⁴ is ethyl, R², R³, R⁵, and R⁶ are hydrogen, A is A10 and Z¹ is 2,4-difluorophenyl.
**Table C-55:** This table provides compounds wherein R¹ is methyl, R², R³, R⁴, R⁵, R⁶ and R⁷ are hydrogen, A is A4 and Z¹ is 4-fluorophenyl.
**Table C-56:** This table provides compounds wherein R¹ is methyl, R², R³, R⁴, R⁵, R⁶ and R⁷ are hydrogen, A is A9 and Z¹ is 4-fluorophenyl.
**Table C-57:** This table provides compounds wherein R¹ is methyl, R², R³, R⁴, R⁵, R⁶ and R⁷ are hydrogen, A is A10 and Z¹ is 4-fluorophenyl.
**Table C-58:** This table provides compounds wherein R¹ is methyl, R² is methyl, R³, R⁴, R⁵, R⁶ and R⁷ are hydrogen, A is A4 and Z¹ is 4-fluorophenyl.
**Table C-59:** This table provides compounds wherein R¹ is methyl, R² is methyl, R³, R⁴, R⁵, R⁶ and R⁷ are hydrogen, A is A9 and Z¹ is 4-fluorophenyl.
**Table C-60:** This table provides compounds wherein R¹ is methyl, R² is methyl, R³, R⁴, R⁵, R⁶ and R⁷ are hydrogen, A is A10 and Z¹ is 4-fluorophenyl.
**Table C-61:** This table provides compounds wherein R¹ is methyl, R² is methyl, R³ is methyl, R⁴, R⁵, R⁶ and R⁷ are hydrogen, A is A4 and Z¹ is 4-fluorophenyl.
**Table C-62:** This table provides compounds wherein R¹ is methyl, R² is methyl, R³ is methyl, R⁴, R⁵, R⁶ and R⁷ are hydrogen, A is A9 and Z¹ is 4-fluorophenyl.
**Table C-63:** This table provides compounds wherein R¹ is methyl, R² is methyl, R³ is methyl, R⁴, R⁵, R⁶ and R⁷ are hydrogen, A is A10 and Z¹ is 4-fluorophenyl.
**Table C-64:** This table provides compounds wherein R¹ is methyl, R⁴ is methyl, R², R³, R⁵, R⁶ and R⁷ are hydrogen, A is A4 and Z¹ is 4-fluorophenyl.
**Table C-65:** This table provides compounds wherein R¹ is methyl, R⁴ is methyl, R², R³, R⁵, R⁶ and R⁷ are hydrogen, A is A9 and Z¹ is 4-fluorophenyl.
**Table C-66:** This table provides compounds wherein R¹ is methyl, R⁴ is methyl, R², R³, R⁵, R⁶ and R⁷ are hydrogen, A is A10 and Z¹ is 4-fluorophenyl.
**Table C-67:** This table provides compounds wherein R¹ is methyl, R⁷ is methyl, R², R³, R⁴, R⁵ and R^{6 7} are hydrogen, A is A4 and Z¹ is 4-fluorophenyl.
**Table C-68:** This table provides compounds wherein R¹ is methyl, R⁷ is methyl, R², R³, R⁴, R⁵ and R^{6 7} are hydrogen, A is A9 and Z¹ is 4-fluorophenyl.
**Table C-69:** This table provides compounds wherein R¹ is methyl, R⁷ is methyl, R², R³, R⁴, R⁵ and R^{6 7} are hydrogen, A is A10 and Z¹ is 4-fluorophenyl.
**Table C-70:** This table provides compounds wherein R¹ is methyl, R⁴ is methyl, R⁷ is methyl, R², R³, R⁵ and R⁶ are hydrogen, A is A4 and Z¹ is 4-fluorophenyl.
**Table C-71:** This table provides compounds wherein R¹ is methyl, R⁴ is methyl, R⁷ is methyl, R², R³, R⁵ and R⁶ are hydrogen, A is A9 and Z¹ is 4-fluorophenyl.
**Table C-72:** This table provides compounds wherein R¹ is methyl, R⁴ is methyl, R⁷ is methyl, R², R³, R⁵ and R⁶ are hydrogen, A is A10 and Z¹ is 4-fluorophenyl.
**Table C-73:** This table provides compounds wherein R¹ is methyl, R² is chlorine, and R³, R⁴, R⁵, R⁶ and R⁷ are hydrogen, A is A4 and Z¹ is 4-fluorophenyl.
**Table C-74:** This table provides compounds wherein R¹ is methyl, R² is chlorine, and R³, R⁴, R⁵, R⁶ and R⁷ are hydrogen, A is A9 and Z¹ is 4-fluorophenyl.
**Table C-75:** This table provides compounds wherein R¹ is methyl, R² is chlorine, and R³, R⁴, R⁵, R⁶ and R⁷ are hydrogen, A is A10 and Z¹ is 4-fluorophenyl.
**Table C-76:** This table provides compounds wherein R¹ and R⁴ are methyl, R² is chlorine, and R³, R⁵, R⁶ and R⁷ are hydrogen, A is A4 and Z¹ is 4-fluorophenyl.
**Table C-77:** This table provides compounds wherein R¹ and R⁴ are methyl, R² is chlorine, and R³, R⁵, R⁶ and R⁷ are hydrogen, A is A9 and Z¹ is 4-fluorophenyl.
**Table C-78:** This table provides compounds wherein R¹ and R⁴ are methyl, R² is chlorine, and R³, R⁵, R⁶ and R⁷ are hydrogen, A is A10 and Z¹ is 4-fluorophenyl.
**Table C-79:** This table provides compounds wherein R¹ and R⁷ are methyl, R² is chlorine, and R³, R⁴, R⁵, and R⁶ are hydrogen, A is A4 and Z¹ is 4-fluorophenyl.
**Table C-80:** This table provides compounds wherein R¹ and R⁷ are methyl, R² is chlorine, and R³, R⁴, R⁵, and R⁶ are hydrogen, A is A9 and Z¹ is 4-fluorophenyl.
**Table C-81:** This table provides compounds wherein R¹ and R⁷ are methyl, R² is chlorine, and R³, R⁴, R⁵, and R⁶ are hydrogen, A is A10 and Z¹ is 4-fluorophenyl.
**Table C-82:** This table provides compounds wherein R¹, R⁴ and R⁷ are methyl, R² is chlorine, and R³, R⁵, and R⁶ are hydrogen, A is A4 and Z¹ is 4-fluorophenyl.
**Table C-83:** This table provides compounds wherein R¹, R⁴ and R⁷ are methyl, R² is chlorine, and R³, R⁵, and R⁶ are hydrogen, A is A9 and Z¹ is 4-fluorophenyl.
**Table C-84:** This table provides compounds wherein R¹, R⁴ and R⁷ are methyl, R² is chlorine, and R³, R⁵, and R⁶ are hydrogen, A is A10 and Z¹ is 4-fluorophenyl.
**Table C-85:** This table provides compounds wherein R¹ is methyl, R² is fluorine, and R³, R⁴, R⁵, R⁶ and R⁷ are hydrogen, A is A4 and Z¹ is 4-fluorophenyl.
**Table C-86:** This table provides compounds wherein R¹ is methyl, R² is fluorine, and R³, R⁴, R⁵, R⁶ and R⁷ are hydrogen, A is A9 and Z¹ is 4-fluorophenyl.
**Table C-87:** This table provides compounds wherein R¹ is methyl, R² is fluorine, and R³, R⁴, R⁵, R⁶ and R⁷ are hydrogen, A is A10 and Z¹ is 4-fluorophenyl.
**Table C-88:** This table provides compounds wherein R¹ and R⁴ are methyl, R² is fluorine, and R³, R⁵, R⁶ and R⁷ are hydrogen, A is A4 and Z¹ is 4-fluorophenyl.
**Table C-89:** This table provides compounds wherein R¹ and R⁴ are methyl, R² is fluorine, and R³, R⁵, R⁶ and R⁷ are hydrogen, A is A9 and Z¹ is 4-fluorophenyl.
**Table C-90:** This table provides compounds wherein R¹ and R⁴ are methyl, R² is fluorine, and R³, R⁵, R⁶ and R⁷ are hydrogen, A is A10 and Z¹ is 4-fluorophenyl.
**Table C-91:** This table provides compounds wherein R¹ and R⁷ are methyl, R² is fluorine, and R³, R⁴, R⁵, and R⁶ are hydrogen, A is A4 and Z¹ is 4-fluorophenyl.
**Table C-92:** This table provides compounds wherein R¹ and R⁷ are methyl, R² is fluorine, and R³, R⁴, R⁵, and R⁶ are hydrogen, A is A9 and Z¹ is 4-fluorophenyl.
**Table C-93:** This table provides compounds wherein R¹ and R⁷ are methyl, R² is fluorine, and R³, R⁴, R⁵, and R⁶ are hydrogen, A is A10 and Z¹ is 4-fluorophenyl.
**Table C-94:** This table provides compounds wherein R¹, R⁴ and R⁷ are methyl, R² is fluorine, and R³, R⁵, and R⁶ are hydrogen, A is A4 and Z¹ is 4-fluorophenyl.
**Table C-95:** This table provides compounds wherein R¹, R⁴ and R⁷ are methyl, R² is fluorine, and R³, R⁵, and R⁶ are hydrogen, A is A9 and Z¹ is 4-fluorophenyl.
**Table C-96:** This table provides compounds wherein R¹, R⁴ and R⁷ are methyl, R² is fluorine, and R³, R⁵, and R⁶ are hydrogen, A is A10 and Z¹ is 4-fluorophenyl.
**Table C-97:** This table provides compounds wherein R¹ and R² is methyl, R⁴ is ethyl, R³, R⁵, and R⁶ are hydrogen, A is A4 and Z¹ is 4-fluorophenyl.
**Table C-98:** This table provides compounds wherein R¹ and R² is methyl, R⁴ is ethyl, R³, R⁵, and R⁶ are hydrogen, A is A9 and Z¹ is 4-fluorophenyl.
**Table C-99:** This table provides compounds wherein R¹ and R² is methyl, R⁴ is ethyl, R³, R⁵, and R⁶ are hydrogen, A is A10 and Z¹ is 4-fluorophenyl.
**Table C-100:** This table provides compounds wherein R¹ is methyl, R⁴ is ethyl, R², R³, R⁵, and R⁶ are hydrogen, A is A4 and Z¹ is 4-fluorophenyl.
**Table C-101:** This table provides compounds wherein R¹ is methyl, R⁴ is ethyl, R², R³, R⁵, and R⁶ are hydrogen, A is A9 and Z¹ is 4-fluorophenyl.
**Table C-102:** This table provides compounds wherein R¹ is methyl, R⁴ is ethyl, R², R³, R⁵, and R⁶ are hydrogen, A is A10 and Z¹ is 4-fluorophenyl.

### EXAMPLES

The Examples which follow serve to illustrate the invention.

The compounds of the invention can be distinguished from known compounds by virtue of greater efficacy at low application rates, which can be verified by a person skilled in the art using the experimental procedures outlined in the Examples, using lower application rates if necessary, for example 60 ppm, 20 ppm or 2 ppm.

Compounds of formula (I) may possess any number of benefits including, *inter alia,* advantageous levels of biological activity for protecting plants against diseases that are caused by fungi or superior properties for use as agrochemical active ingredients (for example, greater biological activity, an advantageous spectrum of activity, an increased safety profile (including improved crop tolerance), improved physicochemical properties, or increased biodegradability).

Throughout this description, temperatures are given in degrees Celsius and "MP" means melting point. LC-MS means Liquid Chromatography Mass Spectroscopy and the description of the apparatus and the methods is as follows.

¹H NMR and ¹⁹F NMR measurements were recorded on a Bruker 400MHz spectrometer, chemical shifts are given in ppm relevant to a TMS (¹H) and CFCl3 (¹⁹F) standard. Spectra measured in deuterated solvents as indicated. Either one of the LCMS methods below was used to characterize the compounds. The characteristic LCMS values obtained for each compound were the retention time ("Rt", recorded in minutes) and the measured molecular ion (M+H)⁺ or (M-H)⁻.

The following HPLC-MS methods were used for the analysis of the compounds:

### Method A:

Spectra were recorded on a mass spectrometer from Waters (SQD, SQDII Single quadrupole mass spectrometer) equipped with an electrospray source (Polarity: positive and negative ions, Capillary: 3.00 kV, Cone range: 30 V, Extractor: 2.00 V, Source Temperature: 150°C, Desolvation Temperature: 350°C, Cone Gas Flow: 50 I/h, Desolvation Gas Flow: 650 I/h, Mass range: 100 to 900 Da) and an Acquity UPLC from Waters: binary pump, heated column compartment, diode-array detector and ELSD detector. Column: Waters UPLC HSS T3, 1.8 µm, 30 x 2.1 mm, Temperature: 60°C, DAD Wavelength range (nm): 210 to 500, Solvent Gradient: A = water + 5% MeOH + 0.05% HCOOH, B = Acetonitrile + 0.05% HCOOH, gradient: 10-100% B in 1.2 min; Flow (ml/min): 0.85.

### Method B:

Spectra were recorded on a ACQUITY mass spectrometer from Waters (SQD or SQDII Single quadrupole mass spectrometer) equipped with an electrospray source (Polarity: positive or negative ions, Capillary: 3.0 kV, Cone: 30V, Extractor: 3.00 V, Source Temperature: 150°C, Desolvation Temperature: 400°C, Cone Gas Flow: 60 L/hr, Desolvation Gas Flow: 700 L/hr, Mass range: 140 to 800 Da) and an ACQUITY UPLC from Waters with solvent degasser, binary pump, heated column compartment and diode-array detector. Column: Waters UPLC HSS T3, 1.8 µm, 30 x 2.1 mm, Temperature: 60°C, DAD Wavelength range (nm): 210 to 400, Solvent Gradient: A = Water/Methanol 9:1 + 0.1% formic acid, B = Acetonitrile + 0.1% formic acid, gradient: 0-100% B in 2.5 min; Flow (ml/min): 0.75.

### Method C:

Spectra were recorded on a ZQ mass spectrometer from Waters (single quadrupole mass spectrometer) equipped with an electrospray source (Polarity: positive or negative ions, Capillary: 3.00 kV, Cone: 30.00 V, Extractor: 2.00 V, Source Temperature: 100 °C, Desolvation Temperature: 250 °C, Cone Gas Flow: 50 L/Hr, Desolvation Gas Flow: 400 L/Hr, Mass range: 100 to 900 Da) and an Acquity UPLC from Waters (Solvent degasser, binary pump, heated column compartment and diode-array detector. Column: Waters UPLC HSS T3, 1.8 µm, 30 x 2.1 mm, Temperature: 60°C, flow rate 0.85 mL/min; DAD Wavelength range (nm): 210 to 500) Solvent Gradient: A = H2O + 5% MeOH + 0.05% HCOOH, B = Acetonitrile + 0.05% HCOOH) gradient: 0 min 10% B; 0-2.7 min 100% B; 2.7-3.0 min 100% B.

### Method D:

**Instrumentation:** Mass spectrometer: Acquity QDA Mass Spectrometer from Waters
HPLC: UPLC 'H' class

### Optimized Mass Parameter:

lonisation method: Electrospray (ESI)
Polarity: Positive and Negative Polarity Switch
Scan Type: Full Scan
Capillary (kV): 0.8
Cone Voltage (V): 25.00
Source Temperature (°C): 120
Desolvation Gas Flow (L/Hr): 1000
Desolvation Temperature (°C): 600
Gas Flow @ Cone (L/Hr): 50
Mass range: 110 to 850 Da
PDA Wavelength range: 230 to 400 nm

### Optimized Chromatographic parameter:

Gradient conditions:
**Solvent A:** Water with 0.1% formic acid: Acetonitrile: 95:5 v/v
**Solvent B:** Acetonitrile with 0.05% formic acid

| **Time (minutes)** | **A (%)** | **B (%)** | **Flow rate (ml/min)** |
|---|---|---|---|
| 0 | 90 | 10 | 0.6 |
| 0.2 | 90 | 10 | 0.6 |
| 0.3 | 50 | 50 | 0.6 |
| 0.6 | 0 | 100 | 0.6 |
| 1.3 | 0 | 100 | 0.6 |
| 1.4 | 90 | 10 | 0.6 |
| 1.6 | 90 | 10 | 0.6 |

Column: Acquity UPLC HSS T3 C18
Column length: 30 mm
Internal diameter of column: 2.1 mm
Particle size: 1.8 µ
Column oven temperature: 40°C

### Method E:

### Instrumentation:

HPLC System: UFLC Shimadzu Nexera X2 \Agilent HPLC 1200
Location: Kilo Analytical Lab

### Optimized Chromatographic parameter:

Gradient conditions:
**Solvent A:** Water with 0.05% formic acid
**Solvent B:** 100% Acetonitrile + 0.1% formic acid

| **Time (minutes)** | **A (%)** | **B (%)** | **Flow rate (ml/min)** |
|---|---|---|---|
| 0.0 | 90 | 10 | 1.8 |
| 0.9 | 0 | 100 | 1.8 |
| 1.8 | 0 | 100 | 1.8 |
| 2.2 | 90 | 10 | 1.8 |
| 2.5 | 90 | 10 | 1.8 |

Column: KINETEX-EVO C18
Column length: 50 mm
Internal diameter of column: 4.6 mm
Particle size: 2.6 µ
Column oven temperature: 40°C

### Method F:

### Instrumentation:

Mass spectrometer: Acquity SQD Mass Spectrometer from Waters
HPLC: UPLC 'H' class

### Optimized Mass Parameter:

lonisation method: Electrospray (ESI)
Polarity: Positive and Negative Polarity Switch
Scan Type: Full Scan
Capillary (kV): 3.00
Cone Voltage (V): 41.00
Source Temperature (°C): 150
Desolvation Gas Flow (L/Hr): 1000
Desolvation Temperature (°C): 500
Gas Flow @ Cone (L/Hr): 50
Mass range: 110 to 800 Da
PDA Wavelength range: 210 to 400 nm

### Optimized Chromatographic parameter:

Gradient conditions:
**Solvent A:** Water with 0.1% formic acid : Acetonitrile: 95:5 v/v
**Solvent B:** Acetonitrile with 0.05% formic acid

| **Time (minutes)** | **A (%)** | **B (%)** | **Flow rate (ml/min)** |
|---|---|---|---|
| 0.0 | 90 | 10 | 0.6 |
| 0.2 | 90 | 10 | 0.6 |
| 0.3 | 50 | 50 | 0.6 |
| 0.6 | 0 | 100 | 0.6 |
| 1.3 | 0 | 100 | 0.6 |
| 1.4 | 90 | 10 | 0.6 |
| 1.6 | 90 | 10 | 0.6 |

Column: Acquity UPLC HSS T3 C18
Column length: 30 mm
Internal diameter of column: 2.1 mm
Particle size: 1.8 µ
Column oven temperature: 40°C

### Formulation Examples

| Wettable powders | a) | b) | c) |
|---|---|---|---|
| active ingredients | 25 % | 50% | 75 % |
| sodium lignosulfonate | 5% | 5% | - |
| sodium lauryl sulfate | 3% | | 5 % |
| sodium diisobutylnaphthalenesulfonate | | 6% | 10 % |
| phenol polyethylene glycol ether (7-8 mol of ethylene oxide) | | 2% | - |
| highly dispersed silicic acid | 5% | 10% | 10 % |
| Kaolin | 62% | 27 % | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | - | 20 % |

The combination is thoroughly mixed with the adjuvants and the mixture is thoroughly ground in a suitable mill, affording powders that can be used directly for seed treatment.

### Emulsifiable concentrate

| | |
|---|---|
| active ingredients | 10% |
| octylphenol polyethylene glycol ether (4-5 mol of ethylene oxide) | 3% |
| calcium dodecylbenzene sulfonate | 3% |
| castor oil polyglycol ether (35 mol of ethylene oxide) | 4% |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

### Extruder granules

| | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

### Coated granules

| | |
|---|---|
| Active ingredients | 8% |
| polyethylene glycol (mol. wt. 200) | 3% |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Abbreviations

- CDCl₃: deuterated chloroform
- DCC: dicyclohexyl carbodiimide
- DMF: dimethylformamide
- DMSO: dimethyl sulfoxide
- DMSO-d6: deuterated Dimethyl sulfoxide
- EDC: 1-ethyl-3-(3-dimethylaminopropyl)carbodiimide
- EtOAc: ethylacetate
- HATU: 1-[bis(dimethylamino)methylene]-1H-1,2,3-triazolo[4,5-b]pyridinium 3-oxide hexafluorophosphate
- HCl: hydrochloric acid
- h/hrs: hour/hours
- LC-MS: Liquid Chromatography Mass Spectrometry (LC-MS or LCMS)
- nOe: nuclear Overhauser effect
- rh: relative humidity
- rt: room temperature
- Rt: retention time
- ssp.: subspecies
- THF: tetrahydrofuran

### PREPARATION EXAMPLES

The compounds of formula (I) according to the invention may be prepared using the synthetic techniques described both above and below.

Syn- and anti-isomers could be distinguished using high field NMR techniques such as ROESY 2D NMR. For example: 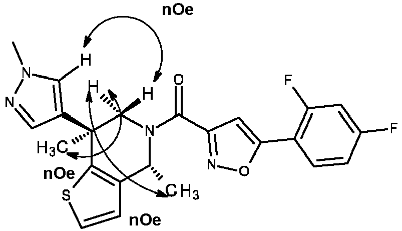 (anti-isomer, compound P-9 of Table T1) 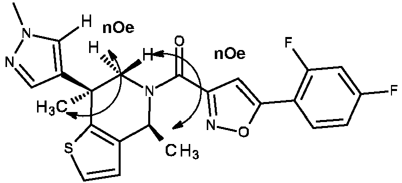 (syn-isomer, compound P-8 of Table T1)

### Example 1: Preparation of [5-(2,4-difluorophenyl)isoxazol-3-yl]-[rac-(4R,7R)-7-methyl-4-(1-methylpyrazol-4-yl)-5,7-dihydro-4H-thieno[2,3-c]pyridin-6-yllmethanone (compound P-17, Table T1) and [5-(2,4-difluorophenyl)isoxazol-3-yl]-[rac-(4R,7S)-7-methyl-4-(1-methylpyrazol-4-yl)-5,7-dihydro-4H-thieno[2,3-clpyridin-6-yllmethanone (compound P-14, Table T1).

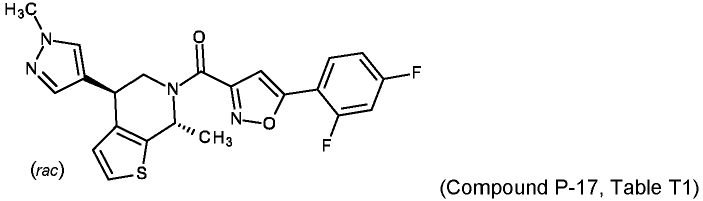

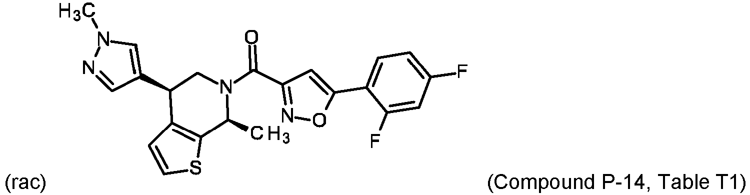

### Step A: Preparation of (1-methylpyrazol-4-yl)-(3-thienyl)methanol

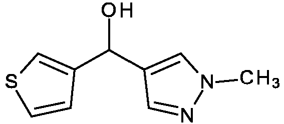

A one necked round bottom flask, equipped with a magnetic stirred bar, was charged with 4-iodo-1-methyl-1h-pyrazole (7.37 g, 33.6 mmol) and tetrahydrofuran (92 mL). Isopropyl magnesium chloride lithium chloride complex (1.3 mol/L) in THF (35 mL) was added dropwise at 0°C under argon atmosphere (white suspension). The mixture was stirred at 0°C for 45 min. Then, 3-thiophenecarboxaldehyde (3.50 g, 30.6 mmol) was added dropwise to the previous mixture at 0°C under argon atmosphere. The mixture was stirred at this temperature for 10 min and then allowed to warm to room temperature and stirred for 1 hour. The reaction mixture was then poured into water (100 mL) and extracted with ethyl acetate (2X80 mL). The combined organic layers were washed with brine, dried over sodium sulfate and concentrated *in vacuo* to yield the title compound.

LC-MS (Method A): retention time 0.56 min, 195 (M+H).

### Step B: Preparation of 2-(1-methylpyrazol-4-yl)-2-(3-thienyl)acetonitrile

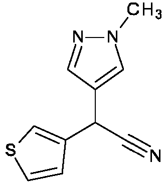

A sealed tube, equipped with a magnetic stirrer bar, was charged with (1-methylpyrazol-4-yl)-(3-thienyl)methanol (5.94 g, 30.6 mmol) and dichloromethane (245 mL). To this solution was added successively at room temperature lithium carbonate (0.456 g, 6.12 mmol), trimethylsilyl cyanide (18.7 mL, 138 mmol) and iodine (14.2 g, 55.0 mmol). After stirring for one hour at 35°C, the reaction mixture was cooled to room temperature and poured into a saturated solution of Na₂S₂O₃ (80 mL) and extracted with dichloromethane (2X60 mL). The combined organic layers were washed with brine, dried over sodium sulfate and concentrated *in vacuo.* The crude material was purified by chromatography over silica gel (20 g SiO₂, ethyl acetate/cyclohexane gradient) to yield the title compound.

LC-MS (Method A): retention time 0.75 min, 204 (M+H).

¹H NMR (400 MHz, CDCl3) δ ppm 3.92 (s, 3 H) 5.13 - 5.22 (m, 1 H) 7.00 - 7.08 (m, 1 H) 7.29 - 7.30 (m, 1 H) 7.36 - 7.40 (m, 2 H) 7.42 - 7.47 (m, 1 H).

### Step C: Preparation of 2-(1-methylpyrazol-4-yl)-2-(3-thienyl)ethanamine

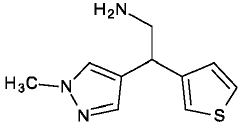

A one necked round bottom flask, equipped with a magnetic stirrer bar, was charged with 2-(1-methylpyrazol-4-yl)-2-(3-thienyl)acetonitrile (1.78 g, 8.76 mmol) in tetrahydrofuran (26 mL). To this solution was added dropwise at room temperature borane dimethyl sulfide complex (2.65 mL, 26.3 mmol) under argon atmosphere, and the mixture then stirred at 65°C for 1 hour. The reaction mixture was cooled to 0°C before adding dropwise hydrochloric acid (6 mol/L, 5.87 mL, 35.2 mmol) followed by heating the mixture to 65°C for 1 hour. The mixture was diluted with water (20 mL), basified (to pH 12) with NaOH 6M, cooled and extracted with ethyl acetate (3X20 mL). The combined organic layers were washed with brine, dried over sodium sulfate and concentrated *in vacuo* to afford 2-(1-methylpyrazol-4-yl)-2-(3-thienyl)ethanamine.

LC-MS (Method A): retention time 0.25 min, 208 (M+H).,

¹H NMR (400 MHz, CDCl₃) δ ppm 7.30 - 7.44 (m, 2 H) 7.06 - 7.27 (m, 3 H) 7.00 (br d, *J*=4.7 Hz, 1 H) 4.06 - 4.30 (m, 1 H) 3.87 (s, 3 H) 3.57 - 3.78 (m, 1 H) 3.21 (br s, 2 H).

### Step D: Preparation of N-[2-(1-methylpyrazol-4-yl)-2-(3-thienyl)ethyl]acetamide

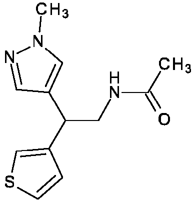

A one necked round bottom flask, equipped with a magnetic stirrer bar, was charged with 2-(1-methylpyrazol-4-yl)-2-(3-thienyl)ethanamine (1.80 g, 8.68 mmol) and sodium bicarbonate aqueous saturated solution (43 mL). Acetyl chloride (0.71 mL, 9.55 mmol) in ethyl acetate (43 mL) was added dropwise at room temperature under vigorous stirring. The mixture was stirred at room temperature for 15 minutes. The reaction mixture was then extracted with ethyl acetate (2X20 mL) and the combined organic layers were washed with brine, dried over sodium sulfate and concentrated *in vacuo* to yield N-[2-(1-methylpyrazol-4-yl)-2-(3-thienyl)ethyl]acetamide.

LC-MS (Method A): retention time 0.25 min, 234 (M+H).

¹H NMR (400 MHz, CDCl3) δ ppm 7.37 - 7.41 (m, 1 H) 7.33 (dd, *J*=5.1, 2.9 Hz, 1 H) 7.21 (s, 1 H) 7.06 (dd, *J*=1.5, 0.7 Hz, 1 H) 6.99 (dd, *J*=4.9, 1.3 Hz, 1 H) 5.50 (br s, 1 H) 4.21 (t, *J*=7.4 Hz, 1 H) 3.89 (s, 3 H) 3.73 (td, *J*=7.9, 6.0 Hz, 2 H) 1.94 (s, 3 H).

### Step E: Preparation of 7-methyl-4-(1-methylpyrazol-4-yl)-4,5,6,7-tetrahydrothieno[2,3-c]pyridine and 4-methyl-7-(1-methylpyrazol-4-yl)-4,5,6,7-tetrahydrothieno[3,4-c]pyridine

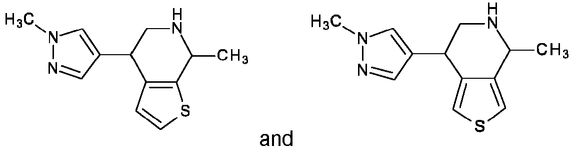

### Step 1

A one necked round bottom flask, equipped with a magnetic stirrer bar, was charged with N-[2-(1-methylpyrazol-4-yl)-2-(3-thienyl)ethyl]acetamide (1.73 g, 6.94 mmol) and phosphoryl chloride (7 mL). The mixture was stirred at 60°C for 1 hour. The reaction mixture was cooled to room temperature and slowly poured into a saturated solution of Na₂CO₃ (20 mL) at 0°C (gas evolution and exothermic!). The mixture was extracted with ethyl acetate (3X20 mL) and the combined organic layers were washed with brine, dried over sodium sulfate and concentrated *in vacuo* to the title products as a mixture which were used in the next steps without further purification.

### Step 2

A one necked round bottom flask, equipped with a magnetic stirrer bar, was charged with the product from step 1 *vide supra* (913 mg, 3.947 mmol) and methanol (12 mL). To this solution was added sodium borohydride (0.233 g, 5.92 mmol) at room temperature (gas evolution) and the mixture then stirred at room temperature for 1 hour. The reaction mixture was poured into water (20 mL) and the mixture was extracted with ethyl acetate (3X20 mL). The combined organic layers were washed with brine, dried over sodium sulfate and concentrated *in vacuo* to yield 7-methyl-4-(1-methylpyrazol-4-yl)-4,5,6,7-tetrahydrothieno[2,3-c]pyridine as a mixture with 4-methyl-7-(1-methylpyrazol-4-yl)-4,5,6,7-tetrahydrothieno[3,4-c]pyridine.

LC-MS (Method A): retention time 0.25 min, 234 (M+H).

### Step F: Preparation of [5-(2,4-difluorophenyl)isoxazol-3-yl]-[rac-(4R,7R)-7-methyl-4-(1-methylpyrazol-4-yl)-5,7-dihydro-4H-thieno[2,3-c]pyridin-6-yl]methanone (compound P-17, Table T1) and [5-(2,4-difluorophenyl)isoxazol-3-yl]-frac-(4R,7S)-7-methyl-4-(1-methylpyrazol-4-yl)-5,7-dihydro-4H-thieno[2,3-c]pyridin-6-yl]methanone (compound P-14, Table T1).

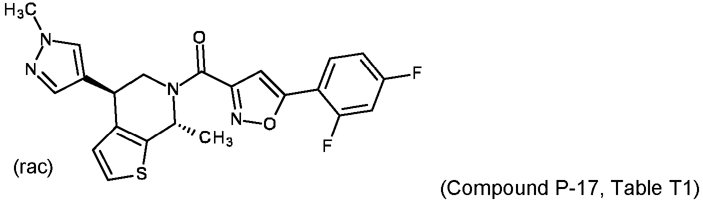

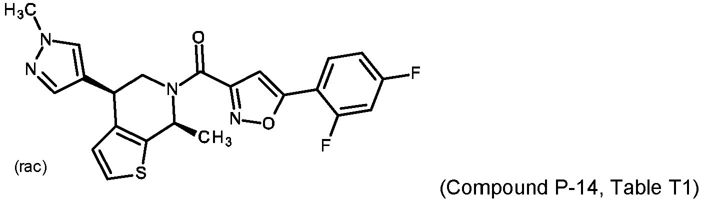

A one necked round bottom flask, equipped with a magnetic stirrer bar, was charged with 7-methyl-4-(1-methylpyrazol-4-yl)-4,5,6,7-tetrahydrothieno[2,3-c]pyridine as a mixture with 4-methyl-7-(1-methylpyrazol-4-yl)-4,5,6,7-tetrahydrothieno[3,4-c]pyridine (117 mg, 0.265 mmol, mixture from step E, step 2, example 1), ethyl acetate (17 mL), N,N-diisopropylethylamine (0.88 mL, 5.14 mmol) and 5-(2,4-difluorophenyl)isoxazole3-carboxylic acid (0.4378 g, 1.88 mmol). Then, propylphosphonic anhydride (1.82 mL, 3.08 mmol) was added dropwise at room temperature and the mixture was stirred at room temperature. After one hour, the reaction mixture was poured into water (20 mL). The mixture was extracted with ethyl acetate (2X20 mL) and the combined organic layers were washed with brine, dried over sodium sulfate and concentrated *in vacuo.* The crude material was purified by preparative HPLC (Preparative HPLC method: Autopurification System from Waters: 2767 sample Manager, 2489 UV/Visible Detector, 2545 Quaternary Gradient Module. MS - RP2: SQDII Single quadrupole mass spectrometer equipped with an electrospray source. Column: BC: Phenomenex Gemini NX C18, 5 micron particle size, 110 Angström, 100 x 50 mm. Solvent: A: water; Solvent: B: acetonitrile; modifier: formic acid, Gradient: 40% B - 60% B, Runtime: 10 min) to yield as first eluting product [5-(2,4-difluorophenyl)isoxazol-3-yl]-[rac-(4R,7R)-7-methyl-4-(1-methylpyrazol-4-yl)-5,7-dihydro-4H-thieno[2,3-c]pyridin-6-yl]methanone.

LC-MS (Method A): retention time 1.05 min, 441 (M+H).

Isolated as second eluting product was [5-(2,4-difluorophenyl)isoxazol-3-yl]-[rac-(4R,7S)-7-methyl-4-(1-methylpyrazol-4-yl)-5,7-dihydro-4H-thieno[2,3-c]pyridin-6-yl]methanone.

LC-MS (Method A): retention time 1.08 min, 441 (M+H).

¹H NMR (400 MHz, CDCl3) δ ppm 7.88 - 8.09 (m, 1 H) 7.30 - 7.49 (m, 2 H) 7.04 - 7.19 (m, 2 H) 6.95 - 7.04 (m, 2 H) 6.67 (d, *J*=5.1 Hz, 1 H) 5.68 - 6.06 (m, 1 H) 4.53 - 4.98 (m, 1 H) 4.14 - 4.31 (m, 1 H) 3.84 - 4.02 (m, 3 H) 3.00 - 3.47 (m, 1 H) 1.67 - 1.83 (m, 3 H).

### Example 2: Preparation of [5-(2,4-difluorophenyl)-1,3,4-thiadiazol-2-yl]-[rac-(4R,7R)-7-methyl-4-(1-methylpyrazol-4-yl)-5,7-dihydro-4H-thieno[2,3-c]pyridin-6-yl]methanone (compound P-16, Table T1) and [5-(2,4-difluorophenyl)-1,3,4-thiadiazol-2-yl]-[rac-(4R,7S)-7-methyl-4-(1-methylpyrazol-4-yl)-5,7-dihydro-4H-thieno[2,3-c]pyridin-6-yllmethanone (compound P-15, Table T1)

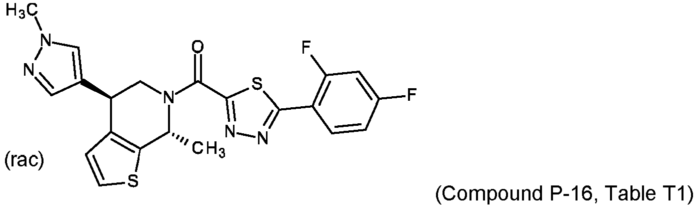

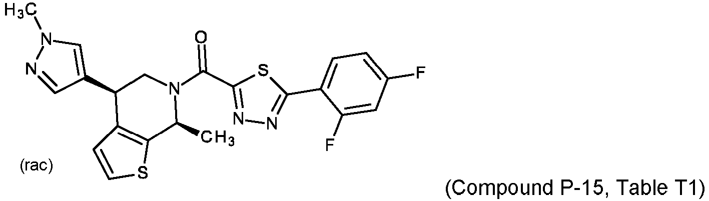

A one necked round bottom flask, equipped with a magnetic stirrer bar, was charged with a mixture of 7-methyl-4-(1-methylpyrazol-4-yl)-4,5,6,7-tetrahydrothieno[2,3-c]pyridine and 4-methyl-7-(1-methylpyrazol-4-yl)-4,5,6,7-tetrahydrothieno[3,4-c]pyridine (117 mg, 0.265 mmol, mixture from step E, step 2, example 1) (468 mg, 2.006 mmol) and toluene (8 mL). Trimethylaluminium (2.01 mL, 4.01 mmol) was added stepwise at room temperature under an argon atmosphere. The reaction was stirred at room temperature for 20 min before adding ethyl 5-(2,4-difluorophenyl)-1,3,4-thiadiazole-2-carboxylate (0.5964 g, 2.207 mmol), diluted in toluene (8 mL) dropwise at room temperature and the mixture was stirred at 90°C for 24 hours. The reaction mixture was cooled to 0°C and quenched with careful addition of HCl 1M (1 mL). The mixture was diluted with a saturated solution of NaHCO₃ (20 mL) and extracted with ethyl acetate (3X20 mL). The combined organic layers were washed with brine, dried over sodium sulfate and concentrated under vacuum. The crude material was purified by preparative HPLC (Preparative HPLC method: Autopurification System from Waters: 2767 sample Manager, 2489 UV/Visible Detector, 2545, Quaternary Gradient Module, MS - RP2: SQDII Single quadrupole mass spectrometer equipped with an electrospray source, Column: MC:Phenomenex Gemini NX C18, 4 micron particle size, 80 Angström, 75 x 30 mm. Solvent: A: water; Solvent: B: acetonitrile; modifier: formic acid. Gradient: 40% B - 50% B, Runtime: 10 min) to yield as first eluting product [5-(2,4-difluorophenyl)-1,3,4-thiadiazol-2-yl]-[rac-(4R,7R)-7-methyl-4-(1-methylpyrazol-4-yl)-5,7-dihydro-4H-thieno[2,3-c]pyridin-6-yl]methanone (compound P-16, Table T1).

LC-MS (Method A): retention time 1.13 min, 458 (M+H).

The second eluting product was [5-(2,4-difluorophenyl)-1,3,4-thiadiazol-2-yl]-[rac-(4R,7S)-7-methyl-4-(1-methylpyrazol-4-yl)-5,7-dihydro-4H-thieno[2,3-c]pyridin-6-yl]methanone (compound P-15, Table T1).

LC-MS (Method A): retention time 1.18 min, 458 (M+H).

¹H NMR (400 MHz, CDCl3) δ ppm 8.39 - 8.58 (m, 1 H) 7.31 - 7.58 (m, 2 H) 6.95 - 7.21 (m, 3 H) 6.65 - 6.73 (m, 1 H) 5.84 - 6.66 (m, 1 H) 4.79 - 5.56 (m, 1 H) 4.18 - 4.46 (m, 1 H) 3.87 - 4.03 (m, 3 H) 3.12 - 3.49 (m, 1 H) 1.64 - 1.89 (m, 3 H).

### Example 3: Preparation of [5-(2,4-difluorophenyl)-1,3,4-thiadiazol-2-yl]-[(4S,7R)-7-methyl-4-(1-methylpyrazol-4-yl)-5,7-dihydro-4H-thieno[2,3-c]pyridin-6-yllmethanone (compound P-1, Table T1) and 5-(2,4-difluorophenyl)-1,3,4-thiadiazol-2-yll-[(4R,7S)-7-methyl-4-(1-methylpyrazol-4-yl)-5,7-dihydro-4H-thieno[2,3-c]pyridin-6-yl]methanone (compound P-2, Table T1)

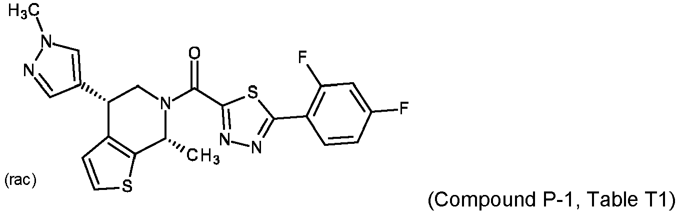

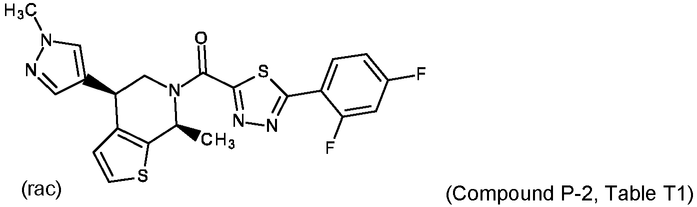

The racemic mixture [5-(2,4-difluorophenyl)-1,3,4-thiadiazol-2-yl]-[rac-(4R,7S)-7-methyl-4-(1-methylpyrazol-4-yl)-5,7-dihydro-4H-thieno[2,3-c]pyridin-6-yl]methanone was separated into its enantiomers by preparative SFC HPLC.

### Preparative SFC method:

Sepiatec Preparative SFC 100, Column: Daicel CHIRALPAK^{®} IB, 5µm, 2.0 cm x 25cm. Mobile phase: A: CO2 - B: MeOH isocratic: 10% B. Backpressure: 150 bar, Flow rate: 60 ml/min, GLS pump: Detection: UV 285 nm, Sample concentration: 97 mg in 3 ml MeOH, Injection: 500 microlitre.

The following eluting peaks were obtained:
[5-(2,4-difluorophenyl)-1,3,4-thiadiazol-2-yl]-[(4S,7R)-7-methyl-4-(1-methylpyrazol-4-yl)-5,7-dihydro-4H-thieno[2,3-c]pyridin-6-yl]methanone
Retention time (min) ~ 2.88
Chemical purity (area% at 285 nm) > 99
Enantiomeric excess (%) > 98.8%
[5-(2,4-difluorophenyl)-1,3,4-thiadiazol-2-yl]-[(4R,7S)-7-methyl-4-(1-methylpyrazol-4-yl)-5,7-dihydro-4H-thieno[2,3-c]pyridin-6-yl]methanone
Retention time (min) ~ 3.30
Chemical purity (area% at 285 nm) > 99
Enantiomeric excess (%) > 95.3%

### Example 4: Preparation of [5-(2,4-difluorophenyl)isoxazol-3-yl]-[rac-(4S,7S)-4,7-dimethyl-4-(1-methylpyrazol-4-yl)-5,7-dihydrothieno[2,3-c]pyridin-6-yl]methanone (compound P-13, Table T1) and [5-(2,4-difluorophenyl)isoxazol-3-yl]-[rac-(4S,7R)-4,7-dimethyl-4-(1-methylpyrazol-4-yl)-5,7-dihydrothieno[2,3-c]pyridin-6-yl]methanone (compound P-12 Table T1)

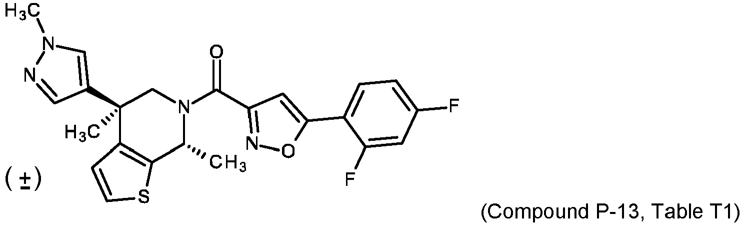

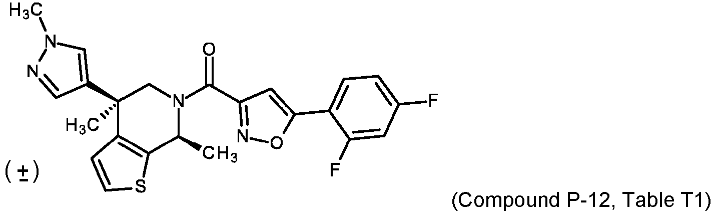

### Step A: Preparation of 2-(1-methylpyrazol-4-yl)-2-(3-thienyl)propanenitrile

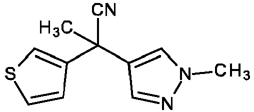

A sealed tube, equipped with a magnetic stirrer bar and a temperature probe, was charged with 2-(1-methylpyrazol-4-yl)-2-(3-thienyl)acetonitrile (2.32 g, 11.4 mmol) and tetrahydrofuran (46 mL). To this solution n-butyllithium (2.5 mol/L, 5.5 mL, 13.7 mmol) was added dropwise at -70°C under argon atmosphere. The mixture was stirred at this temperature for 30 minutes before adding iodomethane (1.08 mL, 17.1 mmol) dropwise at -78°C. The reaction was stirred at -78°C for 5 minutes and then at room temperature for 30 minutes. The reaction mixture was poured into water (60 mL) and extracted with ethyl acetate (2X50 mL). The combined organic layers were washed with brine, dried over sodium sulfate and concentrated *in vacuo* to yield 2-(1-methylpyrazol-4-yl)-2-(3-thienyl)propanenitrile.

LC-MS (Method A): retention time 0.81 min, 218 (M+H).

¹H NMR (400 MHz, CDCl3) δ ppm 2.04 (s, 3 H) 3.91 (s, 3 H) 7.02 - 7.08 (m, 1 H) 7.24 - 7.30 (m, 1 H) 7.31 - 7.34 (m, 1 H) 7.34 - 7.39 (m, 1 H) 7.42 - 7.46 (m, 1 H).

### Step B: Preparation of 2-(1-methylpyrazol-4-yl)-2-(3-thienyl)propan-1-amine

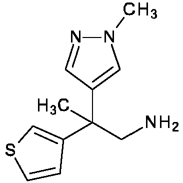

A one necked round bottom flask, equipped with a magnetic stirrer bar, was charged with 2-(1-methylpyrazol-4-yl)-2-(3-thienyl)propanenitrile (2.48 g, 11.4 mmol) and tetrahydrofuran (34 mL). Borane dimethyl sulfide complex (3.45 mL, 34.2 mmol) was added dropwise at room temperature under argon atmosphere and the mixture was stirred at 65°C for 1 hour. The reaction mixture was cooled to 0°C before adding hydrochloric acid (6 mol/L, 7.65 mL, 45.9 mmol) dropwise (strong gas evolution) and the mixture was stirred at 65°C for 1 hour. The mixture was diluted with water (30mL), basified with NaOH 6M (pH 12) and extracted with ethyl acetate (3X20 mL). The combined organic layers were washed with brine, dried over sodium sulfate and concentrated *in vacuo* to yield 2-(1-methylpyrazol-4-yl)-2-(3-thienyl)propan-1-amine.

LC-MS (Method A): retention time 0.28 min, 222 (M+H).

¹H NMR (400 MHz, CDCl3) δ ppm 7.36 (d, *J*=0.7 Hz, 1 H) 7.29 (s, 1 H) 7.17 (s, 1 H) 7.04 (dd, *J*=2.9, 1.5 Hz, 1 H) 6.99 (dd, *J*=5.1, 1.5 Hz, 1 H) 3.88 (s, 3 H) 3.16 (s, 2 H) 1.65 (s, 3 H).

### Step C: Preparation of N-[2-(1-methylpyrazol-4-yl)-2-(3-thienyl)propyl]acetamide

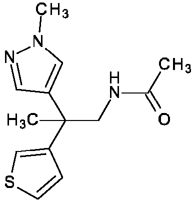

A one necked round bottom flask, equipped with a magnetic stirrer bar, was charged with 2-(1-methylpyrazol-4-yl)-2-(3-thienyl)propan-1-amine (1.54 g, 6.96 mmol) and sodium bicarbonate aqueous saturated solution (35 mL). Then, acetyl chloride (0.573 mL, 7.65 mmol) dissolved in ethyl acetate (35 mL) was added dropwise at room temperature under vigorous stirring. The mixture was stirred at room temperature for 15 minutes. The reaction mixture was extracted with ethyl acetate (2X20 mL) and the combined organic layers were washed with brine, dried over sodium sulfate and concentrated *in vacuo* to get N-[2-(1-methylpyrazol-4-yl)-2-(3-thienyl)propyl]acetamide.

LC-MS (Method A): retention time 0.68 min, 264 (M+H). ¹H NMR (400 MHz, CDCl3) δ ppm 7.29 - 7.36 (m, 2 H) 7.18 (s, 1 H) 7.05 (dd, *J*=2.9, 1.5 Hz, 1 H) 6.99 (dd, *J*=4.9, 1.3 Hz, 1 H) 5.22 - 5.38 (m, 1 H) 3.89 (s, 3 H) 3.77 (dd, *J*=5.8, 5.1 Hz, 2 H) 1.93 (s, 3 H) 1.61 (s, 3 H).

### Step D: Preparation of 4,7-dimethyl-4-(1-methylpyrazol-4-yl)-6,7-dihydro-5H-thieno[2,3-c]pyridine

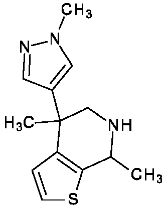

A one necked round bottom flask, equipped with a magnetic stirrer bar, was charged with N-[2-(1-methylpyrazol-4-yl)-2-(3-thienyl)propyl]acetamide (1.46 g, 5.54 mmol) and phosphoryl chloride (5.5 mL). The reaction mixture was stirred at 60°C for 1 hour, cooled down to room temperature and slowly poured into water (30 mL) at 45°C. The mixture was basified with Na₂CO₃ (pH 9) and extracted with ethyl acetate (3X20 mL). The combined organic layers were washed with brine, dried over sodium sulfate and concentrated *in vacuo* to yield 4,7-dimethyl-4-(1-methylpyrazol-4-yl)-5H-thieno[2,3-c]pyridine. This was diluted with methanol (9 mL) and treated with sodium borohydride (0.1728 g, 4.384 mmol) portionwise at room temperature. The reaction mixture was stirred 30 minutes and poured into water (20mL). The mixture was extracted with ethyl acetate (3X20 mL) and the combined organic layers were washed with brine, dried over sodium sulfate and concentrated *in vacuo* to yield 4,7-dimethyl-4-(1-methylpyrazol-4-yl)-6,7-dihydro-5H-thieno[2,3-c]pyridine.

LC-MS (Method A): retention time 0.41 min, 248 (M+H).

### Step E: Preparation of [5-(2,4-difluorophenyl)isoxazol-3-yl]-[rac-(4S,7S)-4,7-dimethyl-4-(1-methylpyrazol-4-yl)-5,7-dihydrothieno[2,3-c]pyridin-6-yllmethanone (compound P-13, Table T1) and [5-(2,4-difluorophenyl)isoxazol-3-yl]-[rac-(4S,7R)-4,7-dimethyl-4-(1-methylpyrazol-4-yl)-5,7-dihydrothieno[2,3-clpyridin-6-yl]methanone (compound P-12, Table T1).

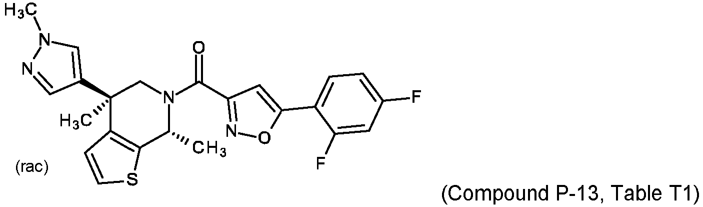

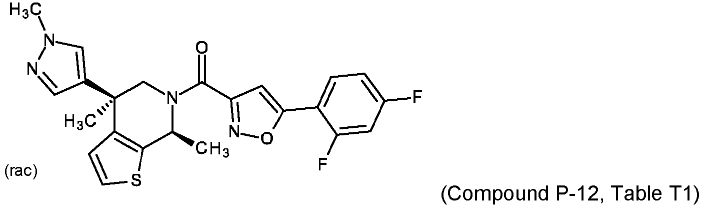

A one necked round bottom flask, equipped with a magnetic stirrer bar, was charged with 4,7-dimethyl-4-(1-methylpyrazol-4-yl)-6,7-dihydro-5H-thieno[2,3-c]pyridine (0.210g, 0.848 mmol), ethyl acetate (9 mL), N,N-diisopropylethylamine (0.437 mL, 2.54 mmol) and 5-(2,4-difluorophenyl)isoxazole-3-carboxylic acid (0.216 g, 0.933 mmol). Then, propylphosphonic anhydride (0.900 mL, 1.528 mmol) was added dropwise at room temperature. The reaction mixture was stirred at room temperature for 1 hour and poured into water (20 mL). The aqueous layer was extracted with ethyl acetate (2X20 mL) and the combined organic layers were washed with brine, dried over sodium sulfate and concentrated under vacuum. The crude material was purified by preparative HPLC (Preparative HPLC method: Autopurification System from Waters: 2767 sample Manager, 2489 UV/Visible Detector, 2545 Quaternary Gradient Module. MS - RP2: SQDII Single quadrupole mass spectrometer) equipped with an electrospray source. Column: MC:Phenomenex Gemini NX C18, 4 micron particle size, 80 Angström, 75 x 30 mm. Solvent: A: water; Solvent: B: acetonitrile; modifier: formic acid. Gradient: 40% B - 50% B. Runtime: 10 min) to afford as first eluting product [5-(2,4-difluorophenyl)isoxazol-3-yl]-[rac-(4S,7S)-4,7-dimethyl-4-(1-methylpyrazol-4-yl)-5,7-dihydrothieno[2,3-c]pyridin-6-yl]methanone.

LC-MS (Method A): retention time 1.14 min, 455 (M+H). ¹H NMR (600 MHz, CDCl₃*d*) δ ppm 1.61 (s, 3 H) 1.67 (d, *J*=6.7 Hz, 3 H) 1.69 - 1.70 (m, 1 H) 1.70 (s, 1 H) 3.20 (d, *J*=12.9 Hz, 1 H) 3.58 (d, *J*=13.6 Hz, 1 H) 3.72 (s, 3 H) 3.82 (s, 1 H) 3.90 (d, *J*=15.6 Hz, 1 H) 4.41 (d, *J*=13.6 Hz, 1 H) 4.92 (d, *J*=12.9 Hz, 1 H) 5.84 (q, *J*=6.6 Hz, 1 H) 6.09 (q, *J*=6.7Hz, 1 H) 6.24 (d, *J*=3.7 Hz, 1 H) 6.63 (s, 1 H) 6.83 (d, *J*=5.1 Hz, 1 H) 6.86 (d, *J*=3.7 Hz, 1 H) 6.99 (s, 1 H) 7.03 - 7.10 (m, 2 H) 7.24 (d, *J*=5.1 Hz, 1 H) 7.88 -8.05 (m, 1 H).

The second eluting product was [5-(2,4-difluorophenyl)isoxazol-3-yl]-[rac-(4S,7R)-4,7-dimethyl-4-(1-methylpyrazol-4-yl)-5,7-dihydrothieno[2,3-c]pyridin-6-yl]methanone (compound P-12, Table T1).

LC-MS (Method A): retention time 1.16 min, 455 (M+H). ¹H NMR (600 MHz, CDCl₃) δ ppm 1.56 (s, 5 H) 1.59 (s, 3 H) 1.68 (d, J=6.6 Hz, 5 H) 1.76 (d, J=6.6 Hz, 4 H) 3.26 (d, J=13.2 Hz, 1 H) 3.58 (d, J=13.8 Hz, 2 H) 3.90 (s, 4 H) 3.94 (s, 3 H) 4.53 (d, J=13.8 Hz, 1 H) 4.74 (d, J=13.1 Hz, 1 H) 5.82 (q, J=6.5 Hz, 1 H) 5.99 (q, J=6.6 Hz, 1 H) 6.62 (d, J=5.1 Hz, 1 H) 6.66 (d, J=5.1 Hz, 1 H) 7.14 (d, J=5.1 Hz, 2 H) 7.25 (s, 1 H) 7.30 (s, 1 H) 7.36 (s, 1 H) 7.40 (s, 1 H).

### Example 5: Preparation of [5-(2,4-difluorophenyl)isoxazol-3-yl]-[7-(1,5-dimethylpyrazol-4-yl)-6,7-dihydro-4H-thieno[3,2-c]pyridin-5-yl]methanone (compound P-33, Table T1)

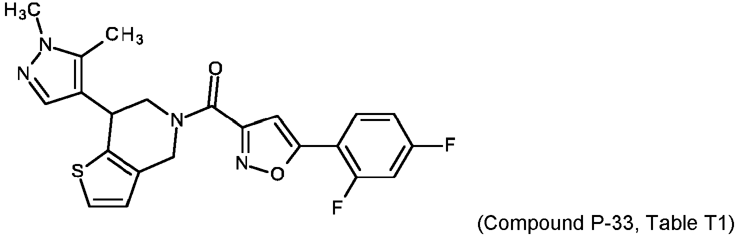

### Step A: Preparation of 7-(1,5-dimethylpyrazol-4-yl)thieno[3,2-c]pyridine

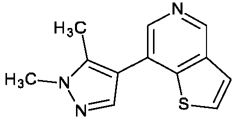

In a microwave vial, to a mixture of 7-bromothieno[3,2-c]pyridine (2.00 g, 9.34 mmol, CAS [1535297-40-5]), 1,5-dimethyl-4-(4,4,5,5-tetramethyl-1,3,2-dioxaborolan-2-yl)pyrazole (4.93 g, 22.2 mmol) and potassium carbonate (2.25 g, 16.28 mmol) were added toluene (37.0 mL) and methanol (3.70 mL). The resulting suspension was degassed with argon for several minutes, followed by the addition of terakis(triphenylphosphine) palladium(0) (0.86 g, 0.74 mmol). The vial was sealed, and the reaction mixture was heated to 100°C and stirred for 1 hour under microwave irradiation. After cooling to room temperature, the reaction mixture was partitioned between a saturated solution of ammonium chloride and ethyl acetate. The aqueous layer was back extracted twice with ethyl acetate and the combined organic layers dried over sodium sulfate, filtered and concentrated *in vacuo.* Purification of the crude material by flash chromatography over silica gel (ethyl acetate ethanol 0 to 30 %) afforded the title compound as a brown solid.

¹H NMR (400 MHz, CDCl₃) δ ppm: 2.39 (s, 3 H) 3.93 (s, 3 H) 7.54 (2d, 2 H) 7.81 (s, 1 H) 8.35 (s, 1 H) 9.08 (s, 1 H).

### Step B: Preparation of 7-(1,5-dimethylpyrazol-4-yl)-4,5,6,7-tetrahydrothieno[3,2-c]pyridine

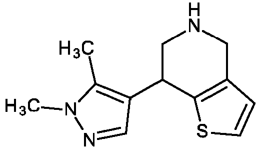

To a solution of 7-(1,5-dimethylpyrazol-4-yl)thieno[3,2-c]pyridine (intermediate prepared as described in step a) *vide supra,* 1.70 g, 7.72 mmol) in methanol (74 mL) was added at room temperature portion wise sodium cyanoborohydride (2.9 g, 46.3 mmol). The reaction mixture was stirred at room temperature, until LC-MS showed reaction completion. The reaction was then treated with hydrochloric acid (44.0 mL 1.25 M in methanol) until the pH reached 3-4. After two hours stirring at room temperature, the reaction mixture was then diluted with water and basified with 2 N sodium hydroxide. Methanol was evaporated under reduced pressure and the remaining aqueous layer extracted three times with ethyl acetate. The combined organic layers were dried over sodium sulfate, filtered and concentrated *in vacuo.* The resulting brown oil was purified by flash chromatography over silica gel (ethyl acetate ethanol 0 to 30 %) to the title compound which was used without further purification.

LC-MS (Method A): retention time 0.26 min, m/z 234 [M+H⁺].

### Step C: Preparation of [5-(2,4-difluorophenyl)isoxazol-3-yl]-[7-(1,5-dimethylpyrazol-4-yl)-6,7-dihydro-4H-thieno[3,2-c]pyridin-5-yl]methanone (compound P-33, Table -T1)

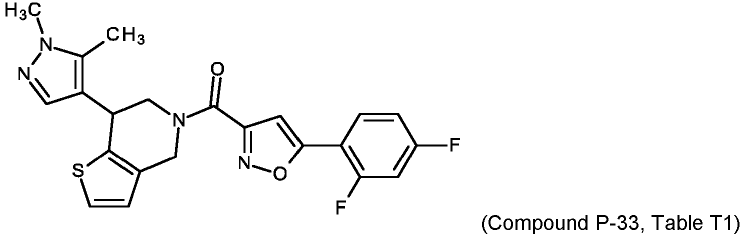

To a solution of 7-(1,5-dimethylpyrazol-4-yl)-4,5,6,7-tetrahydrothieno[3,2-c]pyridine (intermediate prepared as described in step B above, 150 mg, 0.64 mmol) and 5-(2,4-difluorophenyl)isoxazole-3-carboxylic acid (145 mg, 0.64 mmol) in ethyl acetate (1.3 mL) was added at room temperature propylphosphonic acid anhydride as a 50% solution in ethyl acetate (1.02 g, 1.60 mmol), followed by the addition of N,N-diisopropylethylamine (430 mg, 3.20 mmol). The reaction mixture was stirred at room temperature overnight and the reaction mixture was then partitioned between water and ethyl acetate. The aqueous layer was back extracted twice with ethyl acetate and the combined organic layers dried over sodium sulfate, filtered and concentrated *in vacuo.* Purification of the crude material by flash chromatography over silica gel (cyclohexane ethyl acetate 10 to 100 %) afforded the title compound as a white solid. Melting point: 160 - 161°C.

¹H NMR (400 MHz, CDCl₃) δ ppm: 2.20 + 2.29 (2s, 3 H) 3.33-3.42 + 3.68-3.72 (2m, 1 H) 3.75 1 3.85 (2s, 3 H) 4.28-4.32 + 4.34-4.38 (2m, 1 H) 4.43-4.48 + 4.75-4.80 (2m, 1 H) 4.71-4.76 + 4.82-4.87 (2m, 1 H) 5.20-5.25 + 5.30-5.35 (2m, 1 H) 6.78-6.81 (m, 1 H) 6.90-6.92 (m, 1 H) 6.94-7.13 (m, 3 H) 7.19-7.28 (m, 2 H) 7.93-8.14 (m, 1 H).

### Example 6: This example illustrates the preparation of [5-(2,4-difluorophenyl)isoxazol-3-yl]-[7-(1,5-dimethylpyrazol-4-yl)-6,7-dihydro-4H-thiadiazolo[4,5-c]pyridin-5-yl]methanone (compound P-27, Table T1)

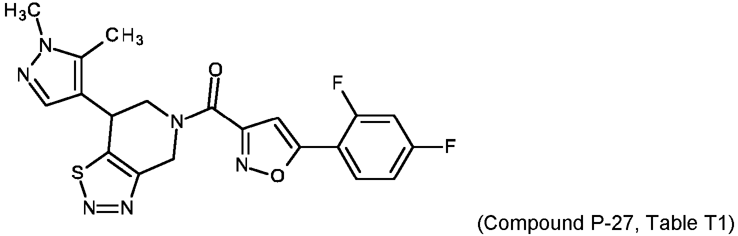

### Step A: Preparation of 3-bromo-4-chloro-5-nitro-pyridine

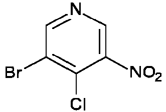

A mixture of phosphoryl chloride (21.5 ml, 228 mmol) and 3-bromo-5-nitro-pyridin-4-ol (10.0 g, 45.6 mmol) in toluene (20 mL) was heated to 90°C for 12 hours. The progress of the reaction was monitored by LC-MS. The reaction mass was cooled to room temperature and phosphoryl chloride was removed under reduced pressure. The reaction was quenched with ice water, extracted with ethyl acetate (X3) and the combined organic layers was washed with brine, dried over sodium sulfate and concentrated *in vacuo* to afford 3-bromo-4-chloro-5-nitro-pyridine as solid.

¹H NMR (400 MHz, DMSO-d6) δ ppm 9.20 (s, 1 H), 9.17 (s, 1 H). LC-MS (Method D): retention time 1.08 min, 237 (M+H).

### Step B: Preparation of 3-bromo-5-nitro-pyridine-4-thiol

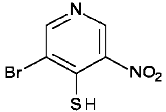

To a solution of 3-bromo-4-chloro-5-nitro-pyridine (2.0 g, 8.42 mmol) in methanol (20 mL) was added sodium hydrosulfide (0.96 g, 16.8 mmol). This reaction mixture was stirred for 2 hours at room temperature. Reaction was monitored by TLC and LC-MS. After completion, the reaction mixture was concentrated *in vacuo* to afford crude 3-bromo-5-nitro-pyridine-4-thiol (1.80 g, 6.90 mmol) was used as such in next step.

LC-MS (Method D): retention time 0.51 min, 235 (M+H).

### Step C: Preparation of 3-amino-5-bromo-pyridine-4-thiol

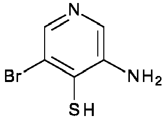

To a solution of 3-bromo-5-nitro-pyridine-4-thiol (2.0 g, 8.50 mmol) in hydrochloric acid (8 mL) was added stannous chloride (3.24 g, 17.0 mmol) in water (8 mL). This reaction mixture was stirred for 3 hours at room temperature. After reaction completion, the reaction mixture was filtered, and the filtrate was concentrated *in vacuo* to afford crude 3-amino-5-bromo-pyridine-4-thiol (2 g) was used as such for the next step.

LC-MS (Method D): retention time 0.19 min, 205 (M+H).

### Step D: Preparation of 7-bromothiadiazolo[4,5-c]pyridine

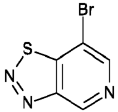

A solution of sodium nitrite (0.52 g, 7.60 mmol) in water (20 mL) was added dropwise to a mixture of 3-amino-5-bromo-pyridine-4-thiol (2.0 g, 5.85 mmol) in hydrochloric acid (2M) (60 mL, 708 mmol) at 0°C. The mixture was kept at this temperature for 12 hours. The progress of the reaction was monitored by LC-MS and upon completion was quenched with sodium bicarbonate, extracted with ethyl acetate (X3) and the combined organic layers were washed with brine, dried over sodium sulfate and concentrated *in vacuo.* The resultant crude residue was purified by chromatography on silica gel, using a cyclohexane / ethyl acetate eluent system, to afford 7-bromothiadiazolo[4,5-c]pyridine.

¹H NMR (400 MHz, CDCl₃) δ ppm 9.90 (s, 1 H) 8.84 (s, 1 H).

LC-MS (Method D): retention time 1.05 min, 216 (M+H).

### Step E: Preparation of 7-(1,5-dimethylpyrazol-4-yl)thiadiazolo[4,5-c]pyridine

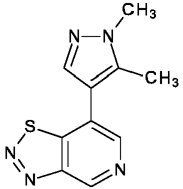

A mixture of 7-bromothiadiazolo[4,5-c]pyridine (0.9 g, 4.16 mmol), 1,5-dimethyl-4-(4,4,5,5-tetramethyl-1,3,2-dioxaborolan-2-yl)pyrazole (1.46 g, 6.24 mmol) and sodium carbonate(1.32 g, 12.5 mmol) in 1,4 dioxane (9 mL) and water (2 mL) was degassed for 5 min with nitrogen. To this solution was added XPhos-Pd-G2 (0.16 g, 0.20 mmol, CAS[1310584-14-5] and the reaction mixture was heated to 80°C for 12 hours. The reaction mass was then diluted with water and extracted twice with ethyl acetate. The combined organic layers were washed water, brine, dried over sodium sulfate, filtered, and concentrated under reduced pressure. The resultant crude residue was purified by trituration with pentane to afford 7-(1,5-dimethylpyrazol-4-yl)thiadiazolo[4,5-c]pyridine.

¹H NMR (400 MHz, DMSO-d6) δ ppm 9.98 (s, 1 H), 8.76 (s, 1 H), 7.79 (s, 1 H), 3.87 (s, 3 H), 2.40 ( s, 3H).

LC-MS (Method D): retention time 0.88 min, 232 (M+H).

### Step F: Preparation of 7-(1,5-dimethylpyrazol-4-yl)-4,5,6,7-tetrahydrothiadiazolo[4,5-c]pyridine

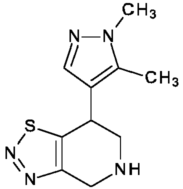

A solution of 7-(1,5-dimethylpyrazol-4-yl)thiadiazolo[4,5-c]pyridine (0.17 g, 0.73 mmol) in methanol (11 mL) was treated with sodium cyanoborohydride (0.48 g, 7.35 mmol) and hydrochloric acid (1.25M in ethanol, 7.35 mL, 9.19 mmol) was added at room temperature. Resulting reaction mixture was stirred 12 hours at room temperature. The reaction was monitored by TLC and LC-MS and after completion was concentrated *in vacuo* to afford crude 7-(1,5-dimethylpyrazol-4-yl)-4,5,6,7-tetrahydrothiadiazolo[4,5-c]pyridine (0.21 g). Crude compound was used as such for the next step.

LC-MS (Method D): retention time 0.17 min, 236 (M+H).

### Step G: Preparation of [5-(2,4-difluorophenyl)isoxazol-3-yl]-[7-(1,5-dimethylpyrazol-4-yl)-6,7-dihydro-4H-thiadiazolo[4,5-c]pyridin-5-yl]methanone (compound P-27, Table T1)

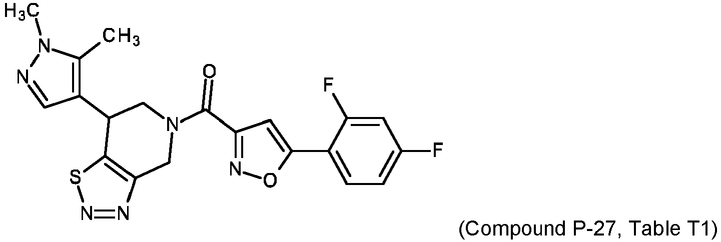

To a solution of 5-(2,4-difluorophenyl)isoxazole-3-carboxylic acid (0.17 g, 0.75 mmol) in acetonitrile (5 mL/mmol) was added N,N-diisopropylethylamine (0.66 mL, 3.77 mmol) followed by 1-propanephosphonic anhydride (1.12 mL, 1.88 mmol). The resulting solution was stirred for 5 minutes under nitrogen atmosphere and then resulting reaction mass then transferred into another reaction flask containing 7-(1,5-dimethylpyrazol-4-yl)-4,5,6,7-tetrahydrothiadiazolo[4,5-c]pyridine (0.21 g, 0.90 mmol) dissolved in 1 mL of acetonitrile. The reaction mixture was stirred overnight at room temperature, monitoring by TLC and LC-MS. After completion, the reaction mixture was dilluted with water, extracted with ethyl acetate (X3) and the combined organic layers washed with brine, dried over sodium sulfate and concentrated *in vacuo.* The resulting crude residue was purified by silica gel chromatography (eluting with cyclohexane/ethyl acetate) to afford [5-(2,4-difluorophenyl)isoxazol-3-yl]-[7-(1,5-dimethylpyrazol-4-yl)-6,7-dihydro-4H-thiadiazolo[4,5-c]pyridin-5-yl]methanone as a solid.

LC-MS (Method D): retention time 1.08 min, 443.2 (M+H).

Melting point: 98°C-100°C.

### Example 7: Preparation of [4-(1,5-dimethylpyrazol-4-yl)-5,7-dihydro-4H-thieno[2,3-c]pyridin-6-yl]-[5-(4-fluorophenyl)isoxazol-3-yl]methanone (compound P-30, Table T1)

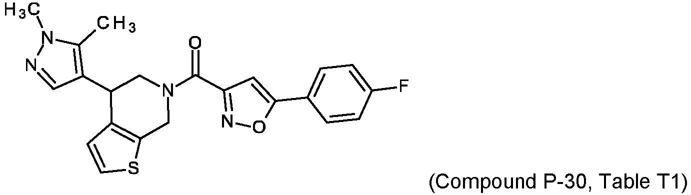

### Step A: Preparation of methyl 4-bromothieno[2,3-c]pyridine-2-carboxylate

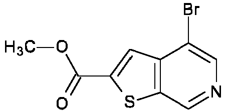

To a stirred solution of 3,5-Dibromopyridine-4-carbaldehyde (1.15 g, 4.12 mmol) in N,N-dimethylacetamide (17.3 mL) cooled to 0°C was added potassium carbonate (0.62 g, 4.54 mmol) and methyl 2-sulfanylacetate (0.46 g, 4.33 mmol) under a nitrogen atmosphere. After 30 minutes, reaction mixture turned to yellowish and the resulting solution was stirred at room temperature and monitored by LC-MS. After reaction completion, the mixture was diluted with water (20 mL) and then extracted with ethyl acetate (X3). The combined organic phases were dried over sodium sulfate and concentrated *in vacuo.* The resulting crude residue was purified by silica gel chromatography (eluting with cyclohexane/ethyl acetate) to afford methyl 4-bromothieno[2,3-c]pyridine-2-carboxylate as white solid.

¹H NMR (400 MHz, CDCl₃) δ = 9.10 (s, 1H), 8.67 (s, 1H), 8.18 (s, 1H), 4.03 (s, 3H).

LC-MS (Method E): retention time 1.47 min, 272 (M+H).

### Step B: Preparation of 4-bromothieno[2,3-c]pyridine

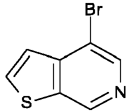

To a solution of methyl 4-bromothieno[2,3-c]pyridine-2-carboxylate (2.4 g, 8.40 mmol) in 1-methyl-2-Pyrrolidinone (42 mL) was added piperazine (3.6 g, 42 mmol) and the mixture stirred at 160°C monitoring reaction progress by LC-MS. After completion, the reaction mass was cooled to room temperature and the reaction mixture carefully diluted with water, and extracted with ethyl acetate (X3). The combined organic layers were washed with brine, dried over sodium sulfate and concentrated *in vacuo.* The resulting crude residue was purified by silica gel chromatography (cyclohexane/ethyl acetate) to afford 4-bromothieno[2,3-c]pyridine (1.5 g, 6.7 mmol) as solid.

¹H NMR (400 MHz, CDCl₃) δ = 9.08 (s, 1H), 8.61 (s, 1H), 7.83 (d, J = 5.5 Hz, 1H), 7.53 (dd, J = 0.7, 5.4 Hz, 1H).

LC-MS (Method E): retention time 1.29 min, 214 (M+H).

### Step C: Preparation of 4-(1,5-dimethylpyrazol-4-yl)thieno[2,3-c]pyridine

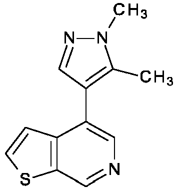

To a degassed mixture of 4-bromothieno[2,3-c]pyridine (0.50 g, 2.34 mmol), 1,5-dimethyl-4-(4,4,5,5-tetramethyl-1,3,2-dioxaborolan-2-yl)pyrazole (1.04 g, 4.67 mmol) and potassium carbonate (0.71 g, 5.13 mmol) in toluene (5.6 mL) and methanol (0.58 mL) in a microwave vial was added tetrakis(triphenylphosphaniumyl)Palladium (0.13 g, 0.12 mmol) and the mixture then heated to 120°C. After reaction completion, the reaction mass was diluted with water and extracted in ethyl acetate (X3). The combined organic layers were dried over sodium sulphate and concentrated *in vacuo.* The resulting crude residue was purified by silica gel chromatography (eluting with cyclohexane/ethyl acetate ) to afford 4-(1,5-dimethylpyrazol-4-yl)thieno[2,3-c]pyridine as a white solid.

¹H NMR (400 MHz, CDCl₃) δ ppm 9.10 (s, 1 H), 8.36 (s, 1 H), 7.72 (d, *J* = 5.50 Hz, 1 H), 7.64 (s, 1 H), 7.39 (d, *J* = 5.38 Hz, 1 H), 3.92 (s, 3 H), 2.32 (s, 3 H).

LC-MS (Method D): Retention time 0.2 min, 230 (M+H).

### Step D: Preparation of 4-(1,5-dimethylpyrazol-4-yl)-4,5,6,7-tetrahydrothieno[2,3-c]pyridin-6-ium chloride

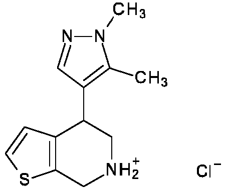

A sample of 4-(1,5-dimethylpyrazol-4-yl)thieno[2,3-c]pyridine (0.11 g, 0.47 mmol) and chloro bis(cyclooctene)Iridium(I) dimer (0.043 g, 0.047 mmol) in a microwave vial under a nitrogen atmosphere were treated with diethylsilane (0.93 mL, 7.02 mmol) and the mixture heated to 55°C for 2 hours under microwave irradiation. The reaction micture was cooled and then was diluted with 1,4-dioxane (1.2 mL). This mixture was transferred to a reaction flask under nitrogen atmosphere and then hydrochloric acid in 1,4-dioxane 4M (2.26 mL) was added slowly dropwise (exotherm observed with gas evolution). This solution was stirred for 1 hour and the solvent was removed *in vacuo* to afford 4-(1,5-dimethylpyrazol-4-yl)-4,5,6,7-tetrahydrothieno[2,3-c]pyridin-6-ium;chloride which was used in the next step without further purification.

### Step E: Preparation of [4-(1,5-dimethylpyrazol-4-yl)-5,7-dihydro-4H-thieno[2,3-c]pyridin-6-yl]-[5-(4-fluorophenyl)isoxazol-3-yl]methanone (compound P-30, Table T1)

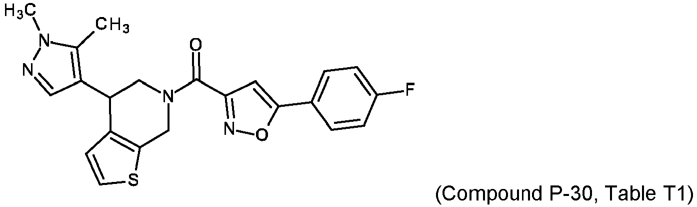

To a two neck round-bottom flask under an argon atmosphere containing 4-(1,5-dimethylpyrazol-4-yl)-4,5,6,7-tetrahydrothieno[2,3-c]pyridine (0.064 g, 0.27 mmol) dissolved in acetonitrile (1.15 mL) was added 5-(4-fluorophenyl)isoxazole-3-carboxylic acid (0.05 g, 0.23 mmol), followed by addition of 1-propanephosphonic anhydride (0.34 mL, 0.57 mmol) and N,N-diisopropylethylamine (0.2 mL, 1.12 mmol). The solution was stirred overnight at room temperature and the reaction mixture then diluted with water and extracted with ethyl acetate (X3). The combined organic layers were washed with brine, dried over sodium sulfate and concentrated *in vacuo.* The crude product was purified by silica gel chromatography (eluting with cyclohexane/ethylacetate) to afford [4-(1,5-dimethylpyrazol-4-yl)-5,7-dihydro-4H-thieno[2,3-c]pyridin-6-yl]-[5-(4-fluorophenyl)isoxazol-3-yl]methanone as gummy mass.

LC-MS (Method E): retention time 1.50 min, 423 (M+H).

### Example 8: Preparation of [5-(2,4-difluorophenyl)-1,3,4-thiadiazol-2-yl]-[4-(1,5-dimethylpyrazol-4-yl)-5,7-dihydro-4H-thieno[2,3-c]pyridin-6-yl]methanone (compound P-25, Table T1)

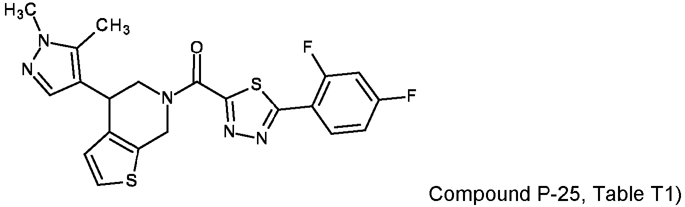

To a solution of ethyl 5-(2,4-difluorophenyl)-1,3,4-thiadiazole-2-carboxylate (0.10 g, 0.33 mmol, prepared as described in OPRD, 2020, 24, 228-234) in toluene (1 mL) was added 4-(1,5-dimethylpyrazol-4-yl)-4,5,6,7-tetrahydrothieno[2,3-c]pyridin-6-ium;chloride (0.10 g, 0.39 mmol, prepared as per example 7) followed by addition of bis(trimethylaluminum)-1,4-diazabicyclo[2.2.2]octane adduct (0.13 g, 0.49 mmol) at 0°C. The resulting mixture was heated to 70°C under microwave irradiation for 3 hours. After this time the reaction was incomplete so a further portion of bis(trimethylaluminum)-1,4-diazabicyclo[2.2.2]octane adduct (0.10 g, 0.39 mmol) was added and the reaction mixture heated under microwave irradiation to 70°C until reaction completion. The reaction mixture was cooled to 0°C, quenched with water and extracted with ethyl acetate (X3). The combined organic layers were washed with brine, dried over sodium sulphate and concentrated *in vacuo.* The crude product was purified by chromatography on silica gel eluting with cyclohexane/ethyl acetate to afford [5-(2,4-difluorophenyl)-1,3,4-thiadiazol-2-yl]-[4-(1,5-dimethylpyrazol-4-yl)-5,7-dihydro-4H-thieno[2,3-c]pyridin-6-yl]methanone as pale yellow soild.

LC-MS (Method E): retention time 1.44 min, 458.1 (M+H).

Melting point: 169-171°C.

### Example 9: Preparation of [5-(2,4-difluorophenyl)isoxazol-3-yl]-[7-(1,5-dimethylpyrazol-4-yl)-6,7-dihydro-4H-thiazolo[4,5-c]pyridin-5-yl]methanone (compound P-28, Table T1)

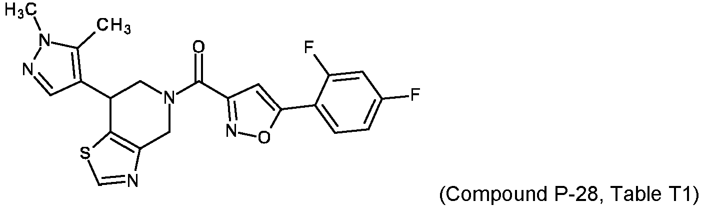

### Step A: Preparation of 7-bromothiazolo[4,5-c]pyridine

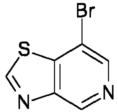

A solution of 3-amino-5-bromo-pyridine-4-thiol (1.5 g, 4.40 mmol, prepared as described in example 6) in formic acid (12 mL) was treated with zinc (0.14 g, 2.20 mmol) and the reaction mixture was heated to 100°C. After reaction completion, it was concentrated *in vacuo* and the resulting crude product purified by silica gel chromatography (eluting with cyclohexane/ethyl acetate) to afford 7-bromothiazolo[4,5-c]pyridine.

¹H NMR (400 MHz, DMSO-d6) δ ppm 9.60 (s, 1 H), 9.35 (s, 1 H), 8.70 (s, 1 H).

LC-MS (Method E): retention time 1.09 min, 214.8 (M+H).

### Step B: Preparation of 7-(1,5-dimethylpyrazol-4-yl)thiazolo[4,5-c]pyridine

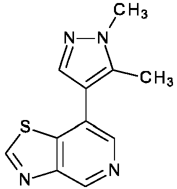

A mixture of 7-bromothiazolo[4,5-c]pyridine (1.0 g, 4.64 mmol) and 1,5-dimethyl-4-(4,4,5,5-tetramethyl-1,3,2-dioxaborolan-2-yl)pyrazole (1.63 g, 6.97 mmol) in 1,4 dioxane (10 mL) and water (2 mL) was degassed for 15 min with nitrogen, followed by addition of sodium carbonate (1.47 g, 13.9 mmol) and XPhos-Pd-G2 (0.18 g, 0.23 mmol, CAS[1310584-14-5]). The reaction mixture was heated to 80°C for 12 hours and then diluted with water and extracted ethyl acetate (X3). The combined organic layers were washed with water, brine, dried over sodium sulfate, filtered and concentrated *in vacuo.* The crude product was purified by washing with pentane to afford 7-(1,5-dimethylpyrazol-4-yl)thiazolo[4,5-c]pyridine as a solid.

¹H NMR (400 MHz, DMSO-d6) δ ppm 9.57 (s, 1 H), 9.29 (s, 1 H), 8.49 (s, 1 H), 7.75 (s, 1 H), 3.86 (s, 3 H), 2.36 (s, 3 H).

LC-MS (Method D): retention time 0.17 min, 231.1 (M+H).

### Step C: Preparation of 7-(1,5-dimethylpyrazol-4-yl)-4,5,6,7-tetrahydrothiazolo[4,5-c]pyridinium;chloride

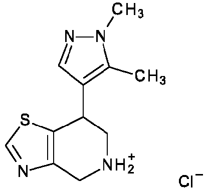

To a solution of 7-(1,5-dimethylpyrazol-4-yl)thiazolo[4,5-c]pyridine (0.25 g, 1.08 mmol) in methanol (16 mL) was added sodium cyanoborohydride (0.71 g, 10.8 mmol) and hydrochloric acid (1.25 M in ethanol 10.8 mL) at room temperature. The reaction mixture was stirred at room temperature for 12 hours. The reaction mixture was concentrated *in vacuo* to afford crude title compound that was used without further purification for the next step.

LC-MS (Method D): retention time 0.20 min, 235.2 (M+H).

### Step D: Preparation of [5-(2,4-difluorophenyl)isoxazol-3-yl]-[7-(1,5-dimethylpyrazol-4-yl)-6,7-dihydro-4H-thiazolo[4,5-c]pyridin-5-yl]methanone (compound P-28, Table T1)

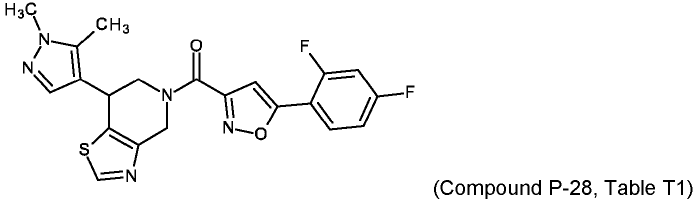

To 5-(2,4-difluorophenyl)isoxazole-3-carboxylic acid (0.25 g, 1.11 mmol) and 7-(1,5-dimethylpyrazol-4-yl)-4,5,6,7-tetrahydrothiazolo[4,5-c]pyridine (0.31 g, 1.33 mmol) in acetonitrile (5.5 mL) was added 1-propanephosphonic anhydride solution (1.65 mL, 2.77 mmol, T3P 50% in ethyl acetate) and N,N-diisopropylethylamine (0.97 mL, 5.55 mmol). The reaction mixture was stirred for 12 hours at room temprature. Reaction was monitored by TLC and LC-MS. After completion, the reaction mixture was dilluted with water extracted with ethyl acetate and the organic layer was washed with brine, dried over sodium sulfate and concentrated under reduced pressure. The resulting crude residue was purified by silica gel chromatography (cyclohexane/ethyl acetate) to afford [5-(2,4-difluorophenyl)isoxazol-3-yl]-[7-(1,5-dimethylpyrazol-4-yl)-6,7-dihydro-4H-thiazolo[4,5-c]pyridin-5-yl]methanone (0.045 g, 0.096 mmol).

LC-MS (Method D): retention time 1.07 min, 442.2 (M+H).

### Example 10: Preparation of [5-(2,4-difluorophenyl)-1,3,4-thiadiazol-2-yl]-[4-(1-methylpyrazol-4-yl)-5,7-dihydro-4H-thieno[2,3-c]pyridin-6-ylimethanone (compound P-24, Table T1)

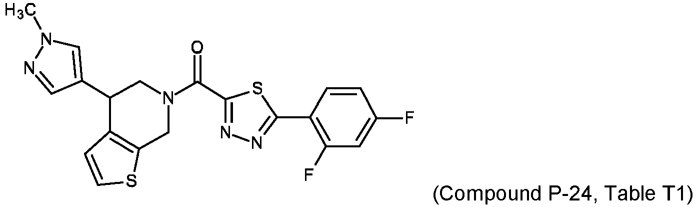

### Step A: Preparation of 4-(1-methylpyrazol-4-yl)thieno[2,3-c]pyridine

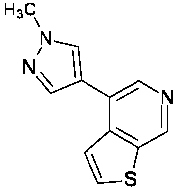

A microwave vial containing a stirring solution of 4-bromothieno[2,3-c]pyridine (0.47 g, 2.19 mmol, prepared as described in example 7), 1-methyl-1H-pyrazole-4-boronic acid (0.42 g, 3.29 mmol) and sodium carbonate (0.69 g, 6.58 mmol) in 1,4-dioxane (4.7 mL) and water (4.3 mL) was degassed with nitrogen for 5 minutes. To this solution Chloro(2-dicyclohexylphosphino-2',4',6'-triisopropyl-1,1'-biphenyl)[2-(2'-amino-1,1'-biphenyl)]palladium(II) (0.088 g, 0.10 mmol) was added and the mixture heated to 120°C under microwave irradiation. The reaction mixture was quenched with ammonium chloride solution, diluted with water and extracted twice with ethyl acetate. The combined organic layers were washed with water, brine, dried over anhydrous sodium sulfate and concentrated *in vacuo.* The product was purified by silica gel chromatography (eluting with cyclohexane/ethyl acetate) to afford 4-(1-methylpyrazol-4-yl)thieno[2,3-c]pyridine as a solid.

¹H NMR (400 MHz, CHLOROFORM-d); δ = 8.99 (s, 1 H), 8.44 (s, 1H), 7.78 (s, 1 H), 7.67 - 7.70 (m, 1 H), 7.64 - 7.66 (m, 1 H), 7.53 (d, *J*=5.50 Hz, 1 H), 3.96 (s, 3 H).

LC-MS (Method D): retention time 0.26 min, 216.1 (M+H).

### Step B: Preparation of 4-(1-methylpyrazol-4-yl)-4,5,6,7-tetrahydrothieno[2,3-c]pyridin-6-ium;chloride

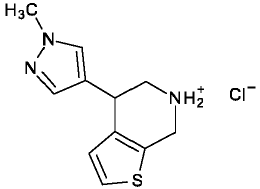

In a microwave vial, 4-(1-methylpyrazol-4-yl)thieno[2,3-c]pyridine (0.1 g, 0.46 mmol), chloro bis(cyclooctane)Iridium(I) dimer (0.42 g., 0.046 mmol) and diethylsilane (0.91 mL., 6.967 mmol) were added under nitrogen atmosphere. The reaction mass was heated to 58°C under microwave irradiation for 2 hours. The reaction mass was diluted with 1,4-dioxane (1 mL) and then transferred to a round bottom flask, under nitrogen atmosphere and then hydrochloric acid (4 mol/L) in 1,4-dioxane (2 mL, 8 mmol) was added slowly dropwise and the mixture stirred for 1 hour. The volatiles were then removed *in vacuo* to obtain the crude 4-(1-methylpyrazol-4-yl)-4,5,6,7-tetrahydrothieno[2,3-c]pyridin-6-ium;chloride which was used as such for the next step.

LC-MS (Method E): retention time 0.31 min, 220.1 (M+H).

### Step C: Preparation of [5-(2,4-difluorophenyl)-1,3,4-thiadiazol-2-yl]-[4-(1-methylpyrazol-4-yl)-5,7- dihydro-4H-thieno[2,3-c]pyridin-6-yl]methanone (compound P-24, Table T1)

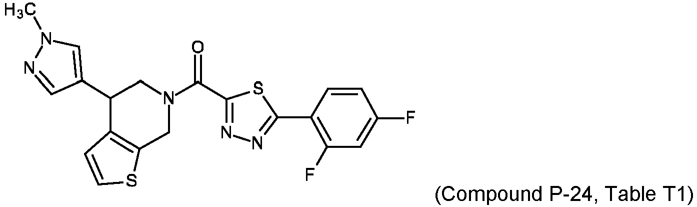

In a microwave vial, ethyl 5-(2,4-difluorophenyl)-1,3,4-thiadiazole-2-carboxylate (0.10 g, 0.37 mmol) in toluene (1 mL) was treated with 4-(1-methylpyrazol-4-yl)-4,5,6,7-tetrahydrothieno[2,3-c]pyridin-6-ium;chloride (0.12 g, 0.44 mmol), and the resulting reaction mixture was cooled to 0°C. To this mixture was added (bis(trimethylaluminum)-1,4-diazabicyclo[2.2.2]octane (0.38 g, 1.48 mmol) and the reaction mixture was then subjected to microwave heating at 70°C for 3 hours. The reaction mixture was quenched with ice water, and extracted with ethyl acetate (X3). The combined organic layers were washed with brine, dried over anhydrous sodium sulfate and concentrated *in vacuo.* The resulting crude product was purified by silica gel chromatography to give [5-(2,4-difluorophenyl)-1,3,4-thiadiazol-2-yl]-[4-(1-methylpyrazol-4-yl)-5,7-dihydro-4H-thieno[2,3-c]pyridin-6-yl]methanone as a solid.

LC-MS (Method E): retention time 1.35 min, 444.1 (M+H).

Melting point: 73-75°C.

### Example 11: This example illustrates the preparation of [5-(2,4-difluorophenyl)isoxazol-3-yl]-[4-(1-methylpyrazol-4-yl)-5,7-dihydro-4H-isothiazolo[5,4-c]pyridin-6-ylimethanone (compound P-26, Table T1)

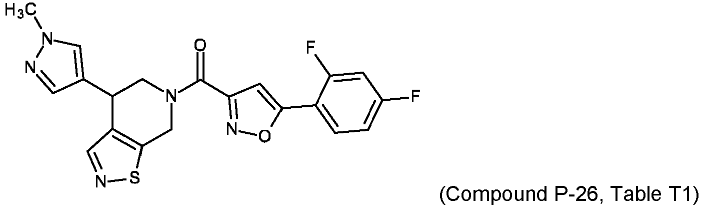

### Step A: Preparation of 3-bromo-5-fluoro-pyridine-4-carbaldehyde

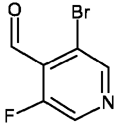

To a solution of 3-bromo-5-fluoro-pyridine (12.0 g, 68.1 mmol) in tetrahydrofuran (120 mL), cooled to -78 °C was added lithium diisopropylamide (75 mL, 150 mmol) dropwise at -78°C (the reaction mixture turned brown in color) and the resulting reaction mixture was stirred at this temperature for 45 minutes. To this reaction mixture was added ethyl formate (56 mL, 681mmol) over 15 minutes and the reaction mixture stirred for additional 1.5 hours at -78 °C. After the completion of reaction (monitored by TLC and GCMS), the reaction mixture was quenched with saturated solution of ammonium chloride and the resulting mixture was extracted with ethyl acetate (X3). The combined organic layers were washed with brine, dried and concentrated *in vacuo* to obtain a brown residue which was subjected to combiflash chromatography using a cyclohexane / ethyl acetate as eluent. This gave 3-bromo-5-fluoro-pyridine-4-carbaldehyde as a bright yellow solid.

¹H NMR (400 MHz, CDCl₃) δ ppm 10.35 (s, 1 H), 8.74 (s, 1 H), 8.60 (s, 1 H).

### Step B: Preparation of 3-bromo-5-tert-butylsulfanyl-pyridine-4-carbaldehyde

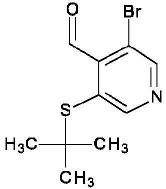

A mixture of 3-bromo-5-fluoro-pyridine-4-carbaldehyde (6.8 g, 30 mmol), potassium carbonate (5.1 g, 36 mmol) and 2-methyl-2-propanethiol (3.0 mL, 30 mmol) in dry N,N-dimethylformamide (20 mL) was taken in Teflon vessel and heated to 110°C in a sealed tube for 32 hours. The reaction was cooled to room temperature, water was added, and the mixture was extracted with ethyl acetate (X3). The combined organic fractions were washed with water, dried over sodium sulfate, filtered, and concentrated *in vacuo* to afford the crude title compound, which was purified using combiflash chromatography using ethyl acetate / cyclohexane as eluent to obtain 3-bromo-5-tert-butylsulfanyl-pyridine-4-carbaldehyde as a yellow oil.

¹H NMR (400 MHz, CDCl₃) δ ppm 10.5 (s, 1 H), 8.85 (s, 1 H), 8.73 (s, 1 H), 1.34 (s, 9 H).

### Step C: Preparation of 4-bromoisothiazolo[5,4-c]pyridine

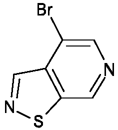

A mixture of 3-bromo-5-tert-butylsulfanyl-pyridine-4-carbaldehyde (3.8 g, 12.0 mmol) and hydroxylamine hydrochloride (4.4 g, 62.0 mmol) in isopropanol (160 mL) and water (33 mL) was heated to 90°C for 16 hours. The reaction mixture was cooled, and 2-propanol was removed *in vacuo.* Water was added to the residue, followed by saturated aqueous sodium bicarbonate until the pH was ~ 8. The mixture was extracted with ethyl acetate (X3) and the combined organic fractions were dried over sodium sulfate, filtered, and concentrated *in vacuo* to afford the crude title compound, which was used in the next step without without further purification. The crude oxime obtained was then suspended in polyphosphoric acid (15 mL) and heated to 110°C for 2 hours. The reaction mixture was cooled to room temperature, and extracted with ethyl acetate, dried over sodium sulfate and concentrated *in vacuo.* The crude reside was subjected to combiflash purification using ethyl acetate / cyclohexane as eluent, to afford pure 4-bromoisothiazolo[5,4-c]pyridine as a white solid.

¹H NMR (400 MHz, CDCl₃) δ ppm 9.29 (s, 1 H), 9.09 (d, *J* = 0.61 Hz, 1 H), 8.69 (s, 1 H).

LC-MS (Method D): retention time 0.98 min, 215 (M+H).

### Step D: Preparation of 4-(1-methylpyrazol-4-yl)isothiazolo[5,4-c]pyridine

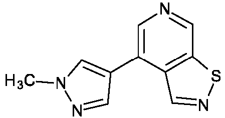

A microwave vial was charged with 4-bromoisothiazolo[5,4-c]pyridine (0.17 g, 0.79 mmol), 1-methyl-1H-pyrazole-4-boronic acid (0.15 g, 1.18 mmol), sodium carbonate (0.25 g, 2.37 mmol), 1,4-dioxane (1.7 mL) and water (1.6 mL). The reaction mixture was degassed with nitrogen for 5.0 minutes, then XPhos-Pd-G2 (0.031 g, 0.039 mmol) was added and the mixture heated to 120°C for 2 hours under microwave irradiation. After completion of the reaction (monitored by TLC and LC-MS), the reaction mixture was filtered through celite, washed with ethyl acetate and the filtrate concentrated *in vacuo* to obtain a brown residue which on purification by combiflash using ethyl acetate / cyclohexane eluent system, afforded pure 4-(1-methylpyrazol-4-yl)isothiazolo[5,4-c]pyridine as a white solid.

¹H NMR (400 MHz, CDCl₃) δ ppm 9.26 (s, 1 H), 9.19 (s, 1 H), 8.62 (s, 1 H), 7.92 (s, 1 H), 7.81 (s, 1 H), 4.07 (s, 3 H).

LC-MS (Method D): retention time 0.55 min; 217.1 (M+H).

### Step E: Preparation of 4-(1-methylpyrazol-4-yl)-4,5,6,7-tetrahydroisothiazolo[5,4-c]pyridine

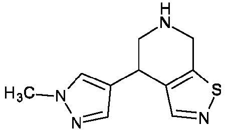

A round bottomed flask, equipped with a magnetic stirrer bar, was charged with 4-(1-methylpyrazol-4-yl)isothiazolo[5,4-c]pyridine (0.15 g, 0.69 mmol) and methanol (10.4 mL) to obtain a clear solution. To this solution was added sodium cyanoborohydride (0.45 g, 6.93 mmol) portionwise at 0°C. Then, methanolic hydrochloric acid (6.9 mL, 20.8 mmol, 3 mol/L) was added dropwise at room temperature (evolution of gas was observed) and the mixture was stirred at room temperature overnight. After the completion of the reaction (monitored by TLC and LC-MS), the reaction mixture was stirred with basic resin until the solution is basic to pH paper. The mixture was filtered and washed with methanol and the clear solution was concentrated to get the crude 4-(1-methylpyrazol-4-yl)-4,5,6,7-tetrahydroisothiazolo[5,4-c]pyridine (0.1 g, 0.43 mmol) brownish yellow residue.

¹H NMR (400 MHz, DMSO-d6) δ ppm 8.17 (s, 1 H), 7.48 (s, 1 H), 7.29 (s, 1 H), 4.08 (br d, *J =* 2.9 Hz, 2 H), 3.92 - 4.24 (m, 1 H), 3.77 (s, 3 H), 3.75 (s, 1 H), 3.12 (br dd, *J =* 12.9, 5.2 Hz, 1 H), 2.73 (dd, *J =* 12.9, 7.2 Hz, 1 H).

LC-MS (Method D): retention time 0.19 min; 221.1 (M+H).

### Step F: Preparation of [5-(2,4-difluorophenyl)soxazol-3-yl]-[4-(1-methylpyrazol-4-yl)-5,7-dihydro-4H-isothiazolo[5,4-c]pyridin-6-yl]methanone (compound P-26, Table T1)

A solution of 5-(2,4-difluorophenyl)isoxazole-3-carboxylic acid (0.09 g, 0.40 mmol) in ethyl acetate (1.7 mL) was treated with N,N-diisopropylethylamine (0.13 mL, 0.73 mmol) under stirring. To this reaction mixture was added 4-(1-methylpyrazol-4-yl)-4,5,6,7-tetrahydroisothiazolo[5,4-c]pyridine (0.085 g, 0.36 mmol) followed by 1-propanephosphonic anhydride (50 mass%) in ethyl acetate (0.65 mL, 1.1 mmol) and the reaction mixture was stirred at room temperature for overnight. After the completion of the reaction (monitored by TLC and LC-MS), the reaction mixture was quenched with NaHCO₃ and extracted with ethyl acetate (X3). The combined organic layers were dried over sodium sulfate, and concentrated *in vacuo.* The crude material was purified by reverse-phase combiflash using acetonitrile / water eluent system to afford [5-(2,4-difluorophenyl)isoxazol-3-yl]-[4-(1-methylpyrazol-4-yl)-5,7-dihydro-4H-isothiazolo[5,4-c]pyridin-6-yl]methanone as a white solid.

LC-MS (Method D): retention time 1.07 min; 428.2 (M+H).

### Example 12: Preparation of [5-(2,4-difluorophenyl)isoxazol-3-yl]-[7-(1-methylpyrazol-4-yl)-6,7-dihydro-4H-isothiazolo[4,5-c]pyridin-5-yl]methanone (compound P-23, Table T1)

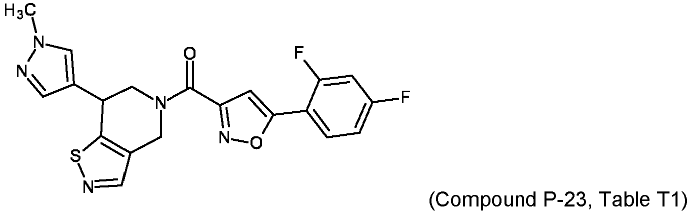

### Step A: Preparation of 4,5-dibromopyridine-3-carboxylic acid

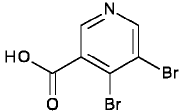

To a cooled (-70 °C) solution of 5-bromopyridine-3-carboxylic acid (12.6 g, 59.3 mmol) in tetrahydrofuran (126 mL) under nitrogen was added lithium diisopropylamide (72 mL, 140 mmol, 2.0 mol/L) dropwise over 1 hour. The solution was stirred for 2.5 hours at -55°C. The mixture was then cooled to -70 °C and treated with 1,2-dibromotetrachloroethane (24.9 g, 74.1 mmol) in portions over 30 minutes. After 1 hour, the reaction mixture was allowed to warm to -20 °C over 2 hours and then water (75 mL) was added slowly. The organic layer was then concentrated *in vacuo* and the aqueous residue was diluted with water (250 mL), then washed with ethyl acetate. The aqueous layer was then acidified to pH 3 by addition of concentrated hydrochloric acid. The precipitated solid was collected by filtration and dried at 60°C *in vacuo* to afford 4,5-dibromopyridine-3-carboxylic acid as an off white to pale brown solid.

¹H NMR (400 MHz, DMSO-d6) δ ppm 8.91 (s, 1 H), 8.73 (s, 1 H).

LC-MS (Method D): retention time 0.23 min, 280.0 (M+H).

### Step B: Preparation of 4,5-dibromo-N-methoxy-N-methyl-pyridine-3-carboxamide

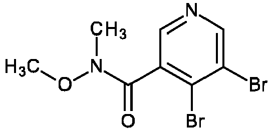

A solution of 4,5-dibromopyridine-3-carboxylic acid (10 g, 35.6 mmol) in ethyl acetate (200 mL) was treated with N,N-Diisopropylethylamine (18.8 mL, 106 mmol) under stirring. To this reaction mixture was added methoxy(methyl)ammonium chloride (5.21 g, 53.4 mmol) followed by 1-propanephosphonic anhydride (50 wt%) in ethyl acetate (200 mL, 106 mmol) and the reaction mixture was stirred at room temperature overnight. After the completion of the reaction (monitored by TLC and LC-MS), the reaction mixture was quenched with NaHCO₃ and extracted with ethyl acetate (X3). The combined organic layers were dried over sodium sulfate and concentrated *in vacuo.* The crude material was purified by combiflash using (ethyl acetate / cyclohexane) as eluent system, to afford 4,5-dibromo-N-methoxy-N-methyl-pyridine-3-carboxamide as a gummy mass.

¹H NMR (400 MHz, CDCl₃) δ ppm 8.73 (s, 1 H), 8.38 (s, 1 H) 3.50 (s, 3 H) 3.40 (s, 3 H).

LC-MS (Method D): retention time 0.97 min, 322.9 (M+H).

### Step C: Preparation of 5-bromo-4-tert-butylsulfanyl-N-methoxy-N-methyl-pyridine-3-carboxamide

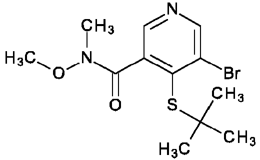

In a 50 mL Teflon vessel was added 4,5-dibromo-N-methoxy-N-methyl-pyridine-3-carboxamide (10.1 g, 29.6 mmol) dissolved in dry *N,N*-dimethylformamide (30 mL). To this solution under nitrogen was added potassium carbonate (5.0 g, 35.8 mmol) followed by addition of 2-methyl-2-propanethiol (3.0 mL, 29.6 mmol) and the reaction mixture was stirred at 130°C for 20 hours. After the completion of the reaction (monitored by TLC and LC-MS), the reaction mixture was cooled to room temperature, quenched with ice and extracted with ethyl acetate (X3). The combined organic layers were dried with sodium sulfate and concentrated *in vacuo* to obtain a brown residue which upon purification by combiflash using (ethyl acetate / cyclohexane) as eluent system, yielded 5-bromo-4-tert-butylsulfanyl-N-methoxy-N-methyl-pyridine-3-carboxamide as a brown gummy mass.

¹H NMR (400 MHz, CDCl3) δ ppm 8.87 (s, 1 H), 8.49 (s, 1 H), 3.38 (s, 3H), 3.37 (s, 3H), 1.38 (s, 9 H).

LC-MS (Method F): retention time 1.13 min, 276.9 (M-tBu).

### Step D: Preparation of 5-bromo-4-tert-butylsulfanyl-pyridine-3-carbaldehyde

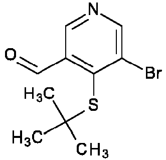

To a solution of compound 5-bromo-4-tert-butylsulfanyl-N-methoxy-N-methyl-pyridine-3-carboxamide (3.05 g, 8.70 mmol) in toluene (122 mL) was added diisobutylaluminium hydride (1.0 mol/L) in toluene (9.6 mL, 9.57 mmol) at -50 °C and stirred at room temperature for 1 hour. After completion of the reaction (monitored by TLC and LC-MS), the mixture was quenched with cold water and extracted with ethyl acetate (X3). The combined organic phases were washed with brine, dried over sodium sulfate and concentrated *in vacuo.* The crude aldehyde was purified by column chromatography using (ethyl acetate / cyclohexane) eluent system, to afford pure 5-bromo-4-tert-butylsulfanyl-pyridine-3-carbaldehyde as an amber coloured oil.

¹H NMR (400 MHz, CDCl3) δ ppm 10.72 (s, 1 H), 9.06 (s, 1 H), 9.01 (s, 1 H), 1.37 (s, 9 H).

LC-MS (Method F): retention time 1.04 min, 218 (M-tBu).

### Step E: Preparation of 5-bromo-4-tert-butylsulfanyl-pyridine-3-carbaldehyde oxime

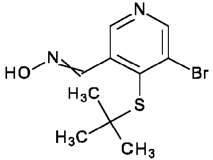

A mixture of 5-bromo-4-tert-butylsulfanyl-pyridine-3-carbaldehyde (1.05 g, 3.64 mmol) and hydroxylamine hydrochloride (1.28 g, 18.2 mmol) in isopropanol (160 mL) and water (33 mL) was heated to 90°C for 16 hours. The reaction mixture was cooled and 2-propanol was removed *in vacuo.* Water was added to residue, followed by saturated aqueous sodium bicarbonate until the pH was ~8. The mixture was extracted with ethyl acetate (X3) and the combined organic layers dried over sodium sulfate, filtered and concentrated *in vacuo* to afford the crude 5-bromo-4-tert-butylsulfanyl-pyridine-3-carbaldehyde oxime as an off white solid which was used without further purification.

¹H NMR (400 MHz, CDCl3) δ ppm 9.07 (s, 1 H), 8.83 (s, 2 H), 1.36 (s, 9 H).

LC-MS (Method D): retention time 1.19 min, 233.0 (M-tBu).

### Step F: Preparation of 7-bromoisothiazolo[4,5-c]pyridine

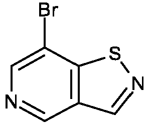

In a 100 mL round-bottomed flask was taken 5-bromo-4-tert-butylsulfanyl-pyridine-3-carbaldehyde oxime (1.09 g, 3.58 mmol) and *p*-toluenesulfonic acid (0.31 g, 1.79 mmol) in 1-butanol (17.9 mL) and heated to 90°C for overnight. After reaction monitoring (by TLC and LC-MS), the reaction was incomplete and thus additional p-toluenesulfonic acid (0.31 g, 1.79 mmol) was added and the mixture stirred at 117°C for 2 hours. The reaction mixture was cooled to room temperature, diluted with water and extracted with ethyl acetate (X3). The combined organic fractions were dried over sodium sulfate and concentrated *in vacuo.* The crude reside was subjected to combiflash purification using (ethyl acetate / cyclohexane) as eluent system to afford pure 7-bromoisothiazolo[4,5-c]pyridine as a white solid.

¹H NMR (400 MHz, CDCl3) δ ppm 9.35 (s, 1 H), 9.18 (s, 1 H), 8.67 (s, 1 H).

LC-MS (Method D): retention time 1.1 min, 215 (M+H).

### Step G: Preparation of 7-(1-methylpyrazol-4-yl)isothiazolo[4,5-c]pyridine

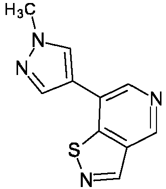

To a solution of 7-bromoisothiazolo[4,5-c]pyridine (0.2 g, 0.88 mmol) in tetrahydrofuran (2 mL) was added 1-methyl-1H-pyrazole-4-boronic acid (0.23 g, 1.76 mmol) followed by addition of cesium fluoride (0.26 g, 1.76 mmol) and the mixture was degassed with argon for 10 minutes before adding bis(tri-tert-butylphosphine)palladium(0) (0.046 g, 0.09 mmol). The mixture was microwaved at 65°C for 2 hours and then cooled to room temperature and diluted with a saturated solution of ammonium chloride. The mixture was extracted with ethyl acetate (X3) and the combined organic layers were washed with brine, dried over sodium sulfate and concentrated *in vacuo.* The crude residue was purified by column chromatography using 10-50% ethyl acetate in cyclohexane to afford pure 7-(1-methylpyrazol-4-yl)isothiazolo[4,5-c]pyridine as a white solid.

¹H NMR (400 MHz, CDCl₃) δ ppm 9.29 (s, 1 H), 9.13 (s, 1 H) 8.73 (s, 1 H), 7.99 (s, 1 H), 7.85 (s, 1 H), 4.06 (s, 3 H).

LC-MS (Method D): retention time 1.06 min, 217.1 (M+H).

### Step H: Preparation of 7-(1-methylpyrazol-4-yl)-4,5,6,7-tetrahydroisothiazolo[4,5-c]pyridine

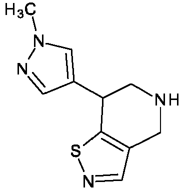

A one necked round bottom flask, equipped with a magnetic stirrer bar, was charged with 7-(1-methylpyrazol-4-yl)isothiazolo[4,5-c]pyridine (0.11 g, 0.48 mmol), methanol (7.25 mL) and sodium cyanoborohydride (0.32. g, 4.83 mmol). Then, a hydrogen chloride solution (4.83 mL, 2 mmol, 0.5 mol/L) was added dropwise at room temperature (gas evolution was observed) and the mixture was stirred at room temperature for overnight. After completion of the reaction (monitored by TLC and LC-MS), the reaction mixture was quenched with water, stirred with basic resin, filtered and then concentrated to get the crude residue which was used as such without further purification.

LC-MS (Method D): retention time 0.23 min, 221.2 (M+H).

### Step I: Preparation of [5-(2,4-difluorophenyl)isoxazol-3-yl]-[7-(1-methylpyrazol-4-yl)-6,7-dihydro-4H-isothiazolo[4,5-c]pyridin-5-yl]methanone (compound P-23, Table T1)

In a 100 mL round bottom flask was taken 5-(2,4-difluorophenyl)isoxazole-3-carboxylic acid (0.12 g, 0.55 mmol) in ethyl acetate (2.2 mL) and to this was added N,N-diisopropylethylamine (0.18 mL, 0.99 mmol) under stirring. To this reaction mixture was added 7-(1-methylpyrazol-4-yl)-4,5,6,7-tetrahydroisothiazolo[4,5-c]pyridine (0.11 g, 0.50 mmol) followed by 1-propanephosphonic anhydride (50 mass%) in ethyl acetate (0.88 mL 1.49 mmol, 50 mass%) and the reaction mixture was stirred at room temperature overnight. After the completion of the reaction (monitored by TLC and LC-MS), the reaction mixture was quenched with saturated solution of NaHCO₃ and extracted with ethyl acetate (X3). The combined organic layers were dried over sodium sulfate and concentrated *in vacuo.* The crude material was purified by reverse-phase combiflash using (acetonitrile / water) eluent system, to afford [5-(2,4-difluorophenyl)isoxazol-3-yl]-[7-(1-methylpyrazol-4-yl)-6,7-dihydro-4H-isothiazolo[4,5-c]pyridin-5-yl]methanone as an off white to pale brown solid.

LC-MS (Method F): retention time 1.03 min, 428.6 (M+H). Melting point: 63-65°C.

Further examples of synthesized compounds of formula (I) are shown in Table T1.

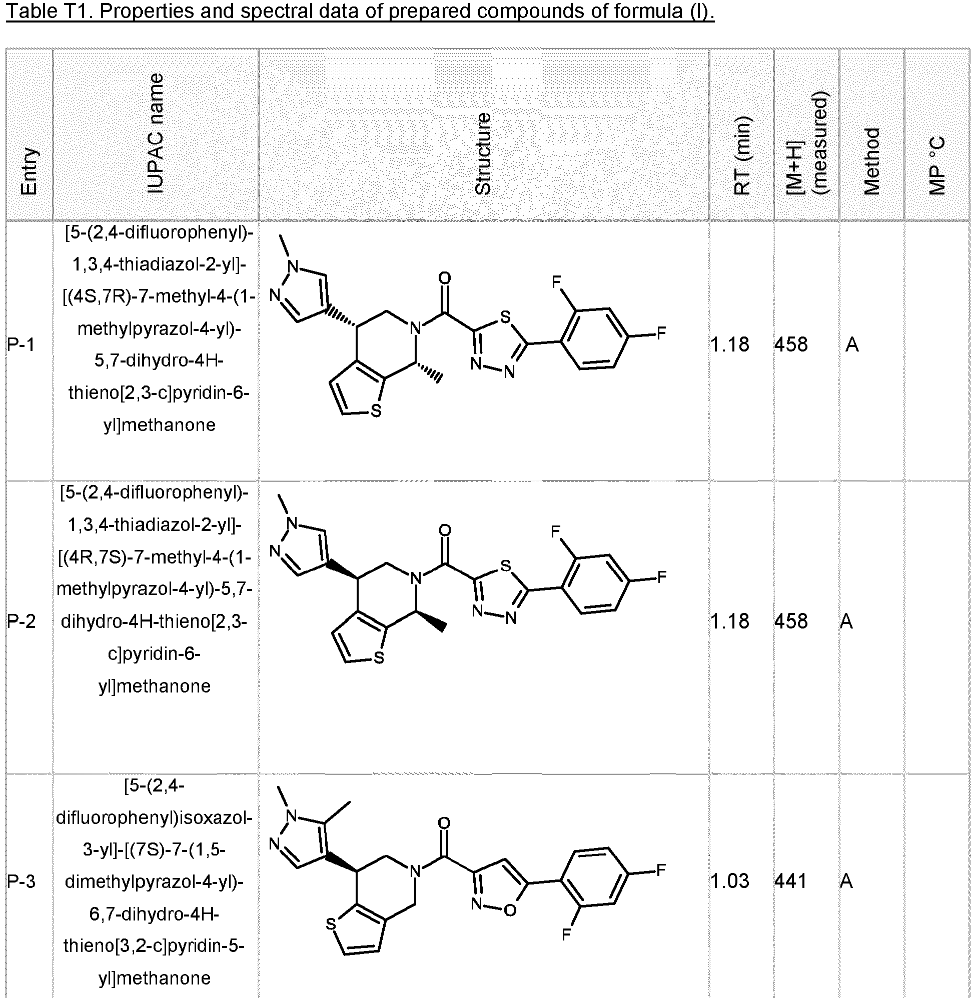

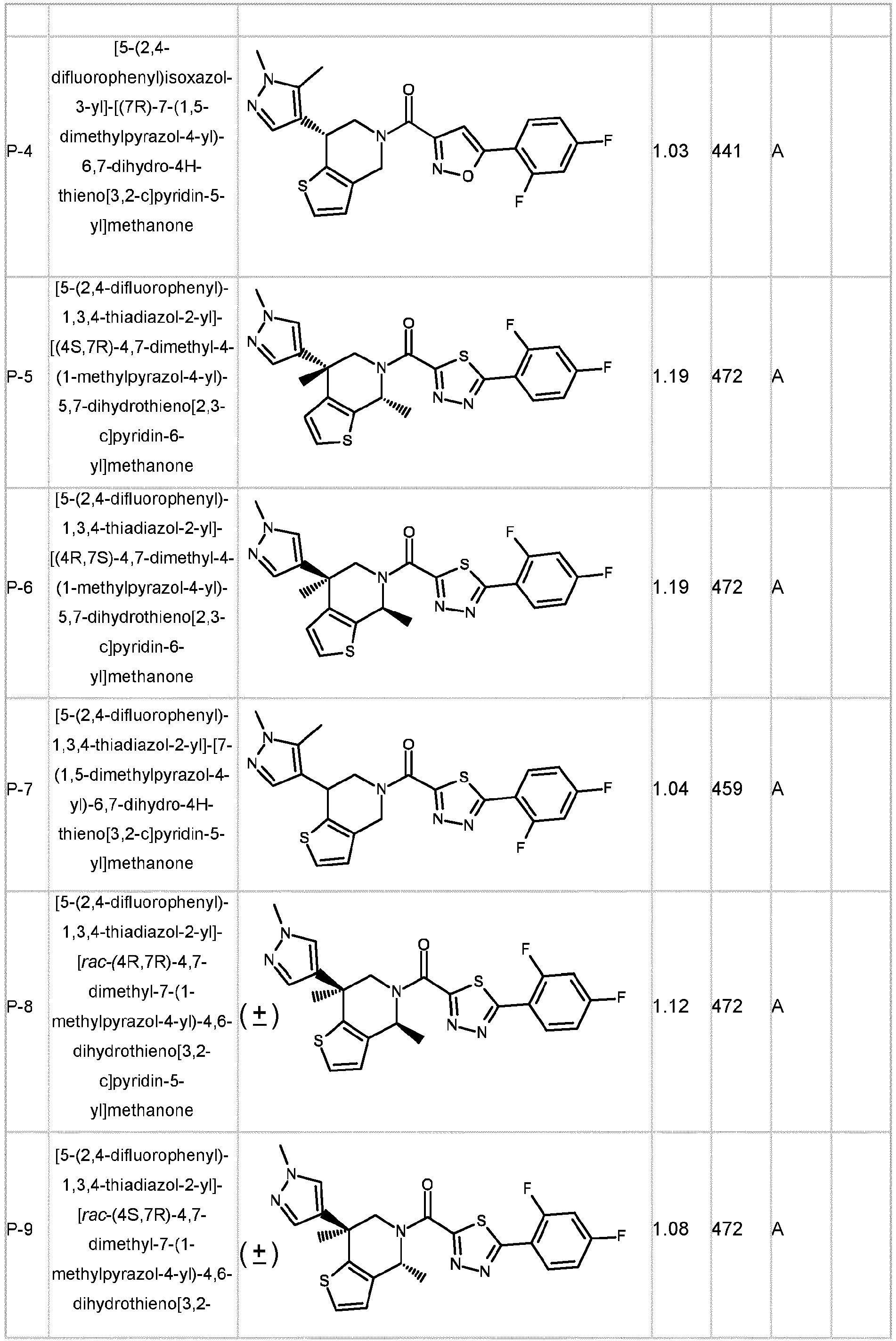

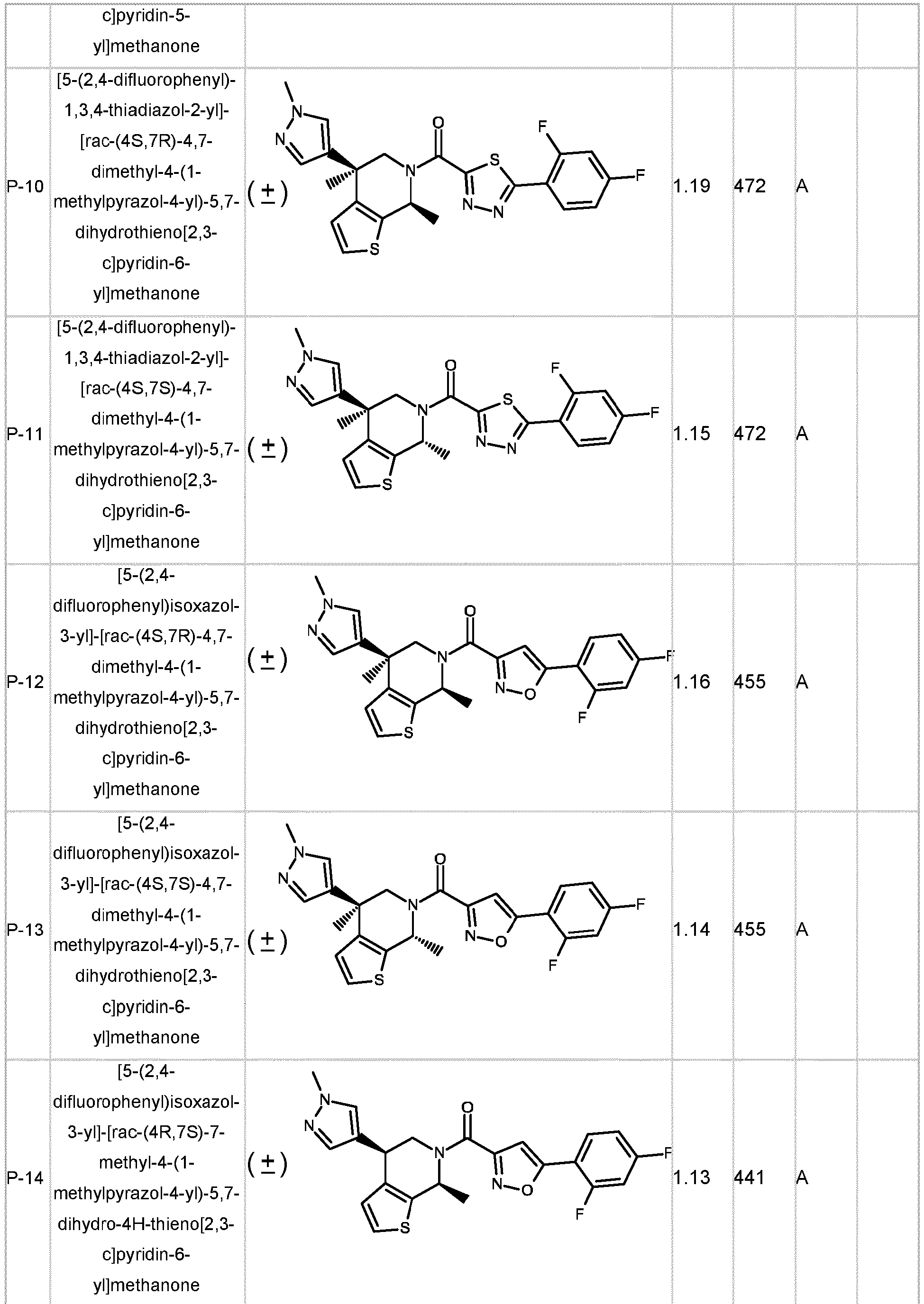

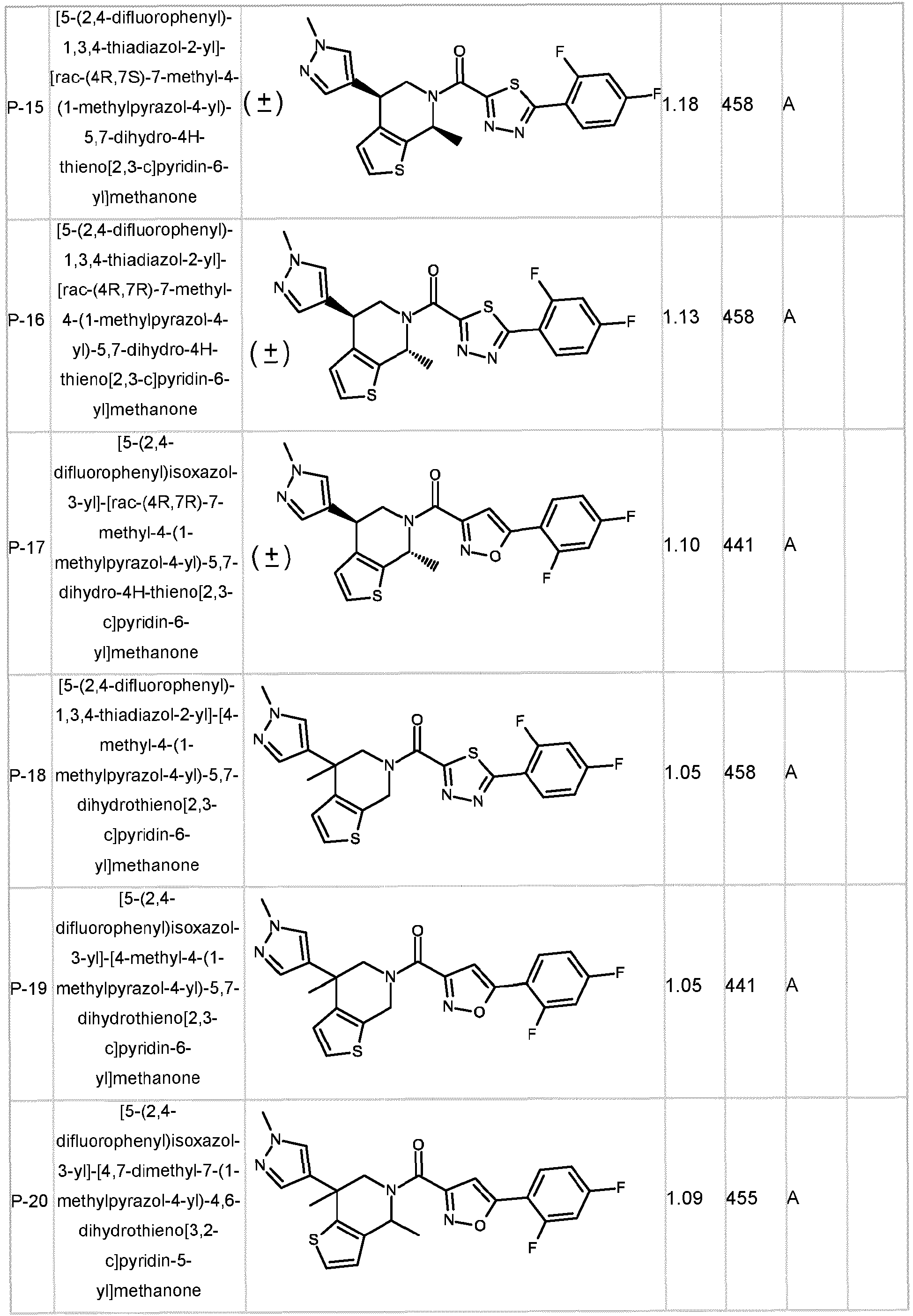

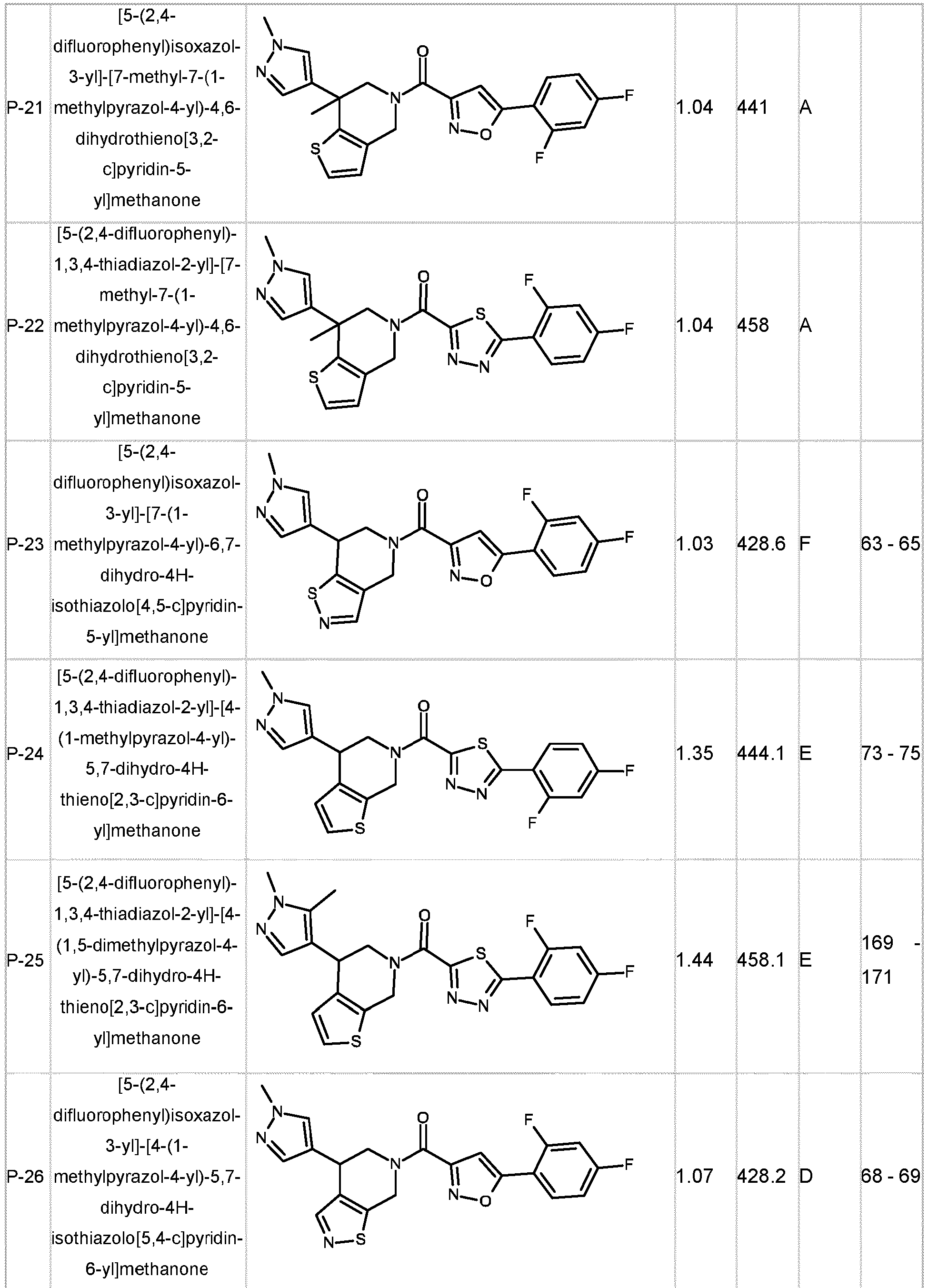

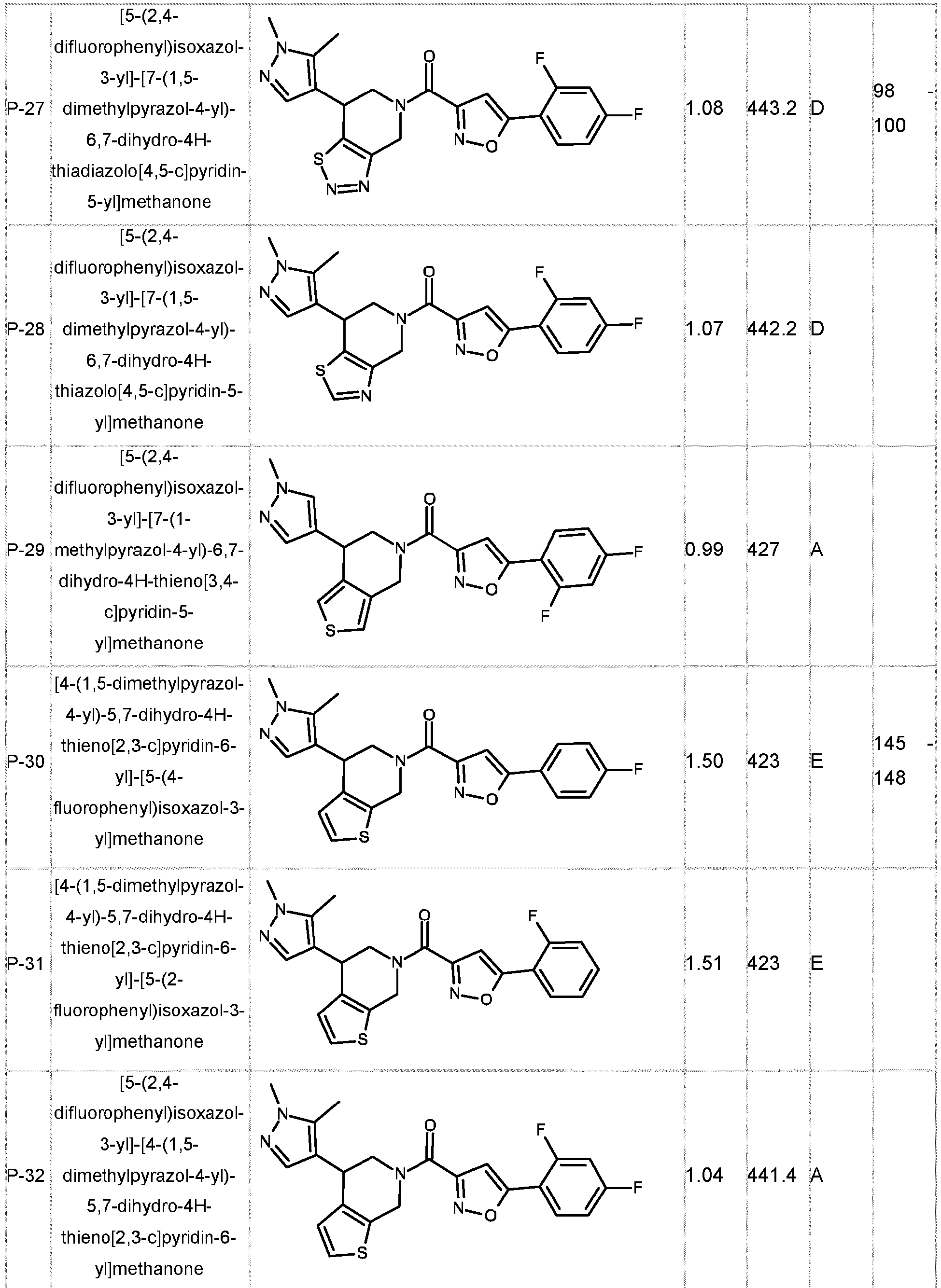

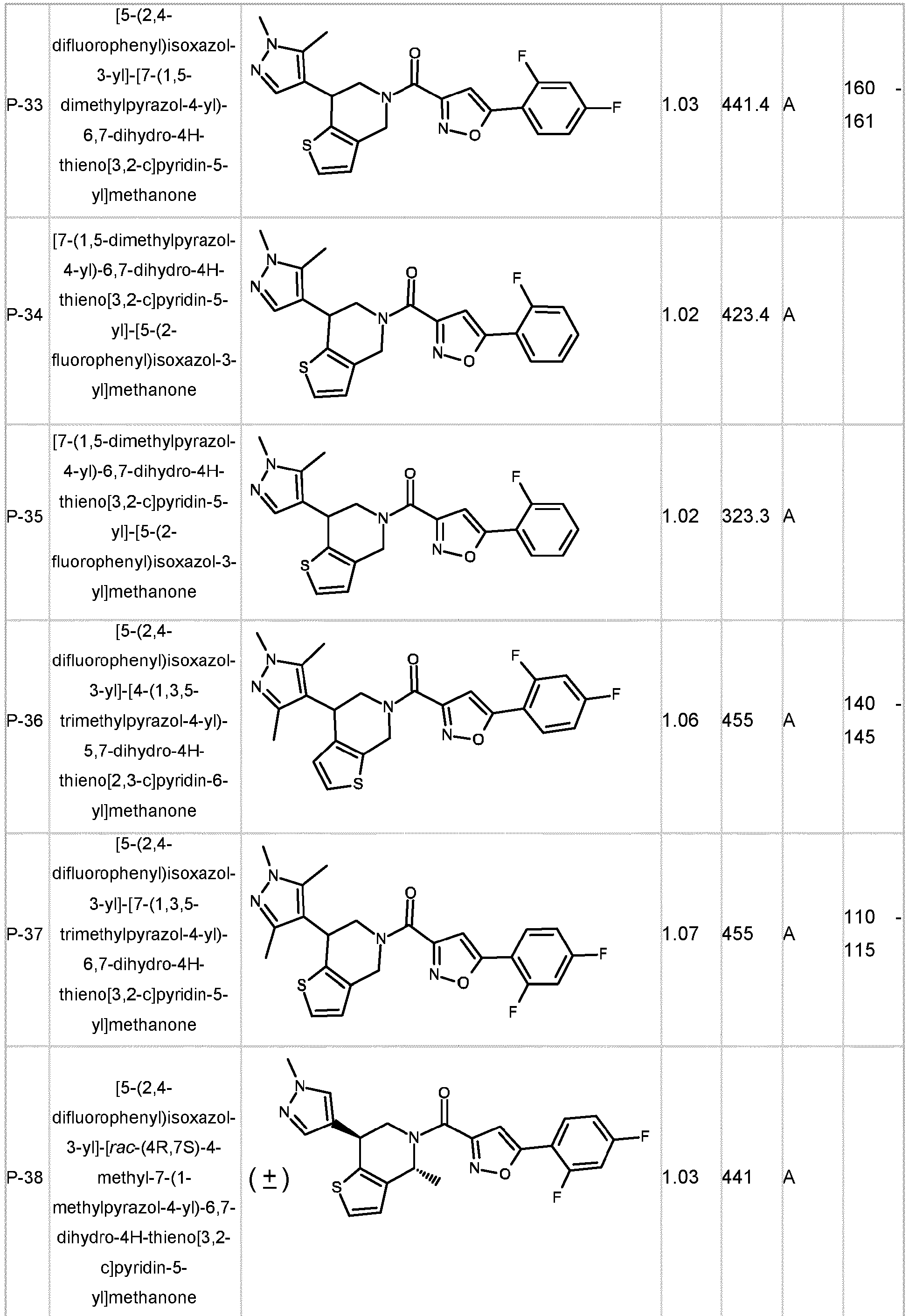

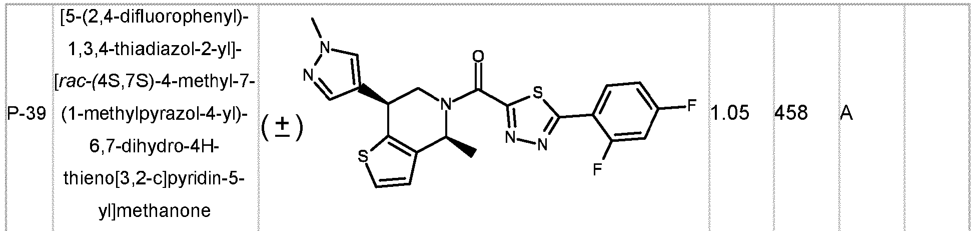

### BIOLOGICAL EXAMPLES

### Example B1: Alternaria solani / tomato / leaf disc (early blight)

Tomato leaf disks cv. Baby are placed on agar in multiwell plates (24-well format) and sprayed with the formulated test compound diluted in water. The leaf disks are inoculated with a spore suspension of the fungus 2 days after application. The inoculated leaf disks are incubated at 23°C / 21°C (day/night) and 80% relative humidity under a light regime of 12 h/12 h (light/dark) in a climate cabinet and the activity of a compound is assessed as percent disease control compared to untreated when an appropriate level of disease damage appears on untreated check disk leaf disks (5-7 days after application).

The following compounds gave at least 80% control of *Alternaria solani* at 200 ppm when compared to untreated control under the same conditions, which showed extensive disease development: P-2, P-3, P-4, P-6, P-8, P-10, P-12, P-14, P-15, P-18, P-19, P-20, P-23, P-24, P-25, P-26, P-27, P-28, P-29, P-30, P-31, P-32, P-33, P-34, P-35, P-36, P-37.

### Example B2: Botryotinia fuckeliana (Botrytis cinerea) / liquid culture (Gray mould)

Conidia of the fungus from cryogenic storage are directly mixed into a nutrient broth (Vogels broth). After placing a DMSO solution of test compound into a microtiter plate (96-well format), the nutrient broth containing the fungal spores is added. The test plates are incubated at 24°C and the inhibition of growth is determined photometrically 3-4 days after application.

The following compounds gave at least 80% control of *Botryotinia fuckeliana* at 20 ppm when compared to untreated control under the same conditions, which showed extensive disease development: P-2, P-3, P-6, P-7, P-8, P-10, P-12, P-14, P-15, P-18, P-19, P-20, P-22, P-24, P-25, P-26, P-29, P-30, P-31, P-32, P-33, P-34, P-35, P-36.

### Example B3: Glomerella lagenarium (Colletotrichum laqenarium) / liquid culture (Anthracnose)

Conidia of the fungus from cryogenic storage are directly mixed into a nutrient broth (potato dextrose broth). After placing a DMSO solution of test compound into a microtiter plate (96-well format), the nutrient broth containing the fungal spores is added. The test plates are incubated at 24°C and the inhibition of growth is measured photometrically 3-4 days after application.

The following compounds gave at least 80% control of *Glomerella lagenarium* at 20 ppm when compared to untreated control under the same conditions, which showed extensive disease development: P-2, P-3, P-4, P-5, P-6, P-7, P-8, P-10, P-12, P-14, P-15, P-18, P-19, P-20, P-22, P-23, P-24, P-25, P-26, P-27, P-28, P-29, P-30, P-31, P-32, P-33, P-34, P-35, P-36.

### Example B4: Blumeria graminis f. sp. tritici (Erysiphe graminis f. sp. tritici) / wheat / leaf disc preventative (Powdery mildew on wheat)

Wheat leaf segments cv. Kanzler are placed on agar in a multiwell plate (24-well format) and sprayed with the formulated test compound diluted in water. The leaf disks are inoculated by shaking powdery mildew infected plants above the test plates 1 day after application. The inoculated leaf disks are incubated at 20°C and 60% relative humidity under a light regime of 24 h darkness followed by 12 h light / 12 h darkness in a climate chamber and the activity of a compound is assessed as percent disease control compared to untreated when an appropriate level of disease damage appears on untreated check leaf segments (6-8 days after application).

The following compounds gave at least 80% control of *Blumeria graminis f. sp. tritici* at 200 ppm when compared to untreated control under the same conditions, which showed extensive disease development: P-2, P-3, P-6, P-7, P-14, P-15, P-18, P-19, P-25, P-27, P-32, P-33, P-35.

### Example B5: Fusarium culmorum / liquid culture (Head blight)

Conidia of the fungus from cryogenic storage are directly mixed into a nutrient broth (potato dextrose broth). After placing a DMSO solution of test compound into a microtiter plate (96-well format), the nutrient broth containing the fungal spores is added. The test plates are incubated at 24°C and the inhibition of growth is determined photometrically 3-4 days after application.

The following compounds gave at least 80% control of *Fusarium culmorum* at 20 ppm when compared to untreated control under the same conditions, which showed extensive disease development: P-2, P-3, P-6, P-7, P-10, P-14, P-15, P-18, P-24, P-25, P-32, P-33, P-34.

### Example B6: Fusarium culmorum / wheat / spikelet preventative (Head blight)

Wheat spikelets cv. Monsun are placed on agar in multiwell plates (24-well format) and sprayed with the formulated test compound diluted in water. The spikelets are inoculated with a spore suspension of the fungus 1 day after application. The inoculated spikelets are incubated at 20°C and 60% relative humidity under a light regime of 72 h semi darkness followed by 12 h light / 12 h darkness in a climate chamber and the activity of a compound is assessed as percent disease control compared to untreated when an appropriate level of disease damage appears on untreated check spikelets (6 - 8 days after application).

The following compounds gave at least 80% control of *Fusarium culmorum* at 200 ppm when compared to untreated control under the same conditions, which showed extensive disease development: P-15, P-25.

### Example B7: Gibberella zeae (Fusarium graminearum) / wheat / spikelet preventative (Head blight)

Wheat spikelets cv. Monsun are placed on agar in multiwell plates (24-well format) and sprayed with the formulated test compound diluted in water. One day after application, the spikelets are inoculated with a spore suspension of the fungus. The inoculated test leaf disks are incubated at 20°C and 60% relative humidity under a light regime of 72 h semi darkness followed by 12 h light / 12 h darkness in a climate chamber, the activity of a compound is assessed as percent disease control compared to untreated when an appropriate level of disease damage appears on untreated check spikelets (6 - 8 days after application).

The following compounds gave at least 80% control of *Gibberella zeae* at 200 ppm when compared to untreated control under the same conditions, which showed extensive disease development: P-10, P-25.

### Example B8: Phaeosphaeria nodorum (Septoria nodorum) / wheat / leaf disc preventative (Glume blotch)

Wheat leaf segments cv. Kanzler are placed on agar in a multiwell plate (24-well format) and sprayed with the formulated test compound diluted in water. The leaf disks are inoculated with a spore suspension of the fungus 2 days after application. The inoculated test leaf disks are incubated at 20°C and 75% relative humidity under a light regime of 12 h light / 12 h darkness in a climate cabinet and the activity of a compound is assessed as percent disease control compared to untreated when an appropriate level of disease damage appears in untreated check leaf disks (5-7 days after application).

The following compounds gave at least 80% control of *Phaeosphaeria nodorum* at 200 ppm when compared to untreated control under the same conditions, which showed extensive disease development: P-2, P-3, P-4, P-6, P-10, P-12, P-14, P-15, P-18, P-19, P-20, P-22, P-24, P-25, P-26, P-27, P-28, P-29, P-30, P-31, P-32, P-33, P-34, P-35.

### Example B9: Monographella nivalis (Microdochium nivale) / liquid culture (foot rot cereals)

Conidia of the fungus from cryogenic storage are directly mixed into a nutrient broth (potato dextrose broth). After placing a DMSO solution of test compound into a microtiter plate (96-well format), the nutrient broth containing the fungal spores is added. The test plates are incubated at 24°C and the inhibition of growth is determined photometrically 4-5 days after application.

The following compounds gave at least 80% control of *Monographella nivalis* at 20 ppm when compared to untreated control under the same conditions, which showed extensive disease development: P-1, P-2, P-3, P-4, P-6, P-7, P-8, P-9, P-10, P-12, P-22, P-23, P-24, P-25, P-26, P-27, P-28, P-29, P-30, P-31, P-32, P-33, P-34, P-35, P-36, P-37.

### Example B10: Mycosphaerella arachidis (Cercospora arachidicola) / liquid culture (early leaf spot)

Conidia of the fungus from cryogenic storage are directly mixed into a nutrient broth (potato dextrose broth). After placing a DMSO solution of test compound into a microtiter plate (96-well format), the nutrient broth containing the fungal spores is added. The test plates are incubated at 24°C and the inhibition of growth is determined photometrically 4-5 days after application.

The following compounds gave at least 80% control of *Mycosphaerella arachidis* at 20 ppm when compared to untreated control under the same conditions, which showed extensive disease development: P-2, P-5, P-6, P-7, P-8, P-9, P-10, P-12, P-14, P-15, P-18, P-19, P-20, P-22, P-24, P-28, P-29, P-30, P-31, P-32, P-33, P-34, P-35, P-36, P-37.

### Example B11: Magnaporthe grisea (Pyricularia oryzae) / rice / leaf disc preventative (Rice Blast)

Rice leaf segments cv. Ballila are placed on agar in a multiwell plate (24-well format) and sprayed with the formulated test compound diluted in water. The leaf segments are inoculated with a spore suspension of the fungus 2 days after application. The inoculated leaf segments are incubated at 22°C and 80% relative humidity under a light regime of 24 h darkness followed by 12 h light / 12 h darkness in a climate cabinet and the activity of a compound is assessed as percent disease control compared to untreated when an appropriate level of disease damage appears in untreated check leaf segments (5-7 days after application). The following compounds gave at least 80% control of *Magnaporthe grisea* at 200 ppm when compared to untreated control under the same conditions, which showed extensive disease development: P-19, P-30.

### Example B12: Pyrenophora teres / barley / leaf disc preventative (Net blotch)

Barley leaf segments cv. Hasso are placed on agar in a multiwell plate (24-well format) and sprayed with the formulated test compound diluted in water. The leaf segmens are inoculated with a spore suspension of the fungus 2 days after application. The inoculated leaf segments are incubated at 20°C and 65% relative humidity under a light regime of 12 h light / 12 h darkness in a climate cabinet and the activity of a compound is assessed as disease control compared to untreated when an appropriate level of disease damage appears in untreated check leaf segments (5-7 days after application).

The following compounds gave at least 80% control of *Pyrenophora teres* at 200 ppm when compared to untreated control under the same conditions, which showed extensive disease development: P-2, P-3, P-4, P-6, P-8, P-14, P-15, P-18, P-19, P-22, P-24, P-25, P-27, P-28, P-30, P-31, P-32, P-33, P-34, P-35, P-36.

### Example B13: Thanatephorus cucumeris (Rhizoctonia solani) / liquid culture (foot rot, damping-off)

Mycelia fragments of a newly grown liquid culture of the fungus are directly mixed into a nutrient broth (potato dextrose broth). After placing a DMSO solution of the test compounds into a microtiter plate (96-well format), the nutrient broth containing the fungal material is added. The test plates are incubated at 24°C and the inhibition of growth is determined photometrically 3-4 days after application.

The following compounds gave at least 80% control of *Thanatephorus cucumeris* at 20 ppm when compared to untreated control under the same conditions, which showed extensive disease development: P-9.

### Example B14: Sclerotinia sclerotiorum / liquid culture (cottony rot)

Mycelia fragments of a newly grown liquid culture of the fungus are directly mixed into a nutrient broth (potato dextrose broth). After placing a DMSO solution of test compound into a microtiter plate (96-well format) the nutrient broth containing the fungal material is added. The test plates are incubated at 24°C and the inhibition of growth is determined photometrically 3-4 days after application.

The following compounds gave at least 80% control of *Sclerotinia sclerotiorum* at 20 ppm when compared to untreated control under the same conditions, which showed extensive disease development: P-2, P-3, P-6, P-7, P-10, P-12, P-14, P-18, P-19, P-24, P-25, P-30, P-32, P-33, P-34, P-35.

### Example B15: Mycosphaerella graminicola (Septoria tritici) / liquid culture (Septoria blotch)

Conidia of the fungus from cryogenic storage are directly mixed into a nutrient broth (potato dextrose broth). After placing a DMSO solution of test compound into a microtiter plate (96-well format), the nutrient broth containing the fungal spores is added. The test plates are incubated at 24°C and the inhibition of growth is determined photometrically 4-5 days after application. The following compounds gave at least 80% control of *Mycosphaerella graminicola* at 20 ppm when compared to untreated control under the same conditions, which showed extensive disease development: P-1, P-2, P-3, P-4, P-6, P-7, P-8, P-9, P-10, P-12, P-14, P-15, P-16, P-18, P-19, P-20, P-22, P-23, P-24, P-25, P-26, P-27, P-28, P-29, P-30, P-31, P-32, P-33, P-34, P-35, P-36, P-37.

## Claims

1. A compound of formula (I): 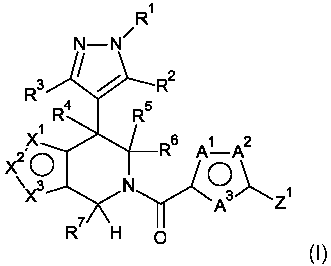 wherein
R¹ is selected from the group consisting of hydrogen, C₁-C₄ alkyl, C₂-C₄ alkenyl, C₂-C₄ alkynyl and C₃-C₆ cycloalkyl;
R² is selected from the group consisting of hydrogen, halogen, C₁-C₄ alkyl, C₂-C₄ alkenyl, C₂-C₄ alkynyl, C₁-C₄ haloalkyl, C₃-C₆ cycloalkyl, C₁-C₄ alkylcarbonyl, N-C₁-C₄ alkoxy-C-C₁-C₄ alkyl-carbonimidoyl, N-hydroxy-C-C₁-C₄ alkyl-carbonimidoyl and C₁-C₄ alkoxycarbonyl;
R³ is selected from the group consisting of hydrogen, halogen and C₁-C₄ alkyl;
R⁴ is selected from the group consisting of hydrogen, halogen and C₁-C₄ alkyl;
R⁵ and R⁶ are independently selected from the group consisting of hydrogen and C₁-C₄ alkyl;
R⁷ is selected from the group consisting of hydrogen, C₁-C₄ alkyl, C₁-C₄ alkylcarbonyl, N-C₁-C₄ alkoxy-C-C₁-C₄ alkyl-carbonimidoyl, N-hydroxy-C-C₁-C₄ alkyl-carbonimidoyl, C₁-C₄ alkoxycarbonyl, N-methoxy-N-methyl-carbonyl, C₁-C₄ alkylaminocarbonyl, di(C₁-C₄ alkylamino)carbonyl, phenyl, 5- or 6-membered heteroaryl and C₃-C₆ cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆-cycloalkyl are optionally substituted by 1, 2 or 3 substituents independently selected from halogen, cyano, C₁-C₄ alkyl, C₁-C₄ haloalkyl or C₁-C₄ alkoxy;
Z¹ is selected from the group consisting of C₁-C₄ alkyl, phenyl, 5- or 6-membered heteroaryl and C₃-C₆-cycloalkyl, wherein the 5- or 6-membered heteroaryl comprises 1, 2, 3 or 4 heteroatoms individually selected from N, O and S, and wherein any of said phenyl, 5- or 6-membered heteroaryl and C₃-C₆-cycloalkyl are optionally substituted by 1, 2 or 3 substituents independently selected from halogen, cyano, C₁-C₄ alkyl, C₁-C₄ haloalkyl, C₁-C₄ alkoxy, C₁-C₄ haloalkoxy, C₁-C₄ alkylsulfanyl, C₁-C₄ alkylsulfinyl, C₁-C₄ alkylsulfonyl or C₂-C₄ alkynyl;
X¹, X² and X³ are independently selected from the group consisting of CR⁸, N and S, with the proviso that one of X¹, X² and X³ is S;
R⁸ is selected from the group consisting of hydrogen, halogen, and C₁-C₄ alkyl;
A¹, A² and A³ are independently selected from the group consisting of CR⁹, N, NR¹⁰, O and S, with the proviso that at least one of A¹, A² and A³ is selected from N, O and S, and that no more than one of A¹, A² and A³ is O or S;
R⁹ is selected from the group consisting of hydrogen, C₁-C₄ alkyl, C₂-C₄ alkenyl and C₂-C₄ alkynyl;
R¹⁰ is selected from the group consisting of hydrogen, C₁-C₄ alkyl, C₂-C₄ alkenyl and C₂-C₄ alkynyl;
or an agrochemically acceptable salt, stereoisomer, enantiomer, tautomer or N-oxide thereof.

2. The compound of formula (I) according to claim 1, wherein R¹ is methyl, ethyl or isopropyl.

3. The compound of formula (I) according to claim 1 or claim 2, wherein R² is selected from the group consisting of hydrogen, fluorine, chlorine and methyl.

4. The compound of formula (I) according to any one of claims 1 to 3, wherein R³ is selected from the group consisting of hydrogen, fluorine, chlorine, methyl and ethyl.

5. The compound of formula (I) according to any one of claims 1 to 4, wherein R⁴ is selected from the group consisting of hydrogen, fluorine, chlorine, methyl and ethyl.

6. The compound of formula (I) according to any one of claims 1 to 5, wherein R⁵ and R⁶ are independently selected from the group consisting of hydrogen, methyl and ethyl.

7. The compound of formula (I) according to any one of claims 1 to 6, wherein R⁷ is selected from the group consisting of hydrogen, methyl, acetyl, -C(CH₃)=NOCH₃, -C(CH₃)=NOCH₂CH₃, -C(CH₃)=NOH, methoxycarbonyl, ethoxycarbonyl, N-methoxy-N-methyl-carbonyl, phenyl and cyclopropyl.

8. The compound of formula (I) according to any one of claims 1 to 7, wherein
X¹ is S, and X² and X³ are independently selected from the group consisting of CR⁸ and N, or
X² is S, and X¹ and X³ are independently selected from the group consisting of CR⁸ and N, or
X³ is S, and X¹ and X² are independently selected from the group consisting of CR⁸ and N,
wherein R⁸ is selected from the group consisting of hydrogen, chlorine, bromine, methyl and ethyl.

9. The compound of formula (I) according to any one of claims 1 to 8, wherein A¹, A² and A³ are independently selected from the group consisting of CR⁹, N, O and S, with the proviso that at least one of A¹, A² and A³ is selected from N, O and S, and that no more than one of A¹, A² and A³ is O or S.

10. The compound of formula (I) according to any one of claims 1 to 9, wherein Z¹ is selected from the group consisting of methyl, cyclobutyl, cyclopentyl, cyclohexyl, 2-methylphenyl, 2-fluorophenyl, 4-fluorophenyl, 3-chlorophenyl, 4-fluoro-2-methoxy-phenyl, 2,4-difluorophenyl, 3,4-difluorophenyl, 2,4,6-trifluorophenyl, 3,5-difluoro-2-pyridyl, 2-furyl, 2-thienyl, 3-thienyl and 1-methylpyrazol-4-yl.

11. An intermediate compound of formula (III) or a salt thereof: 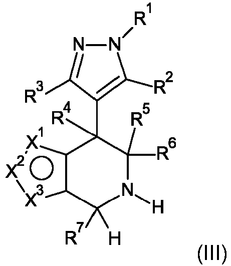 wherein R¹, R², R³, R⁴, R⁵, R⁶, R⁷, X¹, X² and X³ correspond to the same definitions as for the compounds of formula (I) according to any one of claims 1 to 10.

12. An agrochemical composition comprising a fungicidally effective amount of a compound of formula (I) as defined in any one of claims 1 to 10.

13. The composition according to claim 12, further comprising at least one additional active ingredient and/or an agrochemically-acceptable diluent or carrier.

14. A method of controlling or preventing infestation of useful plants by phytopathogenic microorganisms, wherein a fungicidally effective amount of a compound of formula (I) as defined in any one of claims 1 to 10, or a composition comprising the compound of formula (I), is applied to the plants, to parts thereof or the locus thereof.

15. Use of a compound according to any one of claims 1 to 10 as a fungicide, wherein said use excludes methods for the treatment of the human or animal body by surgery or therapy.

## Patentansprüche

1. Verbindung der Formel (I): 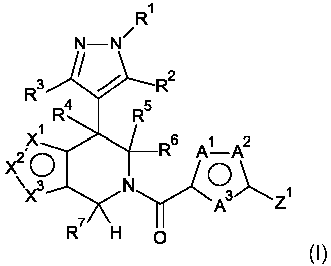 wobei
R¹ aus der Gruppe bestehend aus Wasserstoff, C₁-C₄-Alkyl, C₂-C₄-Alkenyl, C₂-C₄-Alkinyl und C₃-C₆-Cycloalkyl ausgewählt ist;
R² aus der Gruppe bestehend aus Wasserstoff, Halogen, C₁-C₄-Alkyl, C₂-C₄-Alkenyl, C₂-C₄-Alkinyl, C₁-C₄-Halogenalkyl, C₃-C₆-Cycloalkyl, C₁-C₄-Alkylcarbonyl, N-C₁-C₄-Alkoxy-C-C₁-C₄-alkylcarbonimidoyl, N-Hydroxy-C-C₁-C₄-alkylcarbonimidoyl und C₁-C₄-Alkoxycarbonyl ausgewählt ist;
R³ aus der Gruppe bestehend aus Wasserstoff, Halogen und C₁-C₄-Alkyl ausgewählt ist;
R⁴ aus der Gruppe bestehend aus Wasserstoff, Halogen und C₁-C₄-Alkyl ausgewählt ist;
R⁵ und R⁶ unabhängig aus der Gruppe bestehend aus Wasserstoff und C₁-C₄-Alkyl ausgewählt sind;
R⁷ aus der Gruppe bestehend aus Wasserstoff, C₁-C₄-Alkyl, C₁-C₄-Alkylcarbonyl, N-C₁-C₄-Alkoxy-C-C₁-C₄-alkylcarbonimidoyl, N-Hydroxy-C-C₁-C₄-alkylcarbonimidoyl, C₁-C₄-Alkoxycarbonyl, N-Methoxy-N-methylcarbonyl, C₁-C₄-Alkylaminocarbonyl, Di (C₁-C₄-alkylamino)carbonyl, Phenyl, 5- oder 6-gliedrigem Heteroaryl und C₃-C₆-Cycloalkyl ausgewählt ist, wobei das 5- oder 6-gliedrige Heteroaryl 1, 2, 3 oder 4 Heteroatome, die individuell aus N, O und S ausgewählt sind, umfasst und wobei das Phenyl, 5- oder 6-gliedrige Heteroaryl und C₃-C₆-Cycloalkyl jeweils gegebenenfalls durch 1, 2 oder 3 Substituenten, die unabhängig aus Halogen, Cyano, C₁-C₄-Alkyl, C₁-C₄-Halogenalkyl oder C₁-C₄-Alkoxy ausgewählt sind, substituiert sind;
Z¹ aus der Gruppe bestehend aus C₁-C₄-Alkyl, Phenyl, 5-oder 6-gliedrigem Heteroaryl und C₃-C₆-Cycloalkyl ausgewählt ist, wobei das 5- oder 6-gliedrige Heteroaryl 1, 2, 3 oder 4 Heteroatome, die individuell aus N, O und S ausgewählt sind, umfasst und wobei das Phenyl, 5- oder 6-gliedrige Heteroaryl und C₃-C₆-Cycloalkyl jeweils gegebenenfalls durch 1, 2 oder 3 Substituenten, die unabhängig aus Halogen, Cyano, C₁-C₄-Alkyl, C₁-C₄-Halogenalkyl, C₁-C₄-Alkoxy, C₁-C₄-Halogenalkoxy, C₁-C₄-Alkylsulfanyl, C₁-C₄-Alkylsulfinyl, C₁-C₄-Alkylsulfonyl oder C₂-C₄-Alkinyl ausgewählt sind, substituiert sind; X¹, X² und X³ unabhängig aus der Gruppe bestehend aus CR⁸, N und S ausgewählt sind, mit der Maßgabe, dass eines von X¹, X² und X³ für S steht;
R⁸ aus der Gruppe bestehend aus Wasserstoff, Halogen und C₁-C₄-Alkyl ausgewählt ist;
A¹, A² und A³ unabhängig aus der Gruppe bestehend aus CR⁹, N, NR¹⁰, O und S ausgewählt sind, mit der Maßgabe, dass mindestens eines von A¹, A² und A³ aus N, O und S ausgewählt ist und dass nicht mehr als ein A¹, A² und A³ für O oder S steht;
R⁹ aus der Gruppe bestehend aus Wasserstoff, C₁-C₄-Alkyl, C₂-C₄-Alkenyl und C₂-C₄-Alkinyl ausgewählt ist;
R¹⁰ aus der Gruppe bestehend aus Wasserstoff, C₁-C₄-Alkyl, C₂-C₄-Alkenyl und C₂-C₄-Alkinyl ausgewählt ist;
oder ein agrochemisch unbedenkliches Salz, Stereoisomer, Enantiomer, Tautomer oder N-Oxid davon.

2. Verbindung der Formel (I) nach Anspruch 1, wobei R¹ für Methyl, Ethyl oder Isopropyl steht.

3. Verbindung der Formel (I) nach Anspruch 1 oder Anspruch 2, wobei R² aus der Gruppe bestehend aus Wasserstoff, Fluor, Chlor und Methyl ausgewählt ist.

4. Verbindung der Formel (I) nach einem der Ansprüche 1 bis 3, wobei R³ aus der Gruppe bestehend aus Wasserstoff, Fluor, Chlor, Methyl und Ethyl ausgewählt ist.

5. Verbindung der Formel (I) nach einem der Ansprüche 1 bis 4, wobei R⁴ aus der Gruppe bestehend aus Wasserstoff, Fluor, Chlor, Methyl und Ethyl ausgewählt ist.

6. Verbindung der Formel (I) nach einem der Ansprüche 1 bis 5, wobei R⁵ und R⁶ unabhängig aus der Gruppe bestehend aus Wasserstoff, Methyl und Ethyl ausgewählt sind.

7. Verbindung der Formel (I) nach einem der Ansprüche 1 bis 6, wobei R⁷ aus der Gruppe bestehend aus Wasserstoff, Methyl, Acetyl, -C(CH₃)=NOCH₃,-C(CH₃)=NOCH₂CH₃, -C(CH₃)=NOH, Methoxycarbonyl, Ethoxycarbonyl, N-Methoxy-N-methylcarbonyl, Phenyl und Cyclopropyl ausgewählt ist.

8. Verbindung der Formel (I) nach einem der Ansprüche 1 bis 7, wobei
X¹ für S steht und X² und X³ unabhängig aus der Gruppe bestehend aus CR⁸ und N ausgewählt sind oder
X² für S steht und X¹ und X³ unabhängig aus der Gruppe bestehend aus CR⁸ und N ausgewählt sind oder
X³ für S steht und X¹ und X² unabhängig aus der Gruppe bestehend aus CR⁸ und N ausgewählt sind,
wobei R⁸ aus der Gruppe bestehend aus Wasserstoff, Chlor, Brom, Methyl und Ethyl ausgewählt ist.

9. Verbindung der Formel (I) nach einem der Ansprüche 1 bis 8, wobei A¹, A² und A³ unabhängig aus der Gruppe bestehend aus CR⁹, N, O und S ausgewählt sind, mit der Maßgabe, dass mindestens eines von A¹, A² und A³ aus N, O und S ausgewählt ist und dass nicht mehr als eines von A¹, A² und A³ für O oder S steht.

10. Verbindung der Formel (I) nach einem der Ansprüche 1 bis 9, wobei Z¹ aus der Gruppe bestehend aus Methyl, Cyclobutyl, Cyclopentyl, Cyclohexyl, 2-Methylphenyl, 2-Fluorphenyl, 4-Fluorphenyl, 3-Chlorphenyl, 4-Fluor-2-methoxyphenyl, 2,4-Difluorphenyl, 3,4-Difluorphenyl, 2,4,6-Trifluorphenyl, 3,5-Difluor-2-pyridyl, 2-Furyl, 2-Thienyl, 3-Thienyl und 1-Methylpyrazol-4-yl ausgewählt ist.

11. Zwischenverbindung der Formel (III) oder ein Salz davon: 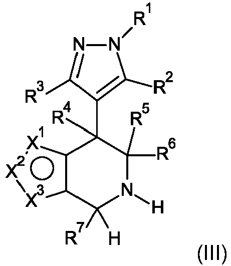 wobei R¹, R², R³, R⁴, R⁵, R⁶, R⁷, X¹, X² und X³ den gleichen Definitionen wie für die Verbindungen der Formel (I) nach einem der Ansprüche 1 bis 10 entsprechen.

12. Agrochemische Zusammensetzung, umfassend eine fungizid wirksame Menge einer Verbindung der Formel (I) gemäß einem der Ansprüche 1 bis 10.

13. Zusammensetzung nach Anspruch 12, weiterhin umfassend mindestens einen zusätzlichen Wirkstoff und/oder ein agrochemisch unbedenkliches Verdünnungsmittel oder einen agrochemisch unbedenklichen Träger.

14. Verfahren zur Bekämpfung oder Prävention des Befalls von Nutzpflanzen durch phytopathogene Mikroorganismen, bei dem man eine fungizid wirksame Menge einer Verbindung der Formel (I) gemäß einem der Ansprüche 1 bis 10 oder eine Zusammensetzung, die die Verbindung der Formel (I) umfasst, auf Pflanzen, Teile davon oder deren Standort ausbringt.

15. Verwendung einer Verbindung nach einem der Ansprüche 1 bis 10 als Fungizid, wobei Verfahren zur chirurgischen oder therapeutischen Behandlung des menschlichen oder tierischen Körpers ausgeschlossen sind.

## Revendications

1. Composé de formule (I) : 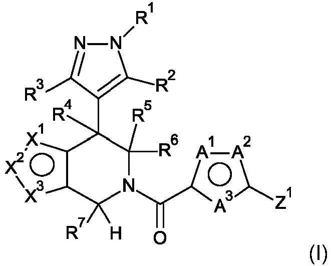
R¹ étant choisi dans le groupe constitué d'hydrogène, C₁-C₄ alkyle, C₂-C₄ alcényle, C₂-C₄ alcynyle et C₃-C₆ cycloalkyle ;
R² étant choisi dans le groupe constitué d'hydrogène, halogène, C₁-C₄ alkyle, C₂-C₄ alcényle, C₂-C₄ alcynyle, C₁-C₄ halogénoalkyle, C₃-C₆ cycloalkyle, C₁-C₄ alkylcarbonyle, N-C₁-C₄ alcoxy-C-C₁-C₄ alkyl-carbonimidoyle, N-hydroxy-C-C₁-C₄ alkyl-carbonimidoyle et C₁-C₄ alcoxycarbonyle ;
R³ étant choisi dans le groupe constitué d'hydrogène, halogène et C₁-C₄ alkyle ;
R⁴ étant choisi dans le groupe constitué d'hydrogène, halogène et C₁-C₄ alkyle ;
R⁵ et R⁶ étant indépendamment choisis dans le groupe constitué d'hydrogène et C₁-C₄ alkyle ;
R⁷ étant choisi dans le groupe constitué d'hydrogène, C₁-C₄ alkyle, C₁-C₄ alkylcarbonyle, N-C₁-C₄ alcoxy-C-C₁-C₄ alkyl-carbonimidoyle, N-hydroxy-C-C₁-C₄ alkyl-carbonimidoyle, C₁-C₄ alcoxycarbonyle, N-méthoxy-N-méthyl-carbonyle, C₁-C₄ alkylaminocarbonyle, di(C₁-C₄ alkylamino)carbonyle, phényle, hétéroaryle à 5 ou 6 chaînons et C₃-C₆ cycloalkyle, l'hétéroaryle à 5 ou 6 chaînons comprenant 1, 2, 3 ou 4 hétéroatomes individuellement choisis parmi N, O et S, et quelconque parmi lesdits phényle, hétéroaryle à 5 ou 6 chaînons et C₃-C₆-cycloalkyle étant éventuellement substitués par 1, 2 ou 3 substituants indépendamment choisis parmi halogène, cyano, C₁-C₄ alkyle, C₁-C₄ halogénoalkyle ou C₁-C₄ alcoxy ;
Z¹ étant choisi dans le groupe constitué de C₁-C₄ alkyle, phényle, hétéroaryle à 5 ou 6 chaînons et C₃-C₆-cycloalkyle, l'hétéroaryle à 5 ou 6 chaînons comprenant 1, 2, 3 ou 4 hétéroatomes individuellement choisis parmi N, O et S, et quelconque parmi lesdits phényle, hétéroaryle à 5 ou 6 chaînons et C₃-C₆-cycloalkyle étant éventuellement substitués par 1, 2 ou 3 substituants indépendamment choisis parmi halogène, cyano, C₁-C₄ alkyle, C₁-C₄ halogénoalkyle, C₁-C₄ alcoxy, C₁-C₄ halogénoalcoxy, C₁-C₄ alkylsulfanyle, C₁-C₄ alkylsulfinyle, C₁-C₄ alkylsulfonyle ou C₂-C₄ alcynyle ; X¹, X² et X³ étant indépendamment choisis dans le groupe constitué de CR⁸, N et S, à la condition que l'un parmi X¹, X² et X³ soit S ;
R⁸ étant choisi dans le groupe constitué d'hydrogène, halogène, et C₁-C₄ alkyle ;
A¹, A² et A³ étant indépendamment choisis dans le groupe constitué de CR⁹, N, NR¹⁰, O et S, à la condition qu'au moins l'un parmi A¹, A² et A³ soit choisi parmi N, O et S, et que pas plus d'un parmi A¹, A² et A³ soit O ou S ; R⁹ étant choisi dans le groupe constitué d'hydrogène, C₁-C₄ alkyle, C₂-C₄ alcényle et C₂-C₄ alcynyle ;
R¹⁰ étant choisi dans le groupe constitué d'hydrogène, C₁-C₄ alkyle, C₂-C₄ alcényle et C₂-C₄ alcynyle ;
ou sel, stéréoisomère, énantiomère, forme tautomère ou N-oxyde acceptable sur le plan agrochimique correspondant(e).

2. Composé de formule (I) selon la revendication 1, R¹ étant méthyle, éthyle ou isopropyle.

3. Composé de formule (I) selon la revendication 1 ou la revendication 2, R² étant choisi dans le groupe constitué d'hydrogène, fluor, chlore et méthyle.

4. Composé de formule (I) selon l'une quelconque des revendications 1 à 3, R³ étant choisi dans le groupe constitué d'hydrogène, fluor, chlore, méthyle et éthyle.

5. Composé de formule (I) selon l'une quelconque des revendications 1 à 4, R⁴ étant choisi dans le groupe constitué d'hydrogène, fluor, chlore, méthyle et éthyle.

6. Composé de formule (I) selon l'une quelconque des revendications 1 à 5, R⁵ et R⁶ étant indépendamment choisis dans le groupe constitué d'hydrogène, méthyle et éthyle.

7. Composé de formule (I) selon l'une quelconque des revendications 1 à 6, R⁷ étant choisi dans le groupe constitué d'hydrogène, méthyle, acétyle, -C(CH₃)=NOCH₃, - C(CH₃)=NOCH₂CH₃, -C(CH₃)=NOH, méthoxycarbonyle, éthoxycarbonyle, N-méthoxy-N-méthyl-carbonyle, phényle et cyclopropyle.

8. Composé de formule (I) selon l'une quelconque des revendications 1 à 7,
X¹ étant S, et X² et X³ étant indépendamment choisis dans le groupe constitué de CR⁸ et N, ou
X² étant S, et X¹ et X³ étant indépendamment choisis dans le groupe constitué de CR⁸ et N, ou
X³ étant S, et X¹ et X² étant indépendamment choisis dans le groupe constitué de CR⁸ et N,
R⁸ étant choisi dans le groupe constitué d'hydrogène, chlore, brome, méthyle et éthyle.

9. Composé de formule (I) selon l'une quelconque des revendications 1 à 8, A¹, A² et A³ étant indépendamment choisis dans le groupe constitué de CR⁹, N, O et S, à la condition qu'au moins l'un parmi A¹, A² et A³ soit choisi parmi N, O et S, et que pas plus d'un parmi A¹, A² et A³ soit O ou S.

10. Composé de formule (I) selon l'une quelconque des revendications 1 à 9, Z¹ étant choisi dans le groupe constitué de méthyle, cyclobutyle, cyclopentyle, cyclohexyle, 2-méthylphényle, 2-fluorophényle, 4-fluorophényle, 3-chlorophényle, 4-fluoro-2-méthoxy-phényle, 2,4-difluorophényle, 3,4-difluorophényle, 2,4,6-trifluorophényle, 3,5-difluoro-2-pyridinyle, 2-furyle, 2-thiényle, 3-thiényle et 1-méthylpyrazol-4-yle.

11. Composé intermédiaire de formule (III) ou sel correspondant : 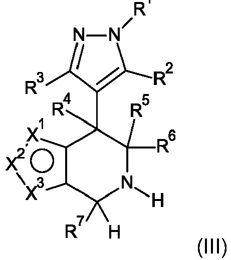 R¹, R², R³, R⁴, R⁵, R⁶, R⁷, X¹, X² et X³ correspondant aux mêmes définitions que pour les composés de formule (I) selon l'une quelconque des revendications 1 à 10.

12. Composition agrochimique comprenant une quantité efficace sur le plan fongicide d'un composé de formule (I) tel que défini dans l'une quelconque des revendications 1 à 10.

13. Composition selon la revendication 12, comprenant en outre au moins un ingrédient actif supplémentaire et/ou un diluant ou véhicule acceptable sur le plan agrochimique.

14. Procédé pour le contrôle ou la prévention contre une infestation de plantes utiles par des microorganismes phytopathogènes, une quantité efficace sur le plan fongicide d'un composé de formule (I) tel que défini dans l'une quelconque des revendications 1 à 10, ou une composition comprenant le composé de formule (I), étant appliquée sur les végétaux, sur des parties de ceux-ci ou sur le lieu où ils se développent.

15. Utilisation d'un composé selon l'une quelconque des revendications 1 à 10 en tant que fongicides, ladite utilisation excluant des procédés pour le traitement du corps humain ou animal par chirurgie ou thérapie.
